# EUROPEAN PATENT SPECIFICATION

(11) **EP 1 948 146 B1**
(45) Date of publication and mention of the grant of the patent: **07.11.2012**
(21) Application number: 06805783.5
(22) Date of filing: 20.09.2006
(51) Int. Cl.: A61K 31/05, A61P 11/00

(54) **NOVEL MULTI-CYCLIC COMPOUNDS AND THEIR USE**
NEUE MULTICYCLISCHE VERBINDUNGEN UND IHRE VERWENDUNG
NOUVEAUX COMPOSES MULTICYCLIQUES

(30) Priority: 20.09.2005 EP 05020511
(43) Date of publication of application: 30.07.2008
(73) Proprietor: Revotar Biopharmaceuticals AG, 16761 Henningsdorf (DE)
(72) Inventor: AYDT, Ewald, 64380 Rossdorf (DE); KRANICH, Remo, 13503 Berlin (DE); BUSEMANN, Anke, 59757 Arnsberg (DE)
(74) Representative: Jacobi, Markus Alexander
(86) International application number: PCT/EP2006/009152
(87) International publication number: WO 2007/039111

(56) References cited:
- US-A- 5 374 772
- US-A1- 2005 113 416
- NOMOTO T ET AL: "Preparation of hydroxybenzamide derivatives as prevention and treatment agents for bone diseases" CHEMICAL ABSTRACTS + INDEXES, AMERICAN CHEMICAL SOCIETY. COLUMBUS, US, vol. 13, no. 125, 23 September 1996 (1996-09-23), XP002047512 ISSN: 0009-2258
- APPELDOORN C C M ET AL: "Rational Optimization of a Short Human P-selectin-binding Peptide Leads t Nanomolar Affinity Antagonists" JOURNAL OF BIOLOGICAL CHEMISTRY, AMERICAN SOCIETY OF BIOLOCHEMICAL BIOLOGISTS, BIRMINGHAM,, US, vol. 278, no. 12, 21 March 2003 (2003-03-21), pages 10201-10207, XP002275710 ISSN: 0021-9258
- YAMAZAKI R ET AL: "Diarylheptanoids suppress expression of leukocyte adhesion molecules on human vascular endothelial cells." EUROPEAN JOURNAL OF PHARMACOLOGY. 22 SEP 2000, vol. 404, no. 3, 22 September 2000 (2000-09-22), pages 375-385, XP009062460 ISSN: 0014-2999

## Description

The present invention relates to compounds, compositions and methods for modulating the *in vitro* and *in vivo* processes mediated by cell adhesion molecules. The disclosed small molecules comprise dimethoxy and dihydroxy phenyl subunits and modulate cell adhesion molecule-mediated functions potently.

Cell-adhesion molecule-mediated functions are part of a complex cascade leading to the migration of circulating white blood cells (leukocytes) from the blood stream into the surrounding tissue (transmigration). Physiologically, leukocyte transmigration is of critical importance for homeostasis and immuno-surveillance of living beings including humans. Lymphocytes for example, are constitutively leaving the blood stream into lymphatic tissues in order to patrol for harmful antigens. Under pathological circumstances however, e.g. local or systemic inflammation and/or injury of the vascular system, this fundamental process is dys-regulated, at least in part, due to an increased surface expression of E- and P-selectin. Consequently, the excessive leukocyte transmigration leads to a pathological cellular infiltrate with subsequent tissue damage in several clinically relevant settings. Disease states such as Acute Lung Injury (ALI), Acute Respiratory Distress Syndrome (ARDS), Asthma bronchiale (asthma), Chronic Obstructive Pulmonary Disease (COPD), Psoriasis, Rheumatoid Arthritis, and Sepsis are all associated with tissue inflammation induced and perpetuated by pathologically activated leukocytes infiltrating the respective tissue. In addition, exaggerated leukocyte infiltration contributes to the pathogenesis of Ischemic-Reperfusion Injury (IRI) associated with organ transplantation, cardiopulmonary bypass or percutaneous transluminal angioplasty.

To transmigrate, leukocytes must bind to the wall of the vascular endothelium to diffuse through the cell wall of the capillary into the surrounding tissue. Therefore, leukocytes have to roll onto and then adhere to the endothelial cell wall (initial rolling or "tethering"). This primary event in transmigration is mediated by the selectin family of cell-adhesion molecules. In addition to directly binding to the endothelium, leukocytes can adhere to other leukocytes, leukocyte-particles, platelets or platelet-derived particles that are already attached to the endothelium.

The selectin family of adhesion molecules is comprised of three structurally related calcium-dependent carbohydrate binding cell surface proteins, E-, P- and L-selectin. E-selectin is expressed only on inflamed endothelium, P-selectin is expressed on inflamed endothelium as well as on platelets and L-selectin is expressed on leukocytes. Selectins are composed of an amino terminal lectin domain, an epidermal growth factor (EGF)-like domain, a variable number of complement receptor-related repeats, a hydrophobic transmembrane domain and a C-terminal cytoplasmic domain. The binding interactions leading to the adhesion of the leukocytes are supposed to be mediated by contact of the lectin domain of the selectins and various carbohydrate ligands on the surface of the leukocytes. All three selectins can bind with low affinity to the carbohydrate sialyl Lewis^{x} (sLe^{x}), a glycosyl moiety present on the surface of most leukocytes. A structurally related glycosyl moiety, sialyl Lewis^{a} (sLe^{a}), is predominantly found on the surface of cancer cells [K. Okazaki et al., J. Surg. Res., 1998, 78(1). 78-84; R. P. McEver et al., Glycoconjugate Journal, 1997, 14(5), 585-591]. In case of P-selectin, a distinct high affinity glycoprotein ligand has been described [R.P. McEver, R.D. Cummings, J.Clin.lnvest., 1997, 100, 485-492], the so-called P-selectin glycoprotein ligand-1 (PSGL-1), which contributes to a high affinity selectin binding by its sLe^{x} moiety as well as by parts of its peptide components, in particular sulphated tyrosine residues [R.P. McEver, Ernst Schering Res. Found. Workshop, 2004, 44, 137-147]. PSGL-1 is one of the most important selectin ligarids binding with highest affinity to P-selectin, but it also binds to E- and L-selectin [G. Constantin; Drug News Perspect; 2004; 17(9); 579-586]. It is a homodimeric sialomucin predominantly expressed on leukocytes.

In inflammatory diseases, dys-regulated transmigration is, at least in part, mediated due to an increased cell surface expression of E- and P-selectin. In contrast to their low basal expression, E- and P-selectin expression is upregulated during inflammation, leading to a substantial recruitment of leukocytes into the inflamed tissue. Although selectin-mediated cell adhesion is required for fighting infection, there are various situations in which such cell adhesion is undesirable or excessive, resulting in severe tissue damage instead of repair. In the case of many acute as well as chronic inflammatory disorders [e.g., asthma, chronic obstructive pulmonary disease (COPD), psoriasis, etc.], an association between infiltration of activated leukocytes into the tissue simultaneously with a marked elevation of tissue expression of corresponding adhesion molecules, particularly E- and P-selectin, has been demonstrated [Muller et al., J. Pathol., 2002, 198(2), 270-275; Di Stefano et al., Am. J. Respir. Crit. Care. Med., 1994, 149(3) 803-810; Terajima et al., Arch. Dermatol. Res., 1998, 290, 246-252]

Leukocyte infiltration may also play a role in inflammatory symptoms in the course of transplant and graft rejection. Also the process of blood clotting is further promoted by leukocyte-leukocyte and leukocyte-platelet binding, which occurs because leukocytes possess both L-selectin and its corresponding ligand PSGL-1 and can thus interact with themselves via PSGL-1, and they can also bind to platelets which carry P-selectin.

Therefore, the modulation of selectin-mediated cell adhesion and other selectin mediated functions, e.g. leukocyte activation, offers a promising possibility to interfere with and stop the inflammation cascade at a very early step. Small molecule selectin antagonists should modulate all three selectins simultaneously as pan-selectin-antagonists to circumvent possible redundancies between the selectins [M. Sperandio et al., Vascular Disease Prevention, 2004, 1, 185-195].

Besides sLe^{x}/sLe^{a}, the natural, high affinity ligand PSGL-1 is another template structure for the design of small molecule selectin antagonists. As compared to sLe^{x}/sLe^{a}, PSGL-1 shows high affinity for all three selectins. To find and to detect novel small molecule drugs that compete with PSGL-1 and PSGL-1-like ligands for selectin binding is therefore a promising strategy to develop a novel class of effective pan-selectin antagonists for treating inflammatory disorders. Selectin antagonists may be designed using selectins as well as using a ligand like PSGL-1 as a template structure, since they are intended to modulate the binding between selectins and PSGL-1 or other ligands with similar binding motifs.

Novel small molecule selectin antagonists could meet certain requirements to be drug-like and to have potential oral bioavailability. The term drug likeness is described in the literature [Lipinski; Adv. Drug Dev. Rev., 1997, 23, 3-25]. Beside other molecular properties, passively transported molecules are supposed to have on average a relative molecular weight of less than 500 in order to be drug like. According to these rules it is common to define compounds with a relative molecular weight of less 500 or closely above that as small molecules. Compounds with relative molecular weights above 500 are unlikely to be orally bioavailable. Also the presence of highly polar carbohydrate moieties or a peptidic components is not in accordance with the concept of drug likeness [H. Ulbrich et al., Trends Pharmacol. Sci., 2003, 24(12), 640-647; D. Slee et al., J. Med. Chem., 2001, 44, 2094-2107]. The same accounts for the development of antibody-based drugs, because they are polypeptides and so oral administration is a problem. Moreover, the desired compounds must be stable during the passage through the gastrointestinal tract so that they can be ingested/absorbed latest by the cells of the small intestines. This is not the case for most glycosidic molecules and peptidic structures.

There have been various investigations to develop low-molecular weight compounds with a modulatory effect on selectin mediated processes. These compounds include disalicylates and disalicylate-based C-glycosides [WO 99/29706], benzyl amino sulfonic acids [WO 03/097658], diglycosylated 1,2-diols [WO 97/01569], substituted 5-membered heterocycles [WO 00/33836], mannopyranosyloxy-phenyl-benzoic acids [EP0758243 B1], piperazine based compounds [US6432957B1], gallic acid derivatives of peptides [WO 2004/018502], gallic acid [C. C. M. Appeldoorn et al., Circulation 2005, 111, 106-112; EP 1481669A1], and quinic acid derivatives [N. Kaila et al., J Med. Chem. 2005, 48, 4346-4357]. However, none of these selectin-antagonizing compounds have successfully passed clinical trials up to date [S. J. Romano, Treat. Respir Med 2005, 4(2), 85-94; M. P. Schön, Therapeutics and Clinical Risk Management, 2005, 1(3), 201-208]. This is due to the fact, that many of these structures have been designed on the basis of the low potency template sLe^{x}. Therefore, sLe^{x}-mimicking structures are unlikely to show low potency. Other compounds show specificity against different members of the selectin family, but antagonizing only selected selectins can be bypassed by other selectins [M. P. Schön, Therapeutics and Clinical Risk Management, 2005, 1(3),201-208]. In addition, most of the compounds developed so far have high molecular weights and often bear carbohydrates and/or peptides making them prone to degradation and modification by peptidases and/or glycosidases. Carbohydrate-bearing structures have further disadvantages such as high degree of chirality, anomericity, and low probability of transport through lipid bilayers. Similar disadvantages are known for peptide-bearing compounds. Some other compounds developed for antagonizing selectin mediated processes contain pyrogallol- and catechol-substructures. These motifs are prone to oxidation processes [Kumamoto M. et al., Biosci. Biotechnol. Biochem., 2001, 65(1), 126-132] making the pharmaceutical development of these compounds difficult. In addition, compounds with pyrogallol substructures, such as gallic acid, are known to be cytotoxic [E. Sergediene et al., FEBS Letters, 1999, 462, 392-396] and induce apoptosis [K. Satoh et al., Anticancer Research, 1997, 17, 2487-2490; N. Sakaguchi et al., Biochemical Pharmacology, 1998, 55, 1973-1981].

The leading compound in the field of selectin antagonists is bimosiamose [S. J. Romano, Treat. Respir Med 2005, 4(2), 85-94]. Presently bimosiamose [D. Bock et al., New Drugs, 2003, D04, 28, p.28; EP 0 840 606 B1] is the most advanced compound in clinical studies Recent investigations support the hypothesis that bimosiamose can be considered as PSGL-1 mimetic [E. Aydt, G. Wolff; Pathobiology; 2002-2003; 70; 297-301]. This distinguishes bimosiamose from other selectin antagonists. It is, however, a high molecular weight compound with carbohydrate structures. The pan-selectin antagonist bimosiamose seems to lack oral bioavailability. Some observations indicate that bimosiamose shows good affinity for P-selectin and a moderate affinity for E- and L-selectin.

There is a strong medical need for novel highly potent pan-selectin antagonists which modulate selectin-mediated function, e.g. of selectin-dependent cell adhesion, and for the development of methods employing such compounds to modulate conditions associated with selectin-ligand interaction. Most of the available anti-inflammatory pharmaceutical therapies, which are available on the market, comprise mostly corticosteroids or NSAIDs (non steroidal anti-inflammatory drugs) having several serious drawbacks/side effects, and target different steps of the inflammatory cascade. Unlike this, modulating the selectin function is a therapeutic concept intervening the inflammation cascade at a very early stage. Almost all promising selectin antagonists so far failed to become marketed drugs, mostly because of low potency and /or high molecular weight that causes problems in their absorption-distribution-metabolism-excretion (ADME) behaviour and thus in oral bioavailability required for the treatment of most inflammatory disorders like rheumatoid arthritis, septic shock, atherosclerosis, reperfusion injury and many others.

Object of the invention is to provide novel small molecules, especially non-glycosylated/non-glycosidic and non-peptidic compounds, which are able to potently antagonize selectin-mediated processes and which have less negative side effects during their application than prior art compounds.

Unlike most of the sLe^{x}-mimicking compounds developed in this field, the inventive compounds are not prone to glycosidases or peptidases. Most of the selectin antagonists developed so far are structurally and biologically based on the properties of sLe^{x} or sLe^{a}. These resulting compounds showed, therefore, low biological activity like their template structures. This invention, however, provides novel potent small and drug like pan-selectin antagonists that have been invented on the basis of biological in vitro assays mimicking PSGL-1 and PSGL-1-like ligands or any ligands bearing sLe^{x} or sLe^{a} and tyrosinesulfate motifs [N. V. Bovin; Biochem Soc Symp.; 2002;(69):143-60. N. V. Bovin; Glycoconj. J.; 1998; 15(5); 431-46. T.V. Pochechueva et al.; Bioorg Med Chem Lett.; 2003;13(10);1709-12. G. Weitz-Schmidt et al.; Anal. Biochem.;1996;238; 184-190].

The present invention provides pharmaceutical compositions comprising at least one compound having the general structure of formulas G1 or G2 or H1 or H2 and a pharmaceutically acceptable carrier which is useful in medicine. 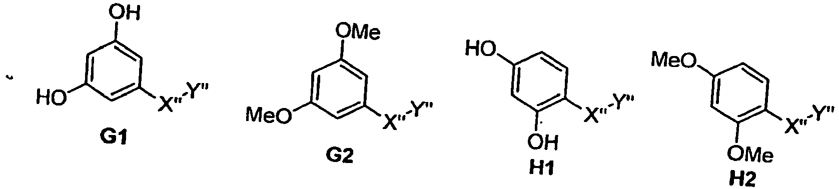 wherein the symbols and substituents have the following meaning.

X"- is 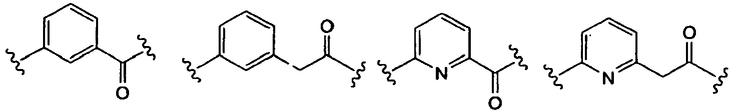 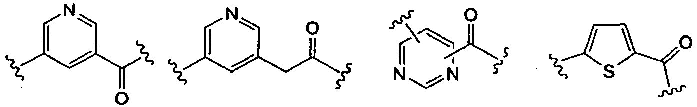 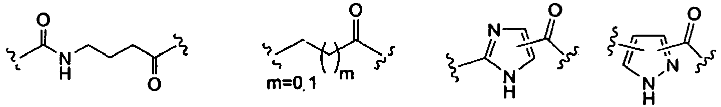 with m = 0,1;

Y" is 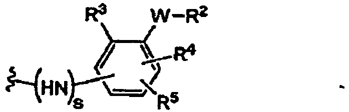 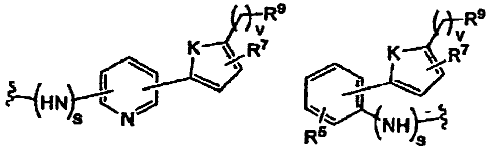 with s being 0 or 1, R² being CO₂H CO₂Akyl, CO₂Aryl, CO₂NH₂, CO₂Aralkyl, SO₃H SO₂NH₂, PO(OH)₂, 1-H-tetrazolyl-, CHO, COCH₃, CH₂OH, NH₂, NHAlkyl, N(Alkyl)Atkyl', OCH₃, CH₂OCH₃, SH, F, Cl, Br, I, CH₃, CH₂CH₃, CN, CF₃
R³ independently from R² being H, CH₃, CH₂CH₃, CF₃, F, Cl, Br, I, CN, NO₂ and R⁴ independently from R² and R³ being H, CH₃, CH₂CH₃, CF₃, F, Cl, Br, I, CN, NO₂, R²
R⁵ being H, NO₂, CF₃, F, Cl. Br, I, CN, CH₃, OCH₃, SH, NH₂
and -W- = -(CH₂-)ᵥ, cis-CH=CH- or *trans*-CH=CH-*,* and v being 0,1,2;
in case that -W- is *cis*-CH=CH- or *trans-*CH=CH-, R² must not be NH₂ or SH;
R⁷ independently from R² being H, NO₂, CF₃, F, Cl, Br, I, CN, CH₃, OCH₃, SH, NH₂, with K=NH, NMe, O, S
with R⁹ being CO₂H, CO₂alkyl, CO₂aryl, CO₂NH₂, CO₂aralkyl, CH₂SO₃H, CH₂SO₂NH₂, CH₂PO(OH)₂, 1-H-tetrazolyl, CHO, COCH₃, CH₂OH, CH₂NH₂, CH₂NHalkyl, CH₂N(alkyl)alkyl', CH₂OCH₃, CH₂SH.
or the pharmaceutically acceptable salts, esters or amides of the above identified compounds of formulas G1 or G2 or H1 or H2.

Particularly preferred pharmaceutical compositions comprise compounds of formulas (E1), (E2), (F1), or (F2) 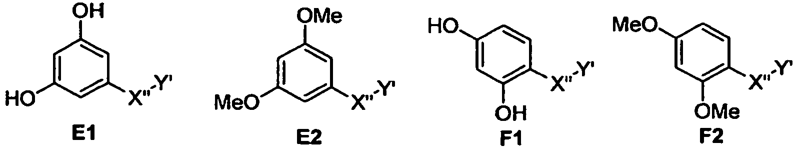 wherein -X" is like defined above,and wherein -Y' is 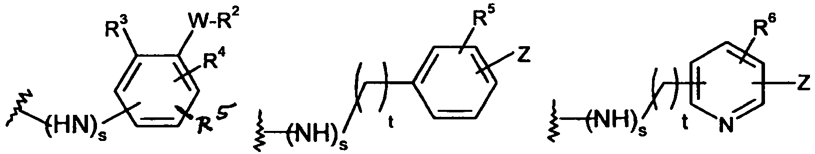 wherein all indices, symbols and substituents are as defined as above.

R⁶ independently from R² being H, F, Cl, Me, tert-Bu, CN, NH₂

These chemical compounds (E1), (E2), (F1), (F2), (G1), (G2), (H1) and (H2) are also new compounds for themselves.

All compounds as described before present the ability of modulating cell adhesion and modulate selectin- as well as PSGL-1-like mediated binding. The compounds have the ability to modulate the interaction of selectins with sLe^{x}/sLe^{a} and also the interaction between selectins and tyrosinesulfate residues. Therefore they are useful for the treatment of acute and chronic inflammatory disorders, as well as other medical conditions where selectin mediated processes play a role.

The term "pharmaceutical" includes also diagnostic applications.

The term "pharmaceutical" includes also prophylactic applications in order to prevent medical conditions where selectin mediated processes play a role.

The term "pharmaceutical" includes also applications, where compounds of the present invention may be used as vehicles for drug targeting of diagnostics or therapeutics.

In a further preferred variant the invention provides pharmaceutical compositions comprising at least one compound of formula (E1), (E2), (F1), (F2), (G1), (G2), (H1), or (H2).

In a particularly preferred variant the invention provides pharmaceutical compositions comprising at least one compound of formula (E1), (E2), (F1) or (F2).

In a very particularly preferred variant the invention provides pharmaceutical compositions comprising at least one compound of formula (G1), (G2), (H1) or (H2).

The present invention further provides a method of nodulating the binding of P-selectin, L-selectin or E-selectin to sLe^{x} or sLe^{a} and tyrosinesulfate residues comprising the step of administering to a patient an effective amounts of at least one compound having the structure of formulas (G1) or (G2) or (H1) or (H2) or (E1) or (E2) or (F1) or (F2) to modulate the binding of P-, E- or L-selectin to sLe^{x} or sLe^{a} and tyrosinesulfate. It has been found that compounds having the formulas (G1) or (G2) or (H1) or (H2) or (E1) or (E2) or (F1) or (F2) shown above act to modulate E-, P- or L-selectin binding.

As used herein the terms "alkyl" shall mean a monovalent straight chain or branched chain group of 1 or 2 or 3 or 4 or 5 or 6 or 7 or 8 or 9 or 10 or 11 or 12 carbon atoms for example, methyl, ethyl, n-propyl, isopropyl, n-butyl, sec-butyl, tert-butyl and the like. "Alkyl" are independently from each other and can be different or identical.

The term "aryl" shall mean carbocyclic and heterocyclic aromatic groups, for example phenyl, 1-naphthyl, 2-naphthyl, fluorenyl, (1,2)-dihydronaphthyl, indenyl, indanyl, thienyl, benzothienyl, thienopyridyl and the like.

The term "aralkyl" (also called arylalkyl) shall mean an aryl group appended to an alkyl group, for example benzyl, 1-naphthylmethyl, 2-naphthylmethyl, fluorobenzyl, chlorobenzyl, bromobenzyl, iodobenzyl, alkoxybenzyl (wherein "alkoxy" means methoxy, ethoxy, isopropoxy, n-butoxy, sec-butoxy, tert-butoxy an the like), hydroxybenzyl, aminobenzyl, nitrobenzyl, guanidinobenzyl, fluorenylmethyl, phenylmethyl(benzyl), 1-phenylethyl, 2-phenylethyl, 1-naphthylethyl and the like.

The term "acyl" shall mean -(CHO) or ―(C=O)-alkyl or ―C=O)-aryl or ―(C=O)-aralkyl, for example formyl, acetyl, n-propionyl, isopropionyl, n-butyryl, isobutyryl, pivaloyl, benzoyl, 4-nitrobenzoyl and the like.

The term "pharmaceutically acceptable salts, esters, amides and prodrugs" as used herein refers to those carboxylate salts, amino acid addition salts, esters, amides and prodrugs of the compounds of the present invention which are, within the scope of sound medical judgement, suitable for use in contact with tissues of patients without undue toxicity, irritation, allergic response and the like, commensurate with a reasonable benefit/risk ratio, and effective for their intended use, as well as the zwitterionic forms, where possible, of the compounds of the present invention. The term "salts" refers to the relatively non-toxic, inorganic and organic acid addition salts of the compounds of the present invention. These salts can be prepared *in situ* during the final isolation and purification of the compounds or by separately reacting the purified compounds in its free form with a suitable inorganic or organic acid or base and isolating the salt thus formed. Representative salts of the compounds of the present invention include the hydrobromide, hydrochloride, sulfate, bisulfate, nitrate, acetate, oxalate, valerate, palmitate, stearate, laurate, borate, benzoate, lactate, phosphate, tosylate, citrate, maleate, fumarate, succinate, tartrate, naphthylate, mesylate, glucoheptonate, lactiobionate, laurylsulphonate salts and the like. These may include cations based on the alkali and alkalineearth metals, such as sodium, lithium, potassium, calcium, magnesium and the like, as well as non-toxic ammonium, quaternary ammonium and amine cations, for example ammonium, tetramethylammonium, tetraethylammonium, methylamine, dimethylamine, trimethylamine, triethylamine, ethylamine, and the like.

Examples of the pharmaceutically acceptable, non-toxic esters of the compounds of this invention include C₁, C₂, C₃, C₄, C₅ and C₆ alkyl esters wherein the alkyl group is a straight or branched chain. Acceptable esters also include C₅, C₆ and C₇ cycloallcyl esters as well arylalkyl esters such as, benzyl. C₁, C₂, C₃, C₄, C₅ and C₆ alkyl ester are preferred. Esters of the compounds of the present invention may be prepared according to conventional methods.

Examples of pharmaceutically acceptable, non-toxic amides of compounds of this invention include amides derived from ammonia, primary C₁, C₂, C₃, C₄, C₅ and C₆ alkyl amines and secondary C₁, C₂, C₃, C₄, C₅ and C₆ dialkyl amines wherein the alkyl groups are straight or branched chains. In the case of secondary amines the amine may also be in the form of a 5 or 6 membered heterocycle containing one nitrogen atom. Amides derived from ammonia, C₁, C₂ and C₃ alkyl primary amides and C₁ to C₂ dialkyl secondary amides are preferred. Amides of the compounds of the present invention may be prepared according to conventional methods.

The term "prodrug" refers to one or more compounds that are rapidly transformed *in vitro* and from a non-active to active state *in vivo* to yield the parent compound of the above formulas (Ia) or (Ib) or (IIa) or (IIb), for example by hydrolysis in blood or *in vivo* metabolism.

It is also contemplated that pharmaceutically active compositions may contain a compound of the present invention or other compounds that modulate or compete with E-selectin or P-selectin or L-selectin binding.

Pharmaceutically active compositions of the present invention comprise a pharmaceutically acceptable carrier and a compound of formulas (Ia) or (Ib) or (IIa) or (IIb), whereby a pharmaceutically acceptable carrier can also be a medically appropriate nano-particle, dendrimer, liposome, microbubble or polyethylene glycol (PEG). The pharmaceutical compositions of the present invention may include one or more of the compounds having the above structure (Ia) or (Ib) or (IIa) or (IIb) formulated together with one or more, physiologically acceptable carriers, adjuvants or vehicles, which are collectively referred to herein as carriers, for parenteral injection, for oral administration in solid or liquid form, for rectal or topical administration and the like.

The compositions can be administered to humans and animals either orally, rectally, parenterally (intravenously, intramuscularly, intradermaly or subcutaneously), intracisternally, intravaginally, interperitoneally, locally (powders, ointments or drops), or as a buccal or by inhalation (nebulized, or as nasal sprays).

Compositions suitable for parenteral injection may comprise physiologically acceptable sterile aqueous or nonaqueous solutions, stabilizers, antioxidants, preservatives (e.g. ascorbic acid, sodium sulfite, sodium hydrogene sulfite, benzyl alcohol, EDTA), dispersions, suspensions or emulsions and sterile powders for reconstitution into sterile injectable solution or dispersion. Examples of suitable aqueous and nonaqueous carriers, diluents, solvents or vehicles include water, ethanol, polyol, (propylene glycol, polyethylene glycol, glycerol and the like), suitable mixtures thereof, vegetable oils (such as olive or canola oil) and injectable organic esters such as ethyl oleate. Proper fluidity can be maintained, for examples, by the use of a coating such as lecithin, by the maintenance of the required particle size in the case of dispersions and by the use of surfactants.

These compositions may also contain adjuvants such as preserving, wetting, emulsifying, and dispersing agents. Prevention of the actions of microorganisms can be ensured by various antibacterial and antifungal agents, for examples, parabens, chlorobutanol, phenol, sorbic acid, and the like. It may also be desirable to include isotonic agents, for examples sugars, sodium chloride and the like. Prolonged absorption of the injectable pharmaceutical form can be brought about by the use of agents delaying absorption, for examples aluminium monostearate and gelatin.

If desired, and for more effective distribution, the compounds can be incorporated into slow or timed release or targeted delivery systems such as polymer matrices, liposomes, and microspheres. They may be sterilized, for example, by filtration through a bacteria-retaining filter, or by incorporating sterilizing agents in the form of sterile water, or some other sterile injectable medium immediately before use.

Solid dosage forms for oral administration include capsules, tablets, pills, powders and granules. In such solid dosage forms, the active compound or a prodrug is admixed with at least one inert customary excipient (or carrier) such as sodium citrate or dicalcium phosphate or (i) fillers or extenders, as for example, starches, lactose, sucrose, glucose, mannitol and silicic acid, (ii) binders, as for example, carboxymethylcellulose, alginates, gelatine, polyvinylpyrrolidone, sucrose and acacia, (iii) humectants, as for example, glycerol, (div disintegrating agents, as for example, agar-agar, calcium carbonate, potato or tapioca starch, aliginic acid, certain complex silicates and sodium carbonate, (v) solution retarders, as for examples, paraffin, (vi) absorption-accelerators, as for example, quaternary ammonium compounds, (vii) wetting agents, as for examples, cetyl alcohol and glycerol monostearate, (viii) adsorbents, as for example, kaolin and bentonite, and (ix) lubricants, as for example, talc, calcium stearate, magnesium stearate, solid polyethylene glycols, sodium lauryl sulfate and mixtures thereof. In the case of capsules, tablets, and pills, the dosage forms may also comprise buffering agents.

Solid compositions of a similar type may also be employed as fillers in soft and hard-filled gelatine capsules using excipients as lactose or milk sugars as well as high molecular polyethylene glycols and the like. Solid dosage forms such as tablets, dragées, capsules, pills and granules can be prepared with coatings and shells, such as enteric coatings and others well known in the art. They may contain opacifying agents, and can also be of such compositions that they release the active compound or compounds in a certain part of the intestinal tract in a delayed manner. Examples of embedding compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, cannola oil, caster oil and sesame seed oil, glycerol, tetrahydrofurfuryl alcohol, polyethylene glycols and fatty acid esters of sorbitan or mixtures of these substances, and the like. Besides such inert diluents, the compositions can also include adjuvants, such as wetting agents, emulsifying and suspending agents, sweeting, flavouring and perfuming agents.

Suspensions, in addition to the active compounds, may contain suspending agents, for example, ethoxylated isostearyl alcohols, polyoxyethylene sorbitol and sorbitan esters, microcrystalline cellulose, aluminium metahydroxide, bentonite, agar-agar, tragacanth or mixtures of these substances and the like.

Compositions for rectal administrations are preferably suppositories, which can be prepared by mixing the compounds of the present invention with suitable nonirritating excipients or carriers such as cacao butter, polyethylene glycol or a suppository wax, which are solid at ordinary temperatures but liquid at body temperature and therefore melt in the rectal or vaginal cavity and release the active component. Dosage forms for topical administration of a compound of this invention include ointments, powder, sprays and inhalants.

The active component is admixed under sterile conditions with a physiologically acceptable carrier and any needed preservatives, buffers or propellants as may be required. Ophthalmic formulations, eye ointments, suspensions, powder and solutions are also contemplated as being within the scope of this invention.

The compounds of the present invention can also be incorporated into or connected to liposomes or administrated in the form of liposomes. As is known in the art, liposomes are generally derived from phospholipids or other lipid substances. Liposomes are formed by mono or multilamellar hydrated liquid crystals that are dispersed in an aqueous medium. Any non-toxic, physiologically acceptable metabolized lipid capable of forming liposomes can be used. The present compositions in liposome form can contain, in addition to the selectin binding antagonists of the present invention, stabilizers, preservatives, excipients and the like. The preferred lipids are the phospholipids and the phosphatidyl cholines (lecithins), both natural and synthetic. Methods to form liposomes are well known in the art.

Non-parenteral dosage forms may also contain a bioavailability enhancing agent (e.g. enzyme modulateors, antioxidants) appropriate for the protection of the compounds against degradation. Actual dosage levels of active ingredient in the composition of the present invention may be varied so as to obtain an amount of active ingredient that is effective to obtain the desired therapeutic response for a particular composition and method of administration. The selected dosage level, therefore, depends on the desired therapeutic effect, on the route of administration, on the desired duration of treatment and other factors. The total daily dosage of the compounds on this invention administered to a host in single or divided doses may be in the range up to 50 mg per kilogram of body weight. Dosage unit compositions may contain such submultiples thereof as may be used to make up the daily dosage. It will be understood, however, that the specific dose level for any particular patient, whether human or other animal, will depend upon a variety of factors including the body weight, general health, sex diet, time and route of administration, rates of absorption and excretion, combination with other drugs and the severity of the particular disease being treated.

In particular, the compounds of the present invention may be used to treat a variety of diseases relating to inflammation and cell-cell recognition and adhesion. For example, the compounds of the present invention may be administrated to a patient to treat Chronic Obstructive Pulmonary Disease (COPD), acute lung injury (ALI), cardiopulmonary bypass, acute respiratory distress syndrome (ARDS), Crohn's disease, septic shock, sepsis, chronic inflammatory diseases such as psoriasis, atopic dermatitis, and rheumatoid arthritis, and reperfusion injury that occurs following heart attacks, strokes, atherosclerosis, and organ transplants, traumatic shock, multi-organ failure, autoimmune diseases like multiple sclerosis, percutaneous transluminal angioplasty, asthma and inflammatory bowel disease. In each case, an effective amount of the compounds of the present invention is administered either alone or as part of a pharmaceutically active composition to a patient in need of such treatment. It is also recognized that a combination of the compounds may be administered to a patient in need of such administration. The compounds of the present invention may also be administered to treat other diseases that are associated with cell-cell adhesion. As the present compounds modulate the binding of E-selectin or P-selectin or L-selectin, any disease that is related to this interaction may potentially be treated by the modulation of this binding interaction.

In addition to being found on some white blood cells, sLe^{a} is found on various cancer cells, including lung and colon cancer cells. It has been suggested that cell adhesion involving sLe^{a} may be involved in the metastasis of certain cancers and antagonists of sLe^{a} binding might be useful in treatment of some forms of cancer.

The use of the active ingredients according to the invention or of cosmetic compositions with an effective content of active ingredient according to the invention surprisingly enables effective treatment, but also prophylaxis of skin ageing caused by extrinsic and intrinsic factors.

The invention particularly relates to the use of a compound of formula (G1) or (G2) or (H1) or (H2) or (E1) or (E2) or (F1) or (F2) or a stereoisomeric form thereof for the preparation of a cosmetic or dermatological composition.

The amount used of the active compound or a stereoisomeric form thereof corresponds to the amount required to obtain the desired result using the cosmetic or dermatological compositions. One skilled in this art is capable of evaluating this effective amount, which depends on the derivative used, the individual on whom it is applied, and the time of this application. To provide an order of magnitude, in the cosmetic or dermatological compositions according to the invention, the compound of formula (G1) or (G2) or (H1) or (H2) or (E1) or (E2) or (F1) or (F2) or a stereoisomeric form thereof may be administered in an amount representing from 0.001% to 40% by weight, preferentially 0.005% to 30% by weight and more preferentially from 0.01% to 20% by weight.

A further aspect covers cosmetic compositions comprising a compound of formula (G1) or (G2) or (H1) or (H2) or (E1) or (E2) or (F1) or (F2) or a stereoisomeric form thereof and at least one cosmetically tolerable component, e.g. a cosmetically tolerable component for skin applications.

The amounts of the various components of the physiological medium of the cosmetic composition according to the invention are those generally included in the fields under consideration. When the cosmetic composition is an emulsion, the proportion of the fatty phase may range from 2% to 80% by weight and preferably from 5% to 50% by weight relative to the total weight of the cosmetic composition.

Thus, the cosmetic composition should contain a non-toxic physiologically acceptable medium that can be applied to human skin. For a topical application to the skin, the cosmetic composition may be in the form of a solution, a suspension, an emulsion or a dispersion of more or less fluid consistency and especially liquid or semi-liquid consistency, obtained by dispersing a fatty phase in an aqueous phase (O/W) or, conversely, (W/O), or alternatively a gel. A cosmetic composition in the form of a mousse or in the form of a spray or an aerosol then comprising a pressurized propellant may also be provided. Also the compositions may be in the form of a haircare lotion, a shampoo or hair conditioner, a liquid or solid soap, a treating mask, or a foaming cream or gel for cleansing the hair. They may also be in the form of hair dye or hair mascara.

The cosmetic compositions of the invention may also comprise one or more other ingredients usually employed in the fields under consideration, selected from among formulation additives, for instance aqueous-phase or oily-phase thickeners or gelling agents, dyestuffs that are soluble in the medium of the cosmetic composition, solid particles such as mineral or organic fillers or pigments in the form of microparticles or nanoparticles, preservatives, fragrances, hydrotopes or electrolytes, neutralizers (acidifying or basifying agents), propellants, anionic, cationic or amphoteric surfactants, polymers, in particular water-soluble or water-dispersible anionic, nonionic, cationic or amphoteric film-forming polymers, mineral or organic salts, chelating agents; mixtures thereof.

The cosmetic compositions may be used to inhibit the micro-inflammatory cycle. Thus, the present invention also relates to cosmetic compositions comprising a compound of formula (G1) or (G2) or (H1) or (H2) or (E1) or (E2) or (F1) or (F2) or a stereoisomeric form thereof that is used for the cosmetic treatment or cosmetic prophylaxis of micro-inflammatory conditions.

Cosmetic compositions comprising a compound of formula (G1) or (G2) or (H1) or (H2) or (E1) or (E2) or (F1) or (F2) or a stereoisomeric form thereof that is used for the cosmetic treatment or cosmetic prophylaxis of skin ageing caused by intrinsic factors are also subject of the present invention. Intrinsic factors responsible for skin ageing are genetically programmed determinants including age, hormonal status, and psychological factors.

Beside cosmetically inactive ingredients the cosmetic compositions of the present invention may also comprise one or more cosmetically active ingredients with beneficial action on the skin. Thus, the present invention relates to cosmetic compositions comprising a compound of formula (G1) or (G2) or (H1) or (H2) or (E1) or (E2) or (F1) or (F2) or a stereoisomeric form thereof and at least one further cosmetically active ingredient, e.g. an UV-blocker or proteins. Dermatological compositions comprising a compound of formula (G1) or (G2) or (H1) or (H2) or (E1) or (E2) or (F1) or (F2) or a stereoisomeric form thereof and at least one dermatologically tolerable component, e.g. a dermatologically tolerable component for skin applications, are also subject of the invention.

Dermatologically tolerable components that can be used for the dermatological compositions described here are identical to the cosmetically tolerable components as defined in this invention.

A further embodiment of this invention are dermatological compositions comprising a compound of formula (G1) or (G2) or (H1) or (H2) or (E1) or (E2) or (F1) or (F2) or a stereoisomeric form thereof that is used for the dermatological treatment, dermatological diagnosis or dermatological prophylaxis of micro-inflammatory conditions.

In particular the invention covers dermatological compositions comprising a compound of formula or (E1) or (E2) or (F1) or (F2) or a stereoisomeric form thereof that is used for the dermatological treatment, dermatological diagnosis or dermatological prophylaxis of itching and skin ageing caused by extrinsic factors. Extrinsic factors include environmental factors in general; more particularly photo-ageing due to exposure to the sun, to light or to any other radiation, atmospheric pollution, wounds, infections, traumatisms, anoxia, cigarette smoke, hormonal status as response to external factors, neuropeptides, electromagnetic fields, gravity, lifestyle (e.g. excessive consumption of alcohol), repetitive facial expressions, sleeping positions, and psychological stressors.

In addition to dermatologically inactive ingredients the dermatological compositions may also comprise dermatologically or pharmaceutically active ingredients. Thus, the present invention also relates to dermatological compositions comprising a compound of formula or (E1) or (E2) or (F1) or (F2) or a stereoisomeric form thereof and at least one further dermatologically or pharmaceutically active ingredient. The dermatologically or pharmaceutically active ingredients that can be used for the dermatological compositions described herein are defined as the cosmetically active ingredients defined above. Dermatologically or pharmaceutically active ingredients can be identical to the cosmetically active ingredients as defined in this invention.

Another subject of the present invention are dermatological compositions comprising a compound of formula (G1) or (G2) or (H1) or (H2) or (E1) or (E2) or (F1) or (F2) or a stereoisomeric form thereof and at least one further dermatologically or pharmaceutically active ingredient characterized in that it is used for the dermatological treatment, dermatological diagnosis or dermatological prophylaxis of micro-inflammatory conditions.

In particular, the present invention relates to dermatological compositions comprising a compound of formula (G1) or (G2) or (H1) or (H2) or (E1) or (E2) or (F1) or (F2) or a stereoisomeric form thereof and at least one further dermatologically or pharmaceutically active ingredient characterized in that it is used for the dermatological treatment, dermatological diagnosis or dermatological prophylaxis of itching and skin ageing caused by extrinsic factors.

Ageing of the skin may also be caused by a combination of intrinsic and extrinsic factors. Therefore, the present invention also relates to dermatological compositions comprising a compound of formula (G1) or (G2) or (H1) or (H2) or (E1) or (E2) or (F1) or (F2) or a stereoisomeric form thereof and at least one further pharmaceutically or cosmetically active ingredient characterized in that it is used for the cosmetic and dermatological treatment and cosmetic and dermatological prophylaxis of skin ageing caused by a combination of intrinsic and extrinsic factors.

Another embodiment of this invention is a process for the preparation of a cosmetic composition by mixing a compound of formula (G1) or (G2) or (H1) or (F2) or (E1) or (E2) or (F1) or (F2) or a stereoisomeric form thereof, at least one cosmetically tolerable component and eventually further cosmetically active ingredients.

In particular, a process for the preparation of a cosmetic composition by mixing a compound of formula (G1) or (G2) or (H1) or (H2) or (E1) or (E2) or (F1) or (F2) or a stereoisomeric form thereof, at least one of formula (G1) or (G2) or (H1) or (H2) or (E1) or (E2) or (F1) or (F2) or a stereoisomeric form thereof, at least one cosmetically tolerable component and eventually further cosmetically active ingredients, wherein the composition includes from 0.01% to 20% by weight of compound of formula (G1) or (G2) or (H1) or (H2) or (E1) or (E2) or (F1) or (F2) a stereoisomeric form thereof, based on the total weight of the composition is subject of this invention.

A further aspect deals with a process for the preparation of a dermatological composition by mixing a compound of formula (Ia) or (Ib) or (IIa) or (IIb) or a stereoisomeric form thereof, at least one dermatologically tolerable component and eventually further pharmaceutically active ingredients.

Many of the compounds of the present invention may be synthesized according to the following general synthetic schemes.

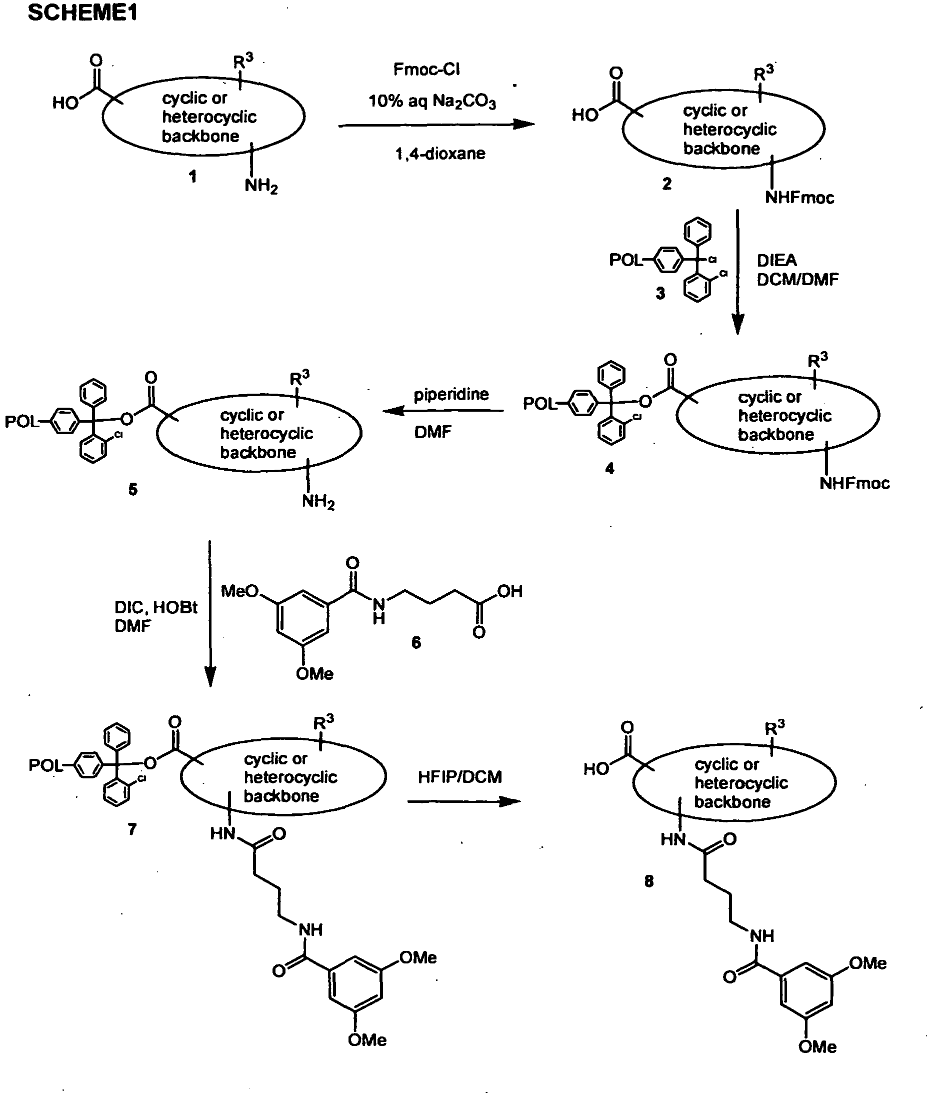

In SCHEME 1 an amino acid of type (1) is reacted with Fmoc-Cl in dioxane under basic conditions (10% Na₂CO₃ in water) to the corresponding *N*-Fmoc protected acid (2). Carboxylic acid (2) is immobilized to a 2-chlorotrityl chloride resin (3) to form the solid phase supported 2-chlorotrityl ester (4). Deprotection of (4) with piperidine in DMF gives amine of type (5). Further reaction of amine (5) with carboxylic acid (6) under standard coupling conditions (DIC and HOBt in DMF) gives amide (7) which is easily released from the resin with Hexafluoroisopropanol (HFIP) in dichloromethane to obtain carboxylic acids of type (8). Alternatively N'-(3-dimethylaminopropyl)-N-ethyl carbodiimide (EDC), triethylamine and 4-dimethylaminopyridine (DMAP) in a chlorinated solvent may be used for the amide coupling reaction step. The synthesis sequence shown in SCHEME 1 leading to compounds like (8) is not only reduced to the Y-H building blocks like (1) but may be generally applied to all other Y-H type building blocks bearing a carboxylic and a NH₂-function.

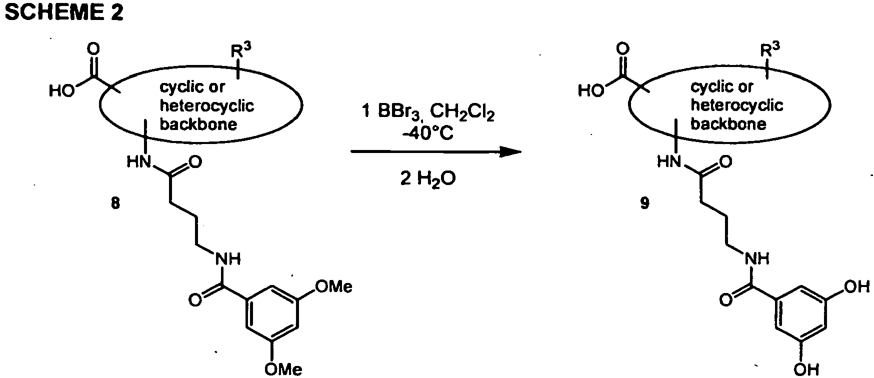

In SCHEME 2 carboxylic acids of type (8) are reacted with boron tribromide in dichloromethane at ―40°C to obtain after following aqueous workup corresponding demethylated acids of type (9). The synthesis sequence shown in SCHEME 2 leading to compounds like (9) is not only reduced to X-Y-H and Y-H building blocks like (8) but may be generally applied to all other X-Y-H and Y-H type building blocks.

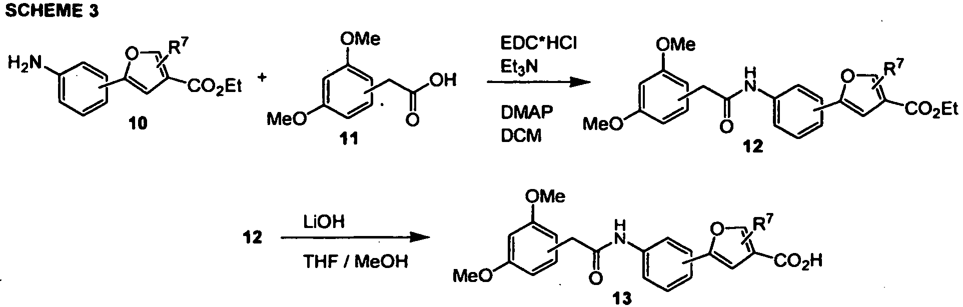

In SCHEME 3 an aniline of type (10) is reacted under inert atmosphere conditions with N'-(3-dimethylaminopropyl)-N-ethyl carbodiimide (EDC), triethylamine, 4-dimethylamino-pyridine (DMAP) and carboxylic acid of type (11) in dichloromethane to give an amide of type (12). Further hydrolysis of ester (12) with aqueous LiOH in THF and MeOH leads to carboxylic acids of type (13).

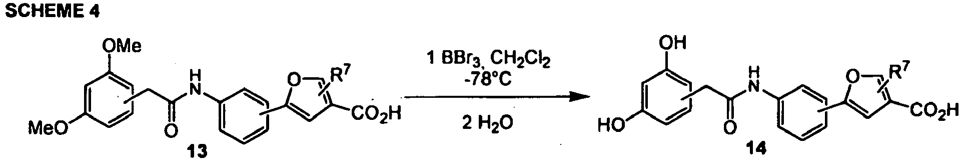

In SCHEME 4 carboxylic acids of type (13) are reacted with boron tribromide in dichloromethane at ―40°C to obtain after following aqueous workup corresponding demethylated acids of type (14). The synthesis sequence shown in SCHEME 4 leading to compounds like (14) is not only reduced to X-Y-H and Y-H building blocks like (13) but may be generally applied to all other X-Y-H and Y-H type building blocks.

The present invention is furthermore illustrated by the following representative examples.

### EXAMPLE 1

### 3-[4-(3,5-Dihydroxy-benzoylamino)-butyrylamino]-benzoic acid (21) and 3-[4-(3,5-Dihydroxy-benzoylamino)-butyrylamino]-benzoic acid methyl ester (22)

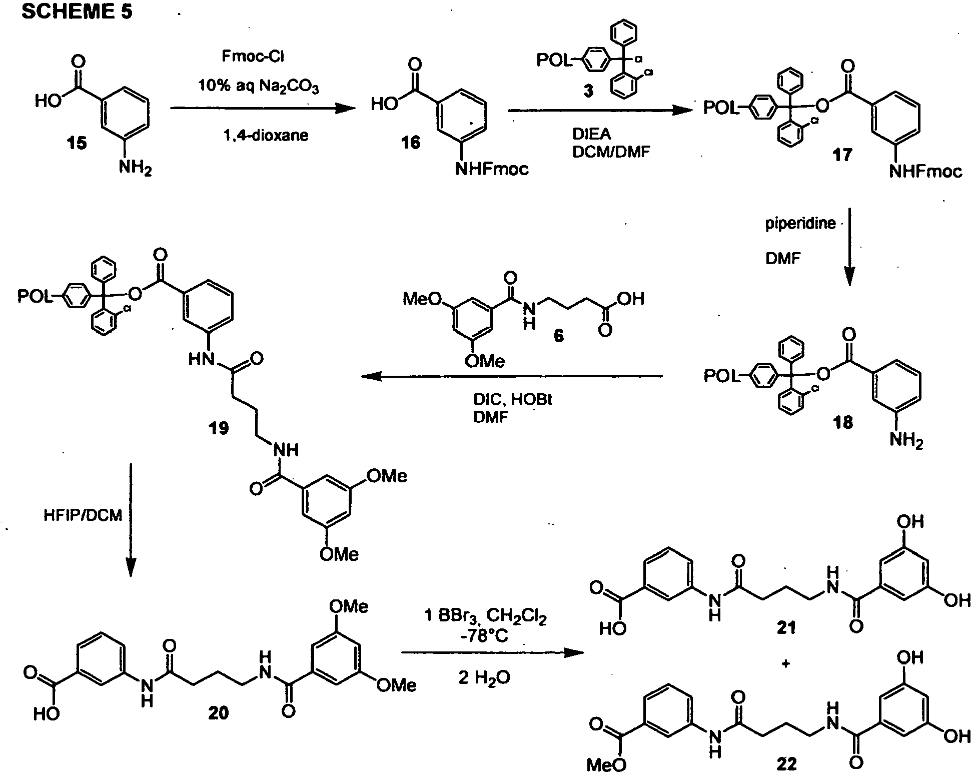

Step 1: Dissolve 3-Aminobenzoic acid ((15); 1.00g, 7.30mmol) in 1,4-dioxane (12.0mL) and 10% aqu. Na₂CO₃ (20.8mL) and add Fmoc-Cl (2.26g, 8.76mmol). Stir the reaction mixture for 2.5h at rt. Add 1M aqu HCl (42.0mL) to the mixture and extract with EtOAc (3 times). Wash the combined organic layers with 1M aqu HCl, water and brine, extract the combined aqu layers once again with EtOAc, dry the combined organic layers with Na₂SO₄ and remove solvent under reduced pressure. The left crude product is washed with ice cold EtOAc and dried in oil pump vacuum to obtain (16) as a white solid (1.61g, 61%). No further purification. [M. Nichifor; E. H. Schacht; Tetrahedron; 1994; 50; 12; 3747-3760]. ¹H NMR (400MHz, DMSO-d₆): 4.31 (t, 1 H, *J* = 6.6Hz); 4.49 (d, 2 H, *J* = 6.6Hz); 7.31-7.45 (m, 5 H); 7.56 (d, 1 H, *J* = 7.6Hz); 7.60-7.70 (br.m, 1 H); 7.75 (d, 2 H, *J* = 7.3Hz); 7.90 (d, 2 H, *J* = 7.3Hz); 8:11 (s, 1 H); 9.88 (s, 1 H).

**Step 2:** Dissolve (16) (180mg, 0.5mmol) in DCM (0.5mL) and DMF (0.25mL) at rt in a pre-dried tube, add DIEA (0.52mL, 1.5mmol) and add this solution to 2-chlorotritylchlorid-polystyrene (3) (82mg, 0.13mmol (loading 1.6mmo1/g)) which is preswollen before in DCM (0.15mL). Shake the reaction suspensions for 14h at rt. The resin bound product (17) is washed with DCM/MeOH/DIEA (17+2+1, 3 times), DCM (once), DMF (3 times) and again DCM (twice) and dried in vacuum. [i.e. Novabiochem® 2000 Catalog; 2000; S 15-S 18].

**Step 3:** Suspend complete amount of resin (17) from step 2 in DMF (0.4mL) and piperidine (0.1mL) and shake it for 1.5h at rt. Washed resin bound product (18) with DMF (3 times) and DCM (3 times for 30min) and dry in vacuum.

**Step 4:** Complete amount of resin (18) from step 3 is preswollen in DMF (0.8mL) for 20min. Dissolve acid (6) (158mg, 0.59mmol) and HOBt (90mg, 0.59mmol) in DMF (2.8mL) at rt, add DIC (75mg, 0.59mmol), stir for 15min and add this solution to the preswollen resin (18). Shake the resin suspension gently for 20h at rt. Wash resin bound product (19) with DMF (3 times) and DCM (4 times) and dry in vacuum.

**Step 5:** Suspend complete amount of resin (19) from step 4 in 1.5mL of DCM+HFIP (2+1) and shake for 45min at rt. Filter the remaining solution of and wash resin once with DCM . Repeat cleavage procedure and washing once. Evaporate solvent of the combined filtrates under reduced pressure to afford crude product (20). No further purification.

Step 6: (The following reaction is done in an anhydrous N₂ atmosphere.) Suspend complete amount of crude (20) from step 5 in anhydrous DCM (2.0mL), cooled it to -40°C and added BBr₃ (0.15mL, 1.59mmol). Shake the reaction suspension for 1h at ―40°C, 2h at -25°C and 30min at +5°C. Add dropwise water under vigorous stirring followed by MeOH. Remove solvent under reduced pressure. Purify the crude product by preparative RP HPLC (gradient, water/MeCN 95:5) to obtain 3-[4-(3,5-Dihydroxy-benzoylamino)-butyrylamino]-benzoic acid (21) (8.9mg, 19% over 5 steps) and 3-[4-(3,5-Dihydroxy-benzoylamino)-butyrylamino]-benzoic acid methyl ester (22) (3.0mg, 6% over 5 steps) as white solids both. ¹H NMR (400MHz, CD₃OD) (21): 2.03 (quint, 2 H, *J* = 7.1 Hz); 2.51 (t, 2H, *J*= 7.5Hz); 3.47 (t, 2 H, *J*= 6.7Hz); 6.44 (br.s, 1 H); 6.75 (br.s, 2 H); 7.44 (dd, 1 H, *J₁* = 8.3Hz, *J₂* = 7.8Hz); 7.78 (d, 1 H, *J* = 7.8Hz); 7.85 (d, 1 H, *J* = 8.6Hz); 8.26 (br.s, 1 H); (22): 2.03 (quint, 2 H, *J=* 6.9 Hz); 2.51 (t, 2H, *J*= 7.5Hz); 3.47 (t, 2 H, *J=* 6.9Hz); 3.94 (s, 3 H); 6.43 (br.t, 1 H, *J =* 2.0Hz); 6.74 (d, 2 H, *J* = 2.0Hz); 7.44 (t, 1 H, *J =* 8.1 Hz); 7.77 (d, 1 H, , *J*= 7.6Hz); 7.83 (br.d, 1 H, *J =* 8.1 Hz); 8.28 (br.s, 1 H).

### EXAMPLE 2.

### {4-[4-(3,5-Dihydroxy-benzoylamino)-butyryl]-piperazin-1-yl}-acetic acid (23)

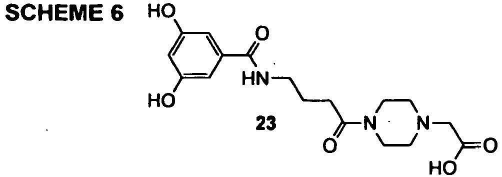

According to the procedure described in EXAMPLE 1 {4-[4-(3,5-Dihydroxy-benzoylamino)-butyryl]-piperazin-l-yl}-acetic acid (23) is obtained as a yellowish oil (6.8mg, 14% over 5 steps). ¹H NMR (400MHz, CD₃OD): 1.96 (quint, 2 H, J = 6.7 Hz); 2.54 (t, 2H, *J=* 6.9Hz); 3.41-3.51 (m, 4 H); 3.90 (br.s, 4 H); 4.12 (s, 2 H); 6.46 (br.s, 1 H); 6.74 (d; 2 H, J = 2.0Hz).

### EXAMPLE 3

### 4-[4-(3,5-Dihydroxy-benzoylamino)-butyrylamino]-benzoic acid (24)

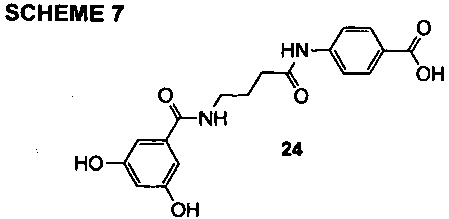

According to the procedure described in EXAMPLE 1 4-[4-(3,5-Dihydroxy-benzoylamino)-butyrylamino]-benzoic acid (24) is obtained as a white solid (1.2mg, 2% over 5 steps). ¹H NMR (400MHz, CD₃OD) 2.03 (quint, 2 H, *J =* 6.9Hz); 2.52 (t, 2H, *J =* 7.8Hz); 3.47 (t, 2H, *J*= 6.6Hz); 6.44 (br.s, 1 H); 6.75 (br.s, 2 H); 7.71 (d, 2 H, *J* = 8.3Hz); 7.99 (d, 2 H, *J*= 8.3Hz).

### EXAMPLE 4

### 5-{4-[2-(2,4-Dimethoxy-phenyl)-acetylamino]-phenyl}-2-methyl-furan-3-carboxylic acid ethyl ester (27) and 5-{4-[2-(2,4-Dimethoxy-phenyl)-acetylamino]-phenyl}-2-methyl-furan-3-carboxylic acid (28)

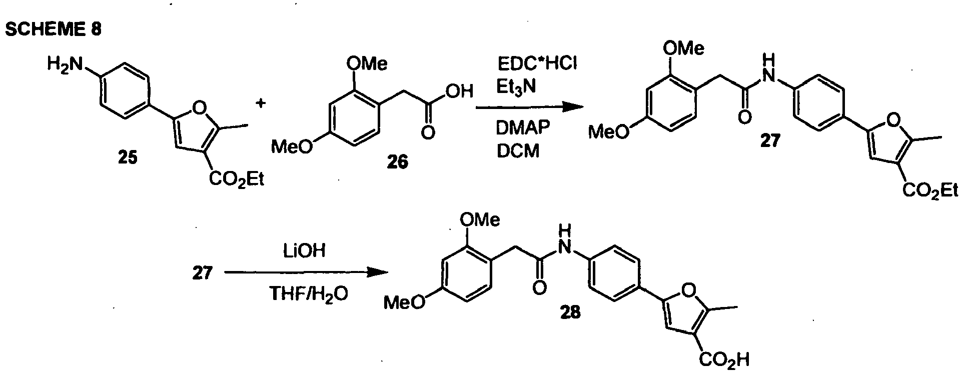

**Step 1:** (The following reaction is done in an anhydrous N₂ atmosphere.) Dissolve EDC hydrochloride (117mg, 0.61mmol) and triethylamine (85µL, 0.61mmol) in anhydrous dichloromethane (2.0mL) and stir for 5min at rt. Add acid (26) (84mg, 0.43mmol) and DMAP (8mg, 0.06mmol) and stir for 10min. Add ethyl ester (25) (100mg, 0.41mmol) and stir the reaction solution overnight at rt. Hydrolize the reaction solution with saturated aqu. NH₄Cl followed by water, separate layers, extract aqu. layer with dichloromethane (3 times) and wash the combined organic layers with water and brine and dry with Na₂SO₄. Remove solvent under reduced pressure. Purify crude product by preparative radial chromatography (silica gel 60PF, EtOAc/CyH 1+1) to obtain 5-{4-[2-(2,4-Dimethoxyphenyl)-acetylamino]-phenyl}-2-methyl-furan-3-carboxylic acid ethyl ester (27) as a yellow solid (153mg, 88%). [K. C. Nicolaou; P. S. Baran; Y.-L. Zhong; K. Sugita; J. Am. Chem. Soc.; 2002; 124*;* 10; 2212-2220]. ¹H NMR (400MHz, CDCl₃): 1.34 (t, 3 H, *J* = 7.2Hz); 2.61 (s, 3 H); 3.63 (s, 2 H); 3.81 (s, 3 H); 3.89 (s, 3 H); 4.28 (q, 2 H, *J* = 7.2Hz); 6.48-6.53 (m, 2 H); 6.77 (s, 1 H); 7.19 (d, 1 H, *J* = 8.1 Hz); 7.42 (d, 2 H, *J* = 8.6Hz); 7.52 (d, 2 H, *J* = 8.8Hz); 7.60 (br.s, 1 H).

**Step 2:** Dissolve 5-{4-[2-(2,4-Dimethoxy-phenyl)-acetylamino]-phenyl}-2-methylfuran-3-carboxylic acid ethyl ester (27) (150mg, 0.35mmol) in MeOH (0.5mL) and THF (8mL) at rt and add 1M aqu LiOH (3.6mL, 3.6mmol). Stir reaction mixture 18h at rt. Quench reaction mixture (cooling bath) with 2M aqu. HCl. Extract the mixture with EtOAc (3x), wash the combined organic layer with brine and dry with Na₂SO₄ to obtain 5-{4-[2-(2,4-Dimethoxy-phenyl)-acetylamino]-phenyl}-2-methyl-furan-3-carboxylic acid (28) (136mg, 97%) as a white solid. ¹H NMR (400MHz, CD₃OD): 2.66 (s, 3 H); 3.65 (s, 2 H); 3.83 (s, 3 H); 3.86 (s, 3 H); 6.54 (dd, 1 H, *J₁* = 8.3Hz, *J₂* = 2.3Hz); 6.59 (d, 1 H, *J =* 2.3Hz); 6.91 (d, 1 H); 7.18 (d, 1 H, *J=* 8.3Hz); 7.64 (s, 4 H).

### EXAMPLE 5

### 5-{4-[2-(2,4-Dihydroxy-phenyl)-acetylamino]-phenyl}-2-methyl-furan-3-carboxylic acid (29)

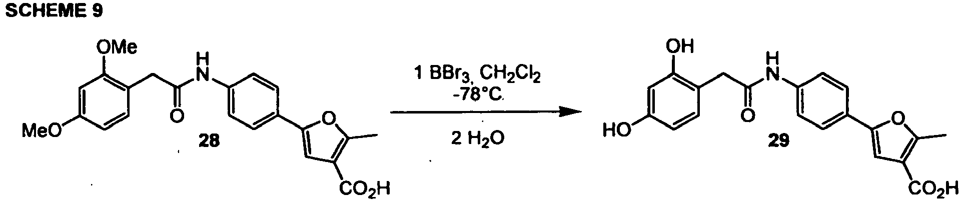

(The following reaction is done in an anhydrous N₂ atmosphere.) Dissolve 5-{4-[2-(2,4-Dimethoxy-phenyl)-acetylamino]-phenyl}-2-methyl-furan-3-carboxylic acid (28) (100mg, 0.25mmol) in anhydrous DCM (2.5mL), cool the solution to ―78°C and add dropwise BBr₃ (95µL, 1.01mmol). Stir the reaction mixture for 30min at ―78°C and after slowly warming up for additional 2h at rt. Add dropwise ice water, separate layers and extract aqu. layer with DCM (3 times). Wash combined organic layer with brine and dry with Na₂SO₄.. Purify the crude product by preparative RP HPLC (gradient, water/CH₃CN 95:5 to 5:95) to obtain 5-{4-[2-(2,4-Dihydroxy-phenyl)-acetylamino]-phenyl}-2-methyl-furan-3-carboxylic acid (29) (30mg, 32%). ¹H NMR (400MHz, CD₃OD): 2.65 (s, 3 H); 3.62 (s, 2 H); 6.33 (dd, 1 H, *J₁* = 8.3Hz, *J₂* = 2.3Hz); 6.39 (d, 1 H, *J* = 2.3Hz); 6.89 (s, 1 H); 7.02 (d,. 1 H, *J* = 8.3Hz); 7.62 (s, 4 H).

### EXAMPLE 6

### 5-{4-[2-(3,5-Dimethoxy-phenyl)-acetylamino]-phenyl}-2-methyl-furan-3-carboxylic acid (30)

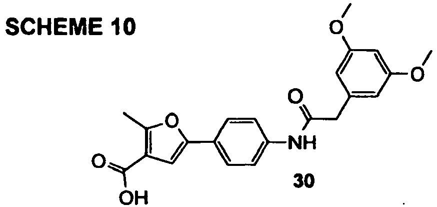

According to the procedure described in EXAMPLE 4 5-{4-[2-(3,5-Dimethoxy-phenyl)-acetylamino]-phenyl}-2-methyl-furan-3-carboxylic acid (30) is obtained as a white solid (138mg, 85% over 2 steps). ¹H NMR (400MHz, CD₃OD): 2.66 (s, 3 H); 3.65 (s, 2 H); 3.81 (s, 6 H); 6.43 (t, 1 H, *J* = 2.0Hz); 6.58 (d, 2 H, *J* = 2.0Hz); 6.92 (s, 1 H); 7.65 (s, 4 H).

### EXAMPLE 7

### 5-{4-[2-(3,5-Dihydroxy-phenyl)-acetylamino]-phenyl}-2-methyl-furan-3-carboxylic acid (31)

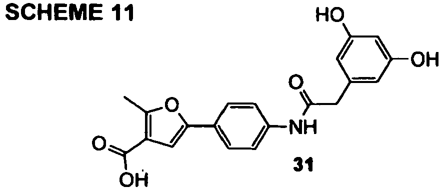

According to the procedure described in EXAMPLE 5 5-{4-[2-(3,5-Dihydroxy-phenyl)-acetylamino]-phenyl}-2-methyl-furan-3-carboxylic acid (31) is obtained as a white solid (57mg, 55% yield). ¹H NMR (400MHz, CD₃OD): 2.66 (s, 3 H); 3.57 (s, 2 H); 6.22 (t, 1 H, *J=* 2.0Hz); 6.35 (d, 2 *H, J=* 2.0Hz); 6.91 (s, 1 H); 7.65 (s, 4 H).

In the following, the preparation of intermediates is described:

### [5-(2-Amino-phenyl)-thiophen-2-yl]-acetic acid methyl ester (43)

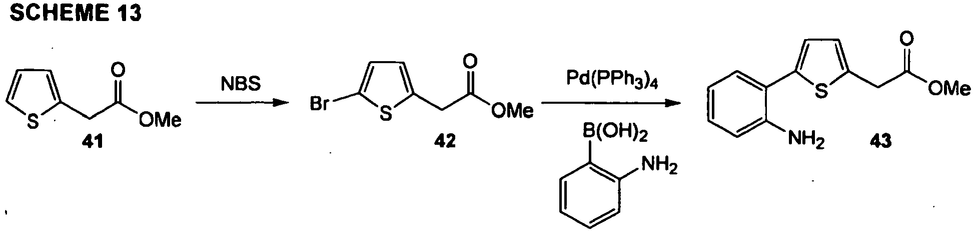

**Step 1:** (The following reaction is carried out in an anhydrous N₂ atmosphere.) Dissolve thiophene-2-yl-acetic acid methyl ester (41) (2.0g, 12.8mmol) in anhydrous chloroform (9.0mL) and glacial acetic acid (9.0mL), add *N*-bromosuccinimide (2.3g, 13.0mmol) in portions and stir the mixture for 3d at rt. Add water to the reaction mixture, separate layers and extract the aqu. layer with dichloromethane. Wash combined organic layer several times with a 1M aqu. NaOH and water and once with brine and dry it with Na₂SO₄. Purify the crude product by preparative radial chromatography (silica gel 60PF, CyH/EtOAc 5+1) to obtain (5-bromo-thiophen-2-yl)-acetic acid methyl ester (42) as a yellow oil (2.46g, 81%) which is used without any further purification. ¹H NMR (400 MHz, CDCl₃): 3.71 (s, 3 H); 3.75 (s, 2 H); 6.67 (d, 1 H, *J*= 3.8 Hz); 6.88 (d, 1 H, *J*= 3.8 Hz).

**Step 2:** (The following reaction is carried out in an N₂ atmosphere.) Ethanol (3.7mL), tetrakis-(triphenylphosphine)-palladium(0) (289mg, 0.25mmol) and Na₂CO₃ decahydrate (4.0g, 14.0mmol) dissolved in water (5.2mL) are subsequently added to a solution of 2-amino-benzeneboronic acid hydrochloride (910mg, 5.25mmol) in toluene (52mL). Degas the reaction mixture carefully (5 times) and flush with N₂ again. A solution of (5-bromothiophen-2-yl)-acetic acid methyl ester (42) (1.17 g, 5.0 mmol) in toluene (4.5 mL) is added. Degas the mixture again (5 times) and stir for 22h at 100°C. Partition the reaction solution between EtOAc and brine and extract the separated aqueous layer with EtOAc (3 times). Wash combined organic layer with water and brine and dry it with Na₂SO₄. Purify the crude product by preparative radial chromatography (silica gel 60PF, CyH/EtOAc 5+1) to obtain [5-(2-amino-phenyl)-thiophen-2-yl]-acetic acid methyl ester (43) as a brown oil (634 mg, 51%). ¹HNMR (400 MHz, CDCl₃): 3.73 (s, 3 H); 3.83 (s, 2 H); 3.92-4.07 (br.s, 2 H); 6.74 (d, 1 H); 6.76 (td, 1 H*, J*₁ = 7.6 Hz, *J*₂ = 1.3 Hz); 6.92 (d, 1 H, *J* = 3.5 Hz); 7.02 (d, 1 H, *J =* 3.5 Hz); 7.11 (td, 1 H, *J*₁ = 7.6 Hz_{,} *J*₂ = 1.5 Hz); 7.23 (dd, 14 H, *J*₁ = 7.6 Hz, *J*₂ = 1.5 Hz).

### 5-(2-Amino-phenyl)-thiophene-2-carboxylic acid methyl ester (45)

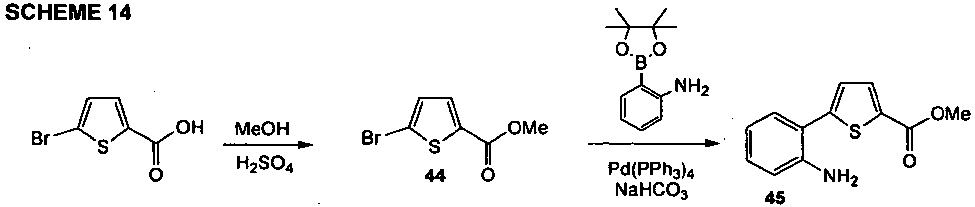

**Step 1:** Dissolve 5-bromo-thiophene-2-carboxylic acid (1.50g, 7.24mmol) in MeOH (10mL) and add conc. sulfuric acid (0.39mL, 7.24mmol). Stir the reaction mixture for 20h at 75°C. Cool mixture to rt, remove solvent under reduced pressure and resolve the residue in EtOAc. Wash this organic layer 3 times with 5% aqu. Na₂CO₃ and extract the combined aqueous layer with EtOAc. Wash the combined organic layers with brine and dry with Na₂SO₄. Remove solvent under reduced pressure and dry the residue without further purification in oil pump vacuum to obtain ester (44) as a white solid (1.48g, 92%). ¹H NMR (400 MHz, CDCl₃): 3.85 (s, 3 H); 7.05 (d, 1 H, *J* = 4.0 Hz); 7.53 (d, 1 H, *J* = 4.0 Hz).

**Step 2:** (The following reaction is carried out in an N₂ atmosphere.) Dissolve tetrakis-(triphenylphosphine)-palladium(0) (510mg, 0.45 mmol) and ester (44) (1.97g, 8.91mmol) in DME (16mL), degas the reaction mixture carefully (5 times) and flush with N₂. Add 2-(4,4,5,5-tetramethyl-[1,3,2]dioxaborolan-2-yl)-phenylamine (2.15g, 9.80mmol) and a 1M aqueous NaHCO₃ solution (27.0mL, 27.0mmol), degas the reaction mixture again carefully (5 times) and flush with N₂. Stir the mixture for 18h at 95°C. Cool the mixture to rt, partition between EtOAc and water and extract the separated aqueous layer with EtOAc (3 times). Wash combined organic layer with brine and dry it with Na₂SO₄. Purify the crude product by flash chromatography (silica gel 60, CyH/EtOAc 5+1 ] to obtain 5-(2-aminophenyl)-thiophene-2-carboxylic acid methyl ester (45) as a yellow solid (1.41g, 67%). ¹H NMR (400 MHz, CDCl₃): 3.88 (s, 3 H); 4.00 (br.s, 2 H); 6.73-6.82 (m, 2 H); 7.13-7.21 (m, 2 H); 7.26 (dd, 1 H, *J*₁ = 7.6 Hz, *J*₂ = 1.0 Hz); 7.78 (d, 1 H, *J* = 3.8 Hz).

### 5-(4-Amino-phenyl)-2-methyl-furan-3-carboxylic acid methyl ester (47)

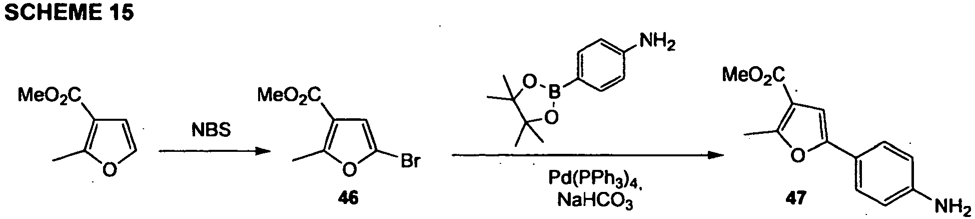

**Step 1:** (The following reaction is carried out under exclusion of light.) Dissolve 2-methyl-furan-3-carboxylic acid methyl ester (3.60mL, 28.5mmol) in chloroform (20mL) and glacial acetic acid (20mL) and add NBS (6.90g, 38.8mmol) portionwise in between a period of 95min. Stir the reaction suspension for additional 19h at rt. Add water to the reaction mixture and extract the aqu. layer with dichloromethane (2 times), wash the combined organic layer with 2M aqu. NaOH, water (3 times) and brine and dry it with Na₂SO₄ to obtain 5-bromo-2-methyl-furan-3-carboxylic acid methyl ester (46) (4.90g, 78%) as a red brown oil. No further purification. ¹H NMR (400 MHz, CDCl₃): 2.54 (s, 3 H); 3.80 (s, 3 H); 6.53 (s, I H).

**Step 2:** (The following reaction is carried out in a N₂ atmosphere.) Dissolve Pd(PPh₃)₄ (1.26g, 1.09mmol) and 5-bromo-2-methyl-furan-3-carboxylic acid methyl ester (46) (4.77g, 21.77mmol) in DME (116mL) and stir for 15min at rt. Add 4-(4,4,5,5-tetramethyl-[1,3,2]dioxaborolan-2-yl)-phenylamine (5.25g, 23.96mmol) followed by an aqu. 1M sodium bicarbonate solution (65.4mL, 65.3mmol). Degas the reaction mixture carefully, flush with N₂ (5 times) and stir for 4h at 95°C (reflux). Cool reaction mixture to rt, remove organic solvent under reduced pressure and partition the residue between water and EtOAc. Extract the aqu. layer with EtOAc (3 times), wash the combined organic layer with water and brine and dry it with Na₂SO₄. Purify the obtained crude product by flash chromatography (silica gel 60, EtOAc/CyH 1+2) to obtain 5-(4-amino-phenyl)-2-methylfuran-3-carboxylic acid methyl ester (47) (2.35g, 46%) as a yellow-brown solid. ¹H NMR (400 MHz, CDCl₃): 2.60 (s, 3 H); 3.74 (br.s, 2 H); 3.82 (s, 3 H); 6.64 (s, 1 H); 6.67 (dt, 1 H, *J*₁ = 8.6 Hz, *J*₂ = 2.3 Hz); 7.42 (dt, 2 H, *J*₁ = 8.8 Hz, *J*₂ = 2.3 Hz).

### 2-Thiophene-2-yl-phenylamine (48)

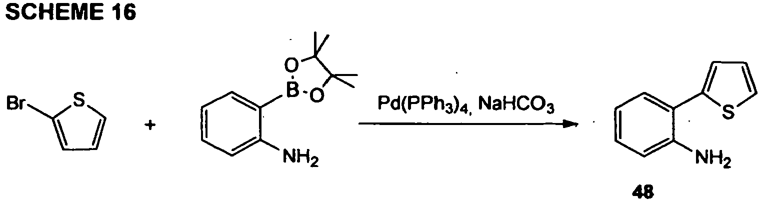

(The following reaction is carried out in an N₂ atmosphere.) Dissolve tetrakis-(triphenylphosphine)-palladium(0) (297mg, 0.26 mmol) and 2-bromo-thiophene (837mg, 5.13mmol) in DME (42mL), degas the reaction mixture carefully (5 times) and flush with N₂. After 10min stirring add 2-(4,4,5,5-tetramethyl-[1,3,2]dioxaborolan-2-yl)-phenylamine (1.24g, 5.64mmol) and a 1M aqueous NaHCO₃ solution (15.4mL, 15.4mmol), degas the reaction mixture again carefully (5 times) and flush with N₂. Stir the mixture for 3h at 95°C. Cool mixture to rt, remove solvent under reduced pressure and partition the residue between EtOAc and water. Extract the separated aqueous layer with EtOAc (3 times). Wash combined organic layer with brine and dry it with Na₂SO₄. Purify the crude product by flash chromatography (silica gel 60, CyH/EtOAc 15+1] to obtain 2-thiophene-2-yl-phenylamine (48) as a brown solid (825mg, 92%). ¹H NMR (400 MHz, CDCl₃): 4.40-6.00 (m, 2 H); 6.88 (td, 1 H, *J*₁ = 7.6 Hz, *J*₂ = 1.0 Hz); 6.93 (dd, 1 H, *J*₁ = 8.0 Hz, *J*₂ = 1.0 Hz); 7.07 (dd, 1 H, *J*₁ = 5.3 Hz, *J*₂ = 3.5 Hz); 7.17 (td, 1 H, *J*₁ = 8.0 Hz, *J*₂ = 1.5 Hz) 7.22 (dd, 1 H, *J*₁ = 3.5 Hz, *J*₂ = 1.3 Hz); 7.30 (dd, 1 H, *J*₁ = 7.6 Hz, *J*₂ = 1.5 Hz); 7.33 (dd, 1 H, *J*₁ = 5.3 Hz, *J*₂ = 1.3 Hz).

### [5-(3-Amino-phenyl)-thiophen-2-yl]-acetic acid methyl ester (50)

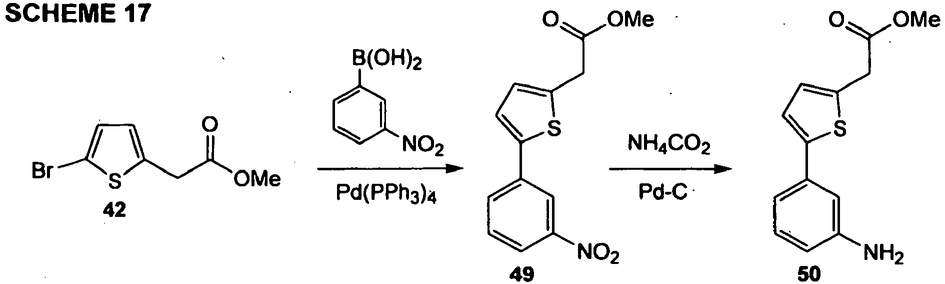

**Step 1:** (The following reaction is carried out in an N₂ atmosphere.) Dissolve Tetrakis-(triphenylphosphine)-palladium(0) (1.12g, 0.97 mmol) and ester (42) (4.57g, 19.44mmol) in toluene (200mL) and EtOH (20.0mL), degas the reaction mixture carefully (5 times) and flush with N₂. Add 3-nitrophenylboronic acid (3.57g, 21.38mmol) and a 3 M aqueous Na₂CO₃ solution (18.1mL, 54.3mmol), degas the reaction mixture again carefully (5 times) and flush with N₂. Stir the mixture for 18h at 100°C. Partition the reaction solution between EtOAc and water and extract the separated aqueous layer with EtOAc (3 times). Wash combined organic layer with brine and dry it with Na₂SO₄. Purify the obtained crude product by preparative radial chromatography (silica gel, EtOAc/CyH 1+5) to obtain [5-(3-nitro-phenyl)-thiophen-2-yl]-acetic acid methyl ester (49) as a yellow solid (3.15g, 58%). ¹H NMR (400 MHz, CDCl₃): 3.75 (s, 3 H); 3.85 (s, 2 H); 6.94 (br.d, 1 H, *J*= 3.8 Hz); 7.27 (d, 1 H, *J*= 3.8 Hz); 7.51 (t, 1 H, *J*= 8.0 Hz); 7.84 (ddd, 1 H, *J*₁ = 7.8 Hz, *J*₂ = 1.5 Hz, *J*₃ = 0.8 Hz); 8.08 (ddd, 1 H, *J*₁ = 8.3 Hz, *J*₂ = 2.1 Hz, *J*₃ = 1.0 Hz); 8.39 (t, 1 H, *J*= 1.9 Hz).

**Step 2:** (The following reaction is done in an N₂ atmosphere.) Dissolve [5-(3-Nitrophenyl)-thiophen-2-yl]-acetic acid methyl ester (49) (3.15g, 11.35mmol) in MeOH (225mL) and add Pd on carbon (10% (w/w) Pd content, 1.20g, 1.13mmol) followed by NH₄CO₂H (7.15g, 113.4mmol) at rt. Degas the reaction mixture carefully (flush with N₂) and stir it for 22h at rt. Filtrate reaction mixture through a short pad of celite and remove solvent. Purify the obtained crude product by preparative radial chromatography (silica gel, EtOAc/CyH 1+3) to obtain [5-(3-Amino-phenyl)-thiophen-2-yl]-acetic acid methyl ester (50) (2.08g, 74%) as a yellow solid. ¹H NMR (400 MHz, CDCl₃): 3.73 (s, 3 H); 3.81 (s, 2 H); 6.59 (dd, 1 H, *J*₁ = 7.8 Hz, *J*₂ = 2.0 Hz); 6.86 (br.d, 1 H, *J* = 3.5 Hz); 6.88 (t, 1 H, *J=* 1.9 Hz); 6.97 (br.d, 1 *H, J=* 7.6 Hz); 7.09 (d, 1 H, *J*= 3.5 Hz); 7.13 (t, 1 *H, J=* 7.7 Hz).

### 5-(6-Amino-pyridin-3-yl)-2-methyl-furan-3-carboxylic acid methyl ester (51)

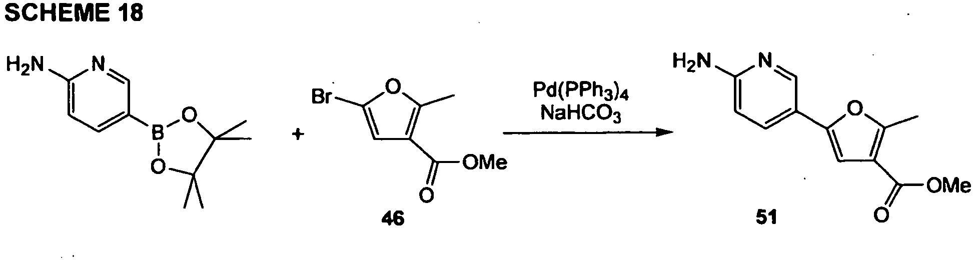

**Step 1:** (The following reaction is carried out in an N₂ atmosphere.) Dissolve 5-(4,4,5,5-tetramethyl-[1,3,2]dioxaborolan-2-yl)-pyridin-2-ylamine (500mg, 2.27mmol) and tetrakis-(triphenylphosphine)-palladium(0) (114mg, 0.10mmol) in DME (12.5mL). Degas the reaction mixture carefully (5 times) and flush with N₂. Stir 10 min at rt, add 5-bromo-2-methyl-furan-3-carboxylic acid methyl ester (46) (433mg, 2.00mmol) and a 1M aqueous NaHCO₃ solution (5.9mL, 5.9mmol), degas the reaction mixture again carefully (3 times) and flush with N₂. Stir the mixture for 3h at 95°C. Cool mixture to rt, dilute with EtOAc and filtrate over a short pad of silica. Remove solvent under reduced pressure and purify the crude product by preparative radial chromatography (silica gel 60PF, CyH/EtOAc 2+1] to obtain the 5-(6-amino-pyridin-3-yl)-2-methyl-furan-3-carboxylic acid methyl ester (51) as yellow solid (382mg, 82%). ¹H NMR (400 MHz, CDCl₃): 2.61 (s, 3 H); 3.82 (s, 3 H); 4.55 (br. s, 2 H); 6.51 (d, 1 H, *J* = 8.6 Hz); 6.69 (s, 1 H); 7.65 (dd, 1 H, *J*₁ = 8.6 Hz, *J*₂ = 2.3 Hz); 8.35 (d, 1 H, *J* = 2.3 Hz).

### 2',4'-Dimethoxy biphenyl-3-carbonyl chloride (54)

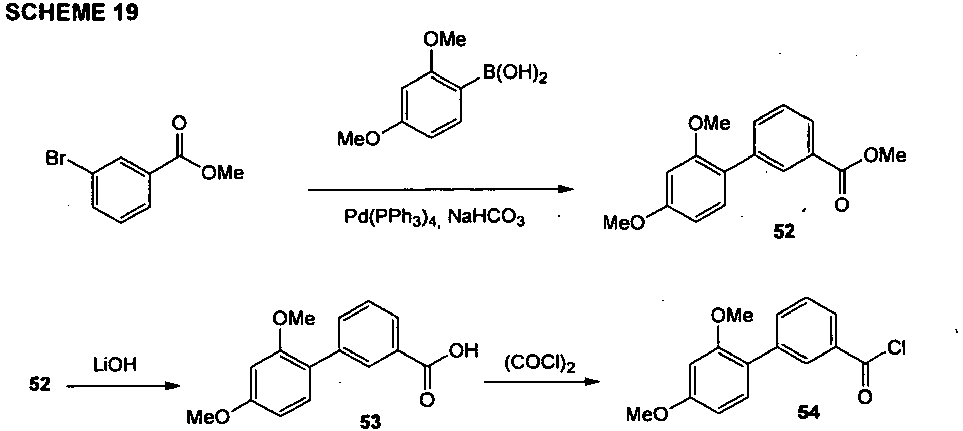

**Step 1:** (The following reaction is carried out in an N₂ atmosphere.) Dissolve tetrakis-(triphenylphosphine)-palladium(0) (410mg, 0.35mmol) and methyl 3-bromobenzoate (2.51g, 11.7mmol) in DME (22mL).Add 2,4-dimethoxyphenylboronic acid (2.50g, 13.7mmol) and a 1M aqueous NaHCO₃ solution (35.5mL, 35.5mmol), degas the reaction mixture carefully (5 times) and flush with N₂. Stir the mixture for 2.5h at 100°C. Cool the reaction solution to rt, partition between EtOAc and water and extract the separated aqueous layer several times with EtOAc. Wash combined organic layer with brine and dry it with Na₂SO₄. Purify the crude product by flash chromatography (silica gel 60, CyH/EtOAc 7+1 then 4+1] to obtain the biphenyl (52) as a yellow oil (2.95g, 93%). ¹H NMR (400 MHz, CDCl₃): 3.78 (s, 3 H); 3.84 (s, 3 H); 3.90 (s, 3 H); 6.55 (s, 1 H); 6.56 (dd, 1 H, *J*₁ = 7.6 Hz, *J*₂ = 2.3 Hz); 7.23 (dd, 1 H, *J*₁ = 7.6 Hz, *J*₂ = 1.0 Hz); 7.43 (t, 1 H, *J =* 7.8 Hz); 7.68 (dt, 1 H, *J*₁ = 7.8 Hz, *J*₂ = 1.5 Hz); 7.94 (dt, 1 H, *J*₁ = 7.8 Hz, *J*₂ = 1.5 Hz); 8.14 (t, 1 H, *J* = 1.5 Hz).

**Step 2:** Dissolve 2',4'-dimethoxy-biphenyl-3-carboxylic acid methyl ester (52) (2.95g, 10.8mmol) in MeCN (110mL) at rt and add 1M aqu LiOH (154mL, 154mmol). Stir reaction mixture overnight at rt and 1h at 50°C. Quench reaction mixture (cooling bath) with 1 M aqu. HCl (160ml, to get pH ca. 3). Extract the mixture with EtOAc (3x), wash the combined organic layer with brine and dry with Na₂SO₄ to obtain 2',4'-dimethoxy-biphenyl-3-carboxylic acid (53) as a white solid (2.59g, 93%). ¹H NMR (400 MHz, CDCl₃): 3.79 (s, 3 H); 3.84 (s, 3 H); 6.55 (s, 1 H); 6.54-6.58 (m, 1 H); 7.23-7.26 (m, 1 H); 7.46 (t, 1 H, *J=* 7.8 Hz); 7.73 (dt, 1 H, *J*₁ = 7.8 Hz, *J*₂ = 1.5 Hz); 7.98 (dt, 1 H *, J*₁ = 7.8 Hz, *J*₂ = 1.5 Hz); 8.19 (t, 1 H, *J* = 1.5 Hz).

**Step 3:** (The following reaction is done in an anhydrous N₂ atmosphere.) Dissolve 2',4'-dimethoxy-biphenyl-3-carboxylic acid (53) (2.59g, 10.0mmol)) in anhydrous dichloromethane (71 mL) and add anhydrous DMF (4mL, cat. amount). Then add slowly oxalyl chloride (1.3mL, 15.0mmol) by keeping temperature at ca. 15°C (water bath) and stir the yellow solution for additional 3h at rt. Remove solvent under reduced pressure and dry the residue in vacuum to obtain crude 2',4'-dimethoxy biphenyl-3-carbonyl chloride (54) as a yellow solid (3.2g, quant.). No further purification.

### (2',4'-Dimethoxy biphenyl-3-yl)-acetic acid (57)

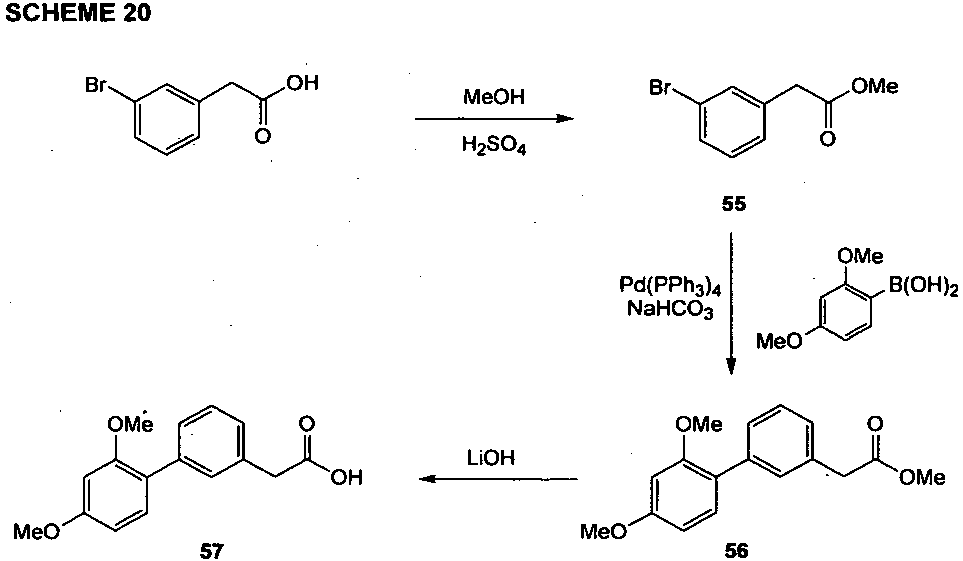

Step 1: Dissolve (3-bromo-phenyl)-acetic acid (4.00g, 18.6mmol) in MeOH (74mL) and add conc. sulfuric acid (1.00mL, 18.6mmol). Stir the reaction mixture for 65h at 45°C. Cool mixture to rt, remove solvent under reduced pressure and partition the residue between EtOAc and sat. aqueous NaHCO₃ solution. Extract the aqueous layer 3 times with EtOAc and wash the combined organic layers with brine and dry with Na₂SO₄. Remove solvent under reduced pressure to obtain (3-bromo-phenyl)-acetic acid methyl ester (55) as a colorless oil (4.25g, 99%). ¹H NMR (400 MHz, CDCl₃): 3.58 (s, 2 H); 3.69 (s, 3 H); 7.17 (t, 1 H, *J* = 7.6 Hz); 7.18-7.21 (m, 1 H); 7.39 (dt, 1 H, *J*₁ = 6.8 Hz, *J*₂ = 2.0 Hz); 7.41-7.43 (m, 1 H).

**Step 2:** (The following reaction is carried out in an N₂ atmosphere.) Dissolve (3-bromophenyl)-acetic acid methyl ester (55) (2.00g, 8.73mmol) and tetrakis-(triphenylphosphine)-palladium(0) (303mg, 0.26mmol) and in DME (18mL). Add 2,4-dimethoxyphenylboronic acid (1.84g, 10.12mmol) and a 1M aqueous NaHCO₃ solution (26mL, 26mmol), degas the reaction mixture carefully (3 times) and flush with N₂. Stir the mixture overnight at 100°C. Cool the reaction solution to rt , partition between EtOAc and water and extract the separated aqueous layer several times with EtOAc. Wash combined organic layer with brine and dry it with Na₂SO₄. Purify the crude product by preparative radial chromatography (silica gel 60PF, CyH/EtOAc 3+1] to obtain the (2',4'-dimethoxy biphenyl-3-yl)-acetic acid methyl ester (56) as a yellow oil (2.25g, 90%). ¹H NMR (400 MHz, CDCl₃): 3.65 (s, 2 H); 3.68 (s, 3 H); 3.77 (s, 3 H); 3.83 (s, 3 H); 6.54 (s, 1 H); 6.55 (dd, 1 H, *J*₁ = 7.0 Hz, *J*₂ = 2.3 Hz); 7.18-7.21 (m, 1 H); 7.22 (dd, 1 H, *J*₁ = 6.8 Hz, *J*₂ = 2.3 Hz); 7.32 (t, 1 H, *J* = 8.0 Hz); 7.39 (d, 1 H, *J* = 1.5 Hz); 7.38-7.41 (m, 1 H).

Step 3: Dissolve (2',4'-dimethoxy biphenyl-3-yl)-acetic acid methyl ester (56) (2.25g, 7.86mmol) in MeCN (79mL) at rt and add 1M aqu LiOH (40mL, 40mmol). Stir reaction mixture overnight at rt. Quench reaction mixture (cooling bath) with 1M aqu. HCl (to get pH ca. 3) and remove MeCN under reduced pressure. Dilute aqueous residue with EtOAc, separate layers and extract the aqueous layer several times with EtOAc. Wash the combined organic layer with brine and dry with Na₂SO₄ to obtain (2',4'-dimethoxy-biphenyl-3-yl)-acetic acid (57) as a yellow oil (2.16g, quant.). ¹H NMR (400 MHz, CDCl₃): 3.67 (s, 2 H); 3.76 (s, 3 H); 3.83 (s, 3 H); 6.53 (s, 1 H); 6.52-6.56 (m, 1 H); 7.18-7.22 (m, 1 H); 7.22 (dd, 1 H, *J*₁ = 7.3 Hz, *J*₂ = 1.5 Hz); 7.33 (t, 1 H, J= 7.8 Hz), 7.39-7.42 (m, 2 H).

### 3',5'-Dimethoxy biphenyl-3-carbonyl chloride (60)

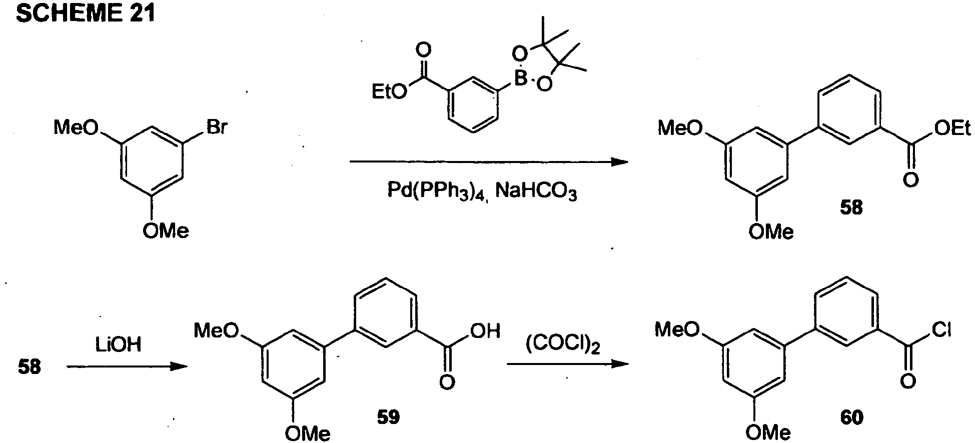

**Step 1:** (The following reaction is carried out in an N₂ atmosphere.) Dissolve tetrakis-(triphenylphosphine)-palladium(0) (410mg, 0.35mmol) and 1-bromo-3,5-dimethoxybenzene (1.80g, 8.29mmol) in DME (17mL).Add 3-(4,4,5,5-tetramethyl-[1,3,2]dioxaborolan-2-yl)-benzoic acid ethyl ester (2.66g, 9.62mmol) and a 1M aqueous NaHCO₃ solution (25mL, 25mmol), degas the reaction mixture carefully (5 times) and flush with N₂. Stir the mixture for overnight at 100°C. Cool the reaction solution to rt, partition between EtOAc and water and extract the separated aqueous layer several times with EtOAc. Wash combined organic layer with brine and dry it with Na₂SO₄. Purify the crude product by preparative radial chromatography (silica gel 60PF, CyH/EtOAc 10+1] to obtain 3',5'-dimethoxy-biphenyl-3-carboxylic acid ethyl ester (58) as a yellow oil (2.04g, 86%). ¹H NMR (400 MHz, CDCl₃): 1.37 (t, 3 H, *J*= 7.1 Hz); 3.84 (s, 6 H); 4.39 (q, 2 H, *J=* 7.1 Hz); 6.48 (t, 1 H, *J*= 2.3 Hz); 6.73 (d, 2 H, *J=* 2.3 Hz); 7.48 (t, 1 H, *J=* 7.6 Hz); 7.74 (d, 1 H, *J*₁ = 7.6 Hz); 8.01 (dt, 1 H, *J*₁ = 7.6 Hz, *J*₂ = 1.5 Hz); 8.23 (t, 1 H, *J*= 1.5 Hz).

**Step 2:** Dissolve 3',5'-dimethoxy-biphenyl-3-carboxylic acid ethyl ester (58) (2.04g, 7.13mmol) in MeCN (71mL) and add 1M aqu LiOH (36mL, 36mmol). Stir reaction mixture overnight at rt and remove solvent under reduced pressure. Add 1M aqu. HCl (to get pH ca. 3) and EtOAc, separate layers and extract the aqueous layer with EtOAc (several times). Wash the combined organic layer with brine and dry it with Na₂SO₄ to obtain 3',5'-dimethoxy-biphenyl-3-carboxylic acid (59) as a white solid (1.73g, 93%). ¹H NMR (400 MHz, CDCl₃): 3.85 (s, 6 H); 6.49 (t, 1 H, *J*= 2.3 Hz); 6.74 (d, 2 H, *J*= 2.3 Hz); 7.52 (t, 1 H, *J=* 7.8 Hz); 7.80 (ddd, 1 H, *J*₁ = 7.8 Hz, *J*₂ = 1.5 Hz, J₃ = 1.3 Hz); 8.07 (dt, 1 H, *J*₁ = 7.8 Hz, *J*₂ = 1.3 Hz); 8.30 (t, 1 H, *J*= 1.5 Hz).

**Step 3:** (The following reaction is done in an anhydrous N₂ atmosphere.) Dissolve 3',5'-dimethoxy-biphenyl-3-carboxylic acid (59) (950mg, 3.68mmol) in anhydrous dichloromethane (25mL) and add anhydrous DMF (3mL, until clear solution occurs). Then add slowly oxalyl chloride (0.48mL, 5.52mmol) by keeping temperature at ca. 15°C (water bath) and stir for additional 3h at rt. Remove solvent under reduced pressure and dry the residue in vacuum to afford crude 3',5'-dimethoxy biphenyl-3-carbonyl chloride (60) as a yellow solid (1.22g, quant.). No further purification.

### EXAMPLE 8

### (5-{2-[(2',4'-Dimethozy-bip6enyl-3-carbonyl)-amino]-phenyl}-thiophen-2-yl) acetic acid methyl ester (61) and (5-{2-[(2',4'-Dimethozy-biphenyl-3-carbonyl)-amino]-phenyl}-thiophen-2-yl) acetic acid (62)

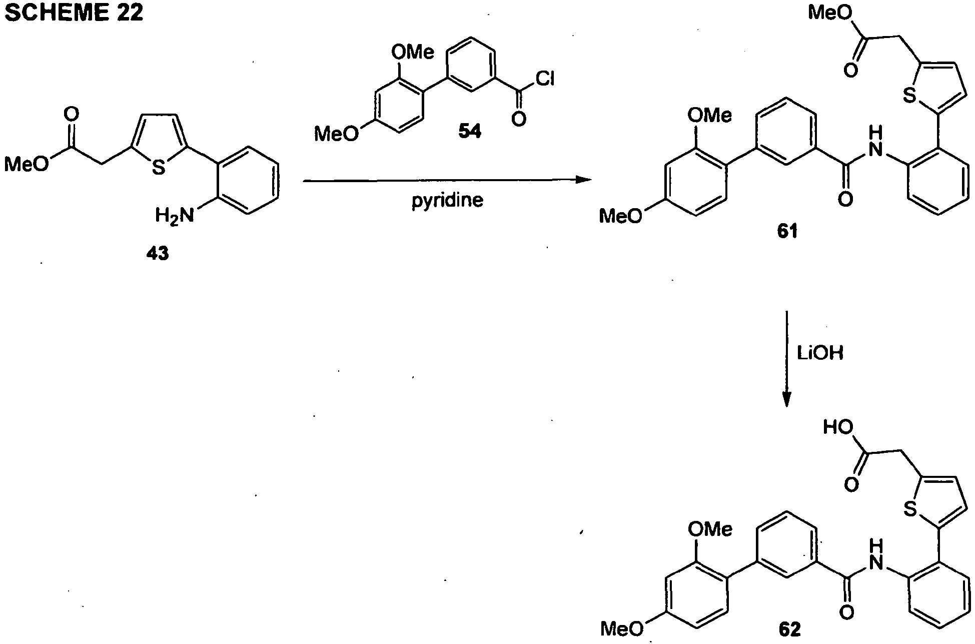

**Step 1: (**The following reaction is carried out in an N₂ atmosphere.) Dissolve [5-(2-amino-phenyl)-thiophen-2-yl]-acetic acid methyl ester (43) (62,0mg, 0.25mmol) in anhydrous dichloromethane (3.7mL) and anhydrous pyridine (1.1mL) and cool the reaction mixture to 0°C. Add a solution of 2',4'-dimethoxy-biphenyl-3-carbonyl-chloride (54) (69mg, 0.25mmol) in dichloromethane (1.5mL) and stir the reaction mixture for 1h at 0°C and overnight at rt. Pour the reaction mixture into ice cooled 1M aqu. HCl,separate layers, extract the aqueous layer with dichloromethane (3x), wash the combined organic layer with brine and dry with Na₂SO₄. Purify the crude product by preparative radial chromatography (silica gel 60PF, EtOAc/CyH 3+1) to obtain (5-{2-[(2',4'-dimethoxy-biphenyl-3-carbonyl)-amino]-phenyl}-thiophen-2-yl) acetic acid methyl ester (61) as a yellow solid (4.2mg, 37%). ¹H NMR (400 MHz, CDCl₃): 3.71 (s, 3 H); 3.76 (s, 3 H); 3.82 (s, 2 H); 3.85 (s, 3 H); 6.55 (d, 1 H, *J* = 2.3 Hz); 6.57 (dd, 1 H, *J*₁ = 8.0 Hz, *J*₂ = 2.3 Hz); 6.95 (d, 1 H, *J* = 3.5 Hz); 7.02 (d, 1 H, *J* = 3.5 Hz); 7.15 (td, 1 H, *J*₁ = 7.6 Hz, *J*₂ = 1.0 Hz); 7.21 (d, 1 H, *J* = 8.0 Hz); 7.39 (d, 1 H, *J* = 7.6 Hz); 7.40 (d, 1 H, *J* = 7.6 Hz); 7.44 (t, 1 H, *J* = 7.6 Hz); 7.61-7.67 (m, 2 H); 7.89 (t, 1 H, *J* = 1.5 Hz); 8.34 (br. s, 1 H); 8.50 (d, 1 H, *J* = 8.0 Hz).

**Step 2:** Dissolve 5-{2-[(2',4'-dimethoxy-biphenyl-3-carbonyl)-amino]-phenyl}-thiophen-2-yl) acetic acid methyl ester (61) (45.0mg, 0.09mmol) in MeCN (0.9mL) and add 1M aqu LiOH (0.5mL, 0.5mmol). Stir reaction mixture overnight at rt. Add dropwise 1M aqu. HCl (to get pH ca. 3) and remove solvent under reduced pressure. Dissolve residue in EtOAc and water and extract the separated aqueous layer several times with EtOAc. Wash combined organic layer with brine and dry it with Na₂SO₄ to obtain (5-{2-[(2',4'-dimethoxy-biphenyl-3-carbonyl)-amino]-phenyl}-thiophen-2-yl) acetic acid (62) as yellow solid (48mg, quant.). No further purification. ¹H NMR (400 MHz, CD₃OD): 3.84 (br. s, 5 H); 3.89 (s, 3 H); 6.65-6.70 (m, 2 H); 6.98 (d, 1 H, *J*= 3.5 Hz); 7.18 (d, 1 H, *J=* 3.5 Hz); 7.31 (d, 1 H, *J*= 8.0 Hz); 7.37 (td, 1 H, *J*₁ = 7.6 Hz, *J*₂ = 1.3 Hz); 7.43 (td, 1 H, *J*₁ = 7.8 Hz, *J*₂ = 1.5 Hz); 7.52 (t, 1 H, *J*= 7.8 Hz); 7.63 (dd, 1 H, *J*₁ = 7.6 Hz, *J*₂ = 1.5 Hz); 7.70-7.76 (m, 2 H); 7.83 (d, 1 H, *J*= 7.8 Hz); 8.01 (br. s, 1 H).

### EXAMPLE 9

### (5-{2-[(2',4'-Dihydroxy-bipbenyl-3-carbonyl)-amino]-phenyl}-thiophen-2-yl) acetic acid (63) and (5-{2-[(2',4'-Dihydroxy-biphenyl-3-carbonyl)-amino]-phenyl}-thiophen-2-yl) acetic acid methyl ester (64)

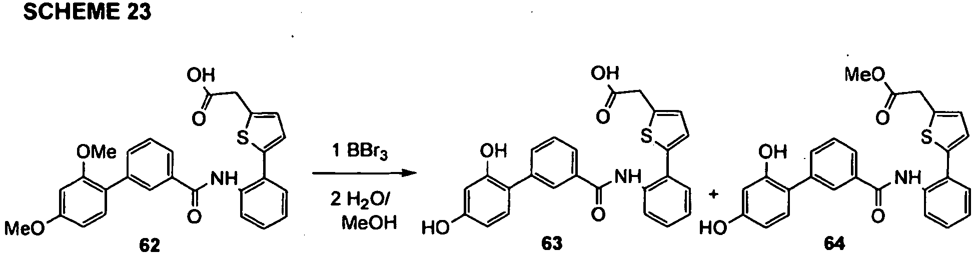

(The following reaction is carried out in an N₂ atmosphere.) Dissolve (5-{2-[(2',4'-dimethoxy-biphenyl-3-carbonyl)-amino]-phenyl}-thiophen-2-yl) acetic acid (62) (48.0mg, 0.10mmol) in anhydrous dichloromethane (1.5mL), cool the solution to -78°C and add dropwise 1M BBr₃ solution in dichloromethane (0.63mL, 0.63mmol). Stir the reaction mixture for 30min at -78°C and after slowly warming up for additional 2h at rt. Cool to 0°C, add dropwise water followed under vigorous stirring by MeOH. Concentrate the resulting mixture under reduced pressure. Purify the crude product by preparative RP HPLC (gradient, water/CH₃CN 95:5 to 5:95) to obtain (5-{2-[(2',4'-dihydroxy-biphenyl-3-carbonyl)-amino]-phenyl}-thiophen-2-yl) acetic acid (63) (3.7mg, 8%) and (5-{2-[(2',4'-dihydroxy-biphenyl-3-carbonyl)-amino]-phenyl}-thiophen-2-yl) acetic acid methyl ester (64) (7.1 mg, 15%) both as yellow solids. ¹H NMR compound (63) (400 MHz, CD₃OD): 3.85 (s, 2 H); 6.42-6.47 (m, 2 H); 6.98 (d, 1 H, *J*= 3.5 Hz);7.1 8 (d, 1 H, *J*= 7.6 Hz); 7.19 (d, 1 H, *J*= 3.5 Hz); 7.36 (td, 1 H, *J*₁ = 7.6 Hz, *J*₂ = 1.3 Hz), 7.43 (td, 1 H*, J*₁= 7.8 Hz, *J*₂ = 1.3 Hz); 7.51 (t, 1 H, *J =* 7.8 Hz); 7.62 (dd, 1 H, *J*₁ = 7.6 Hz, *J*₂ = 1.3 Hz); 7.75-7.81 (m, 3 H); 8.08 (br. s, 1 H), ¹H NMR compound (64) (400 MHz, CD₃OD): 3.66 (s, 3 H); 3.89 (s, 2 H); 6.44 (dd, 1 H, *J*₁ = 7.0 Hz, *J*₂ = 2.3 Hz); 6.45 (s, 1 H); 6.97 (d, 1 H, *J=* 3.5 Hz); 7.18 (d, 1 H, *J=* 7.0 Hz); 7.19 (d, 1 H, *J*= 3.5 Hz); 7.37 (td, 1 H, *J*₁ = 7.6 Hz, *J*₂ *=* 1.3 Hz); 7.43 (td, 1 H, *J*₁ = 7.6 Hz, *J*₂ = 1.5 Hz); 7.51 (t, 1 H, *J =* 7.8 Hz); 7.63 (dd, 1 H, *J*₁ = 7.8 Hz, *J*₂ = 1.3 Hz); 7.72 (d, 1 H, *J=* 7.8 Hz); 7.80 (d, 1 H, *J*= 8.0 Hz); 7.81 (t, 1 H, *J*= 7.6 Hz); 8.07 (s, 1 H).

### EXAMPLE 10

### (5-{2-[(2',4'-Dimethoxy-biphenyl-3-carbonyl)-amino]-phenyl}-thiophene-2-carboxylic acid methyl ester (65)

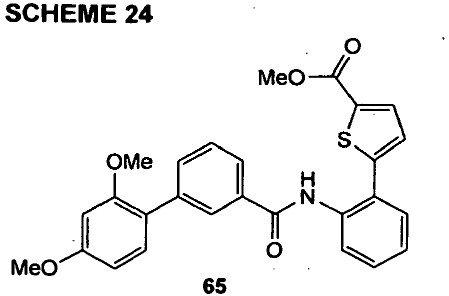

(5-{2-[(2',4'-dimethoxy-biphenyl-3-carbonyl)-amino)-phenyl}-thiophene-2-carboxylic acid methyl ester (65) is synthesized according to the procedure described in step 1 of EXAMPLE 8 starting from the aniline (45) and the carboxylic acid chloride (54) and is isolated as a yellow solid (147mg, 62%). ¹H NMR (400 MHz, CDCl₃): 3.75 (s, 3 H); 3.85 (s, 3 H); 3.87 (s, 3 H); 6.54 (s, 1 H); 6.57 (dd, 1 H, *J*₁ = 8.6 Hz, *J*₂ = 2.0 Hz); 7.17 (d, 1 H, *J=* 4.3 Hz); 7.19 (d, 1 H, *J =* 9.0 Hz); 7.20 (t, 1 H, *J*= 8.6 Hz); 7.41 (d, 1 H, *J=* 7.8 Hz); 7.44 (t, 1 H, *J=* 7.8 Hz); 7.46 (t, 1 H, *J=* 8.0 Hz); 7.64 (d, 1 H, *J =* 8.3 Hz); 7.66 (d, 1 H, *J*= 7.8 Hz); 7.80 (d, 1 H, *J*= 4.0 Hz); 7.83 (s, 1 H); 8.19 (br. s, 1 H); 8.47 (d, 1 H, *J =* 8.3 Hz).

### EXAMPLE 11

### (5-{2-[(2',4'-Dimethoxy-biphenyl-3-carbonyl)-amino]-phenyl}-thiophene-2-carboxylic acid (66)

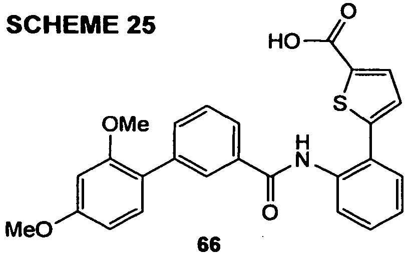

Saponification of carboxylic acid methyl ester (65) in mixture of THF and MeOH (3+2) according to the procedure described in step 2 of EXAMPLE 8 affords (5-{2-[(2',4'-dimethoxy-biphenyl-3-carbonyl)-amino]-phenyl}-thiophene-2-carboxylic acid (66) as a yellow oil (130mg, quant.). ¹H NMR (400 MHz, CD₃OD): 3.83 (s, 3 H); 3.88 (s, 3 H); 6.66 (dd, 1 H, *J*₁ = 8.0 Hz, *J*₂ = 2.3 Hz); 6.67 (s, 1 H); 7.31 (d, 1 H, *J=* 8.0 Hz); 7.36 (d, 1 H, *J= 4.0* Hz); 7.44 (td, 1 H, *J*₁ = 7.8 Hz, *J*₂ = 1.0 Hz); 7.48-7.53 (m, 1 H); 7.52 (t, 1 H, *J*= 7.8 Hz); 7.65 (d, 1 H, *J*= 7.8 Hz); 7.71 (d, 1 H, *J*= 7.8 Hz); 7.73 (d, 1 H, *J* = 8.3 Hz); 7.76 (d, 1 H, *J=* 4.0 Hz); 7.84 (d, 1 H, *J=* 7.8 Hz); 8.01 (s, 1 H).

### EXAMPLE 12

### (5-{2-[(2',4'-Dihydroxy-biphenyl-3-carbonyl)-amino]-phenyl}-thiophene-2-carboxylic acid (67)

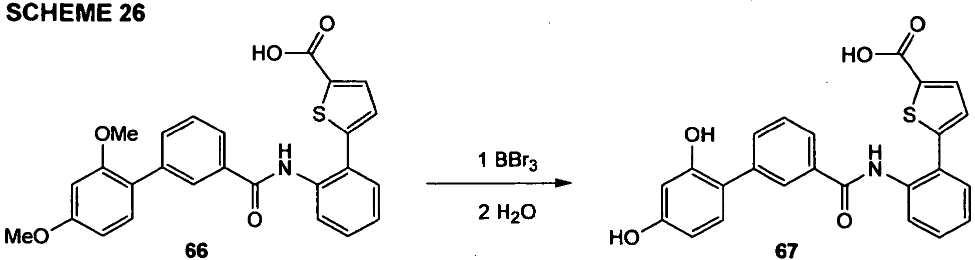

(The following reaction is carried out in an N₂ atmosphere.) Suspend (5-{2-[(2',4'-dimethoxy-biphenyl-3-carbonyl)-amino]-phenyl}-thiophene-2-carboxylic acid (66) (88.6mg, 0.19mmol)) in anhydrous dichloromethane (4.0mL), cool the mixture to -78°C and add dropwise 1M BBr₃ solution in dichloromethane (0.80mL, 0.80mmol). Stir the reaction mixture for 30min at -78°C and after slowly warming up for additional 2h at rt. Cool the reaction mixture to 0°C, add dropwise ice water, separate layers and extract aqu. layer with EtOAc (3 times). Wash combined organic layer with brine and dry with Na₂SO₄. Purify the crude product by preparative RP HPLC (gradient, water/CH₃CN 95:5 to 5:95) to obtain (5-{2-[(2',4'-dihydroxy-biphenyl-3-carbonyl)-amino]-phenyl}-thiophene-2-carboxylic acid (67) as a off white solid (23mg, 26%). ¹H NMR (400 MHz, CD₃OD): 6.44 (dd, 1 H, *J*₁ = 7.8 Hz, *J*₂ = 2.3 Hz); 6.45 (s, 1 H); 7.19 (d, 1 H, *J*= 7.8 Hz); 7.35 (d, 1 H, *J=* 4.0 Hz); 7.42 (t, 1 H, *J* = 7.6 Hz); 7.50 (td, 1 H, *J*₁ = 7.6 Hz, *J*₂ = 1.3 Hz); 7.51 (t, 1 H, *J=* 7.8 Hz); 7.69 (d, 2 H, *J=* 7.6 Hz); 7.72 (d, 1 H, *J=* 3.8 Hz); 7.80 (d, 2 H, *J* = 7.8 Hz); 8.09 (s, 1 H).

### EXAMPLE 13

### (5-{2-[2-(2',4'-Dimethoxy-biphenyl-3-yl)-acetylamino]-phenyl}-thiophen-2-yl)-acetic acid methyl ester (68)

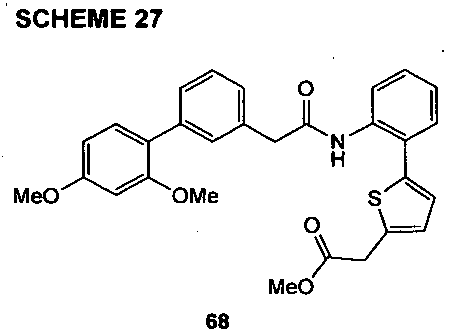

(5-{2-[2-(2',4'-Dimethoxy-biphenyl-3-yl)-acetylamino]-phenyl}-thiophen-2-yl)-acetic acid methyl ester (68) is synthesized according to the procedure described in step 1 of EXAMPLE 4 starting from the aniline (43) and the carboxylic acid (57) and is isolated as a white solid (117mg, 57%). ¹H NMR (400 MHz, CDCl₃): 3.66 (s, 2 H); 3.71 (s, 3 H); 3.72 (s, 2 H); 3.73 (s, 3 H); 3.84 (s, 3 H); 6.39 (d, 1 H, *J*= 3.0 Hz); 6.53 (s, 1 H); 6.51-6.57 (m, 2 H); 7.06 (t, 1 H, *J*= 8.3 Hz); 7.07 (d, 1 H, *J*= 8.0 Hz); 7.13 (d, 1 H, *J*= 8.6 Hz); 7.22-7.27 (m, 1 H); 7.28 (t, 1 H, *J*= 7.8 Hz); 7.31 (t, 1 H, *J*= 7.8 Hz); 7.33 (s, 1 H); 7.42 (d, 1 H, *J*= 7.8 Hz); 7.66 (s, 1 H); 8.38 (d, 1 H, *J*= 8.3 Hz).

### EXAMPLE 14

### (5-{2-[2-(2',4'-Dimethoxy-biphenyl-3-yl)-acetylamino]-phenyl}-thiophen-2-yl)-acetic acid (69)

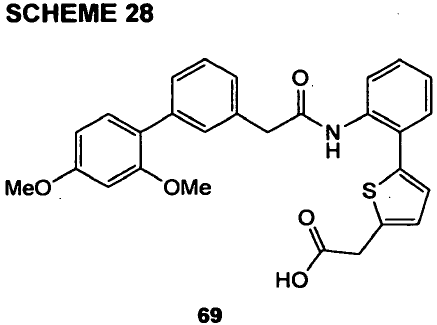

Saponification of the acetic acid methyl ester (68) in MeCN according to the procedure described in step 2 of EXAMPLE 8 affords (5-{2-[2-(2',4'-dimethoxy-biphenyl-3-yl)-acetylamino]-phenyl}-thiophen-2-yl)-acetic acid (69) as a yellow oil (117mg, 96%). ¹H NMR (400 MHz, CD₃OD): 3.72 (s, 2 H); 3.75 (s, 2 H); 3.80 (s, 3 H); 3.88 (s, 3 H); 6.64 (dd, 1 H, *J*₁ = 8.6 Hz, *J*₂ = 2.3 Hz); 6.66 (m, 1 H); 6.70 (d, 1 H, *J*= 3.0 Hz); 6.78 (d, 1 H, *J* = 3.5 Hz); 7.22-7.27 (m, 1 H); 7.25 (d, 1 H, *J*= 8.6 Hz); 7.26 (t, 1 H, *J*= 7.6 Hz); 7.36 (t, 2 H, *J*= 7.8 Hz); 7.42-7.49 (m, 3 H); 7.74 (d, 1 H, *J*= 8.0 Hz).

### EXAMPLE 15

### (5-{2-[2-(2',4'-Dihydroxy-biphenyl-3-yl)-acetylamino]-phenyl}-thiophen-2-yl)-acetic acid (70)

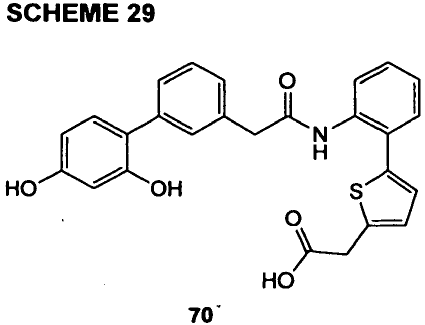

According to the procedure described in EXAMPLE 12 the reaction of the carboxylic acid (69) with a 1M BBr₃ solution affords (5-{2-[2-(2',4'-dihydroxy-biphenyl-3-yl)-acetylamino]-phenyl}-thiophen-2-yl)-acetic acid (70) as a off-white solid (61mg, 55%). ¹H NMR (400 MHz, CD₃OD): 3.75 (s, 2 H); 3.76 (s, 2 H); 6.42 (dd, 1 H, *J*₁ = 8.3 Hz, *J*₂ = 2.3 Hz); 6.44 (d, 1 H, *J*= 2.3 Hz); 6.74 (d, 1 H, *J =* 3.5 Hz); 6.81 (d, 1 H, *J*= 3.5 Hz); 7.11 (d, 1 H, *J*= 8.3 Hz); 7.22 (d, 1 H, *J*= 7.6 Hz); 7.26 (t, 1 H, *J*= 7.6 Hz); 7.35 (t, 2 H, *J* = 7.8 Hz); 7.46 (d, 1 H, *J*= 7.8 Hz); 7.48-7.52 (m, 1 H); 7.51 (s, 1 H); 7.78 (d, 1 H, *J* = 8.1 Hz).

### EXAMPLE 16

### [5-(2-{[5-(2,4-Dihydroxy-phenyl)-pyridine-3-carbonyl]-amino}-phenyl)-thiophen-2-yl]-acetic acid (73) and [5-(2-{[5-(2,4-Dihydroxy-phenyl)-pyridine-3-carbonyl]-amino}-phenyl)-thiophen-2-yl]-acetic acid methyl ester (74)

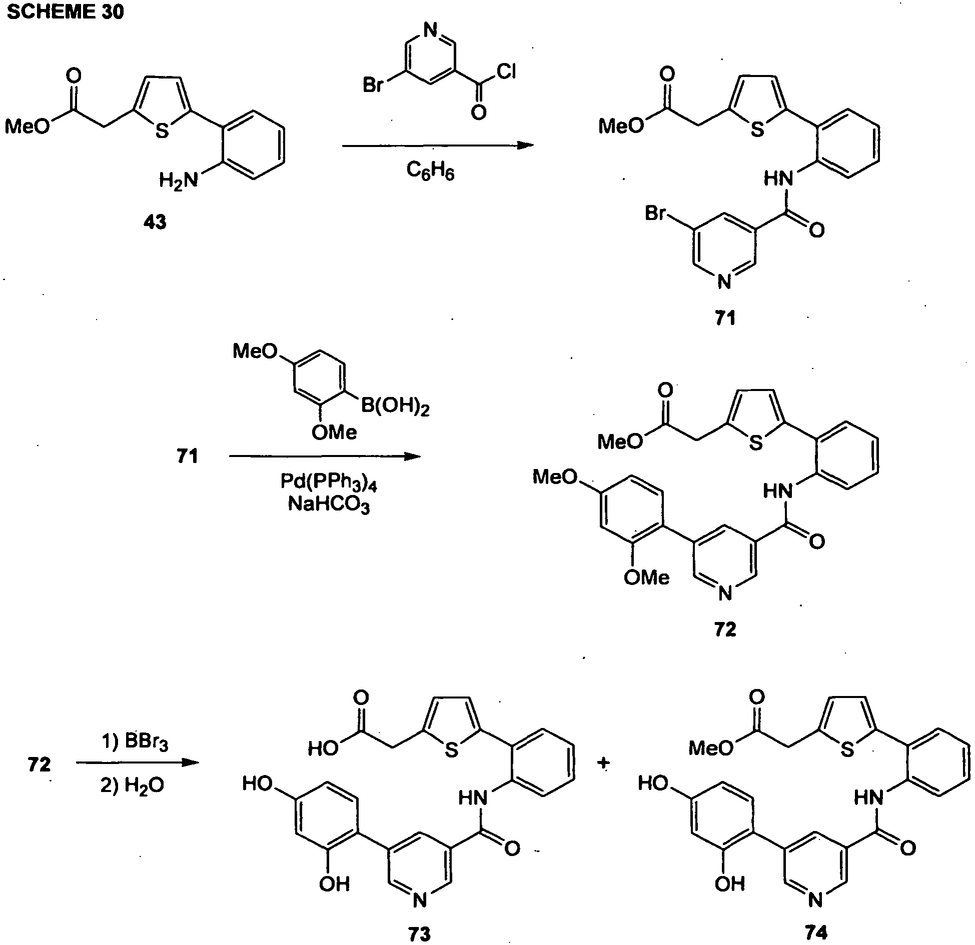

**Step 1:** (The following reaction is carried out in an N₂ atmosphere.) Dissolve [5-(2-aminophenyl)-thiophen-2-yl]-acetic acid methyl ester (43) (250mg, 1.01mmol) in anhydrous C₆H₆ (4.0mL). Add a solution of 5-bromo-nicotinoyl chloride (223mg, 1.01mmol) and stir the resulting suspension overnight at rt. Remove solvent under reduced pressure. Purify the crude product by preparative radial chromatography (silica gel 60PF, CyH/EtOAc 3+1, then 1+1 and later EtOAc and EtOAc/MeOH 1+1) to obtain (5-{2-[(5-bromo-pyridine-3-carbonyl)-amino]-phenyl}-thiophen-2-yl)-acetic acid methyl ester (71) as a yellow solid (216mg, 49%). ¹H NMR (400 MHz, CD₃OD): 3.71 (s, 3 H); 3.90 (s, 2 H); 6.97 (d, 1 H, *J=* 3.5 Hz); 7.18 (d, 1 H, *J*= 3.5 Hz); 7.41 (td, 1 H, *J*₁ = 7.3 Hz, *J*₂ = 1.8 Hz); 7.45 (td, 1 H, *J*₁ = 7.3 Hz, *J*₂ = 2.0 Hz); 7.59 (dd, 1 H, *J*₁ = 7.3 Hz, *J*₂ = 2.0 Hz); 7.67 (dd, 1 H, *J*₁ = 7.1 Hz, *J*₂ = 2.0 Hz; 8.52 (s, 1 H); 8.88 (m, 1 H); 9.05 (s, 1 H).

Step 2: (The following reaction is carried out in an N₂ atmosphere.) Dissolve tetrakis-(triphenylphosphine)-palladium(0) (12mg, 0.01 mmol) and (5-{2-[(5-bromo-pyridine-3-carbonyl)-amino]-phenyl}-thiophen-2-yl)-acetic acid methyl ester (71) (150mg, 0.35mmol) in DME (1.4mL). Add 2,4-dimethoxyphenylboronic acid (95.0mg, 0.52mmol) and a 1M aqueous NaHCO₃ solution (1.0mL, 1.0mmol), degas the reaction mixture carefully (5 times) and flush with N₂. Stir the resulting mixture for 1h at 90°C and cool down to rt again. Remove solvent under reduced pressure, resolve the residue in MeOH and filtrate over a short pad of silica gel. Concentrate the filtrate under reduced pressure and purify the crude product by preparative radial chromatography (silica gel 60PF, CyH/EtOAc 1+1] to obtain [5-(2-{[5-(2,4-dimethoxy-phenyl)-pyridine-3-carbonyl]-amino}-phenyl)-thiophen-2-yl]-acetic acid methyl ester (72) as a yellow sticky oil (121mg, 71%). ¹H NMR (400 MHz, CD₃OD): 3.64 (s, 3 H); 3.89 (br.s, 5 H); 3.90 (s, 3 H); 6.70-6.75 (m, 1 H); 6.74 (s, 1 H); 6.97 (d, 1 H, *J*= 3.3 Hz); 7.20 (d, 1 H, *J*= 3.5 Hz); 7.37-7.44 (m, 2 H); 7.47 (td, 1 H, *J*₁ = 7.3 Hz, *J*₂ = 1.3 Hz); 7.61-7.72 (m, 3 H); 8.44 (s, 1 H); 8.87 (s, 1 H); 8.97 (s, 1 H).

**Step 3:** (The following reaction is carried out in an N₂ atmosphere.) Dissolve [5-(2-{[5-(2,4-dimethoxy-phenyl)-pyridine-3-carbonyl]-amino}-phenyl)-thiophen-2-yl]-acetic acid methyl ester (72) (50mg, 0.10mmol) in anhydrous dichloromethane (1.2mL), cool the solution to -78°C and add dropwise 1M BBr₃ solution in dichloromethane (1.10mL, 1.10mmol). Stir the reaction mixture for 30min at -78°C and for additional 1h at rt. Cool the mixture to 0°C, add dropwise water and concentrate under reduced pressure. Purify the crude product by preparative RP HPLC (gradient, water/CH₃CN 95:5 to 5:95) to afford [5-(2-{[5-(2,4-dihydroxy-phenyl)-pyridine-3-carbonyl]-amino}-phenyl)-thiophen-2-yl]-acetic acid (73) as a yellow solid (12,6mg, 46%) and [5-(2-{[5-(2,4-dihydroxy-phenyl)-pyridine-3-carbonyl]-amino}-phenyl)-thiophen-2-yl]-acetic acid methyl ester (74) as a brown solid (21.9mg, 47%). ¹H NMR compound (73) (400 MHz, CD₃OD): 3.86 (s, 2 H); 6.49 (dd, 1 H, *J*₁ = 9.1 Hz, *J*₂ = 2.3 Hz); 6.49 (d, 1 H, *J*= 2.0 Hz); 6.98 (d, 1 H, *J*= 3.5 Hz); 7.20 (d, 1 H, *J=* 3.5 Hz); 7.26 (d, 1 H, *J*= 9.1 Hz); 7.40 (td, 1 H, *J*₁ = 7.6 Hz, *J*₂ = 1.5 Hz); 7.45 (td, 1 H, *J*₁ = 7.6 Hz, *J*₂ = 1.8 Hz); 7.66 (dd, 1 H, *J*₁ = 7.6 Hz, *J*₂ = 1.8 Hz); 7.66-7.70 (m, 1 H); 8.50 (br.s, 1 H); 8.92 (br.s, 1 H); 8.95 (s, 1 H). ¹H NMR compound (74) (400 MHz, CD₃OD): 3.65 (s, 3 H); 3.89 (s, 2 H); 6.51 (s, 1 H); 6.52 (dd, 1 H, *J*₁ = 8.3 Hz, *J*₂ = 2.3 Hz); 6.97 (d, 1 H, *J*= 3.5 Hz); 7.21 (d, 1 H, *J*= 3.5 Hz); 7.36 (d, 1 H, *J*= 8.3 Hz); 7.43 (td, 1 H, *J*₁ = 7.3 Hz, *J*₂ = 1.5 Hz); 7.46 (td, 1 H, *J*₁ = 7.6 Hz, *J*₂ = 1.8 Hz); 7.63-7.69 (m, 2 H); 8.80 (br.s, 1 H); 9.00 (br.s, 1 H); 9.11 (s, 1 H).

### EXAMPLE 17

### 5-(4-{[5-(2,4-Dimethoxy-phenyl)-pyridine-3-carbonyl]-amino}-phenyl)-2-methylfuran-3-carboxylic acid methyl ester (75)

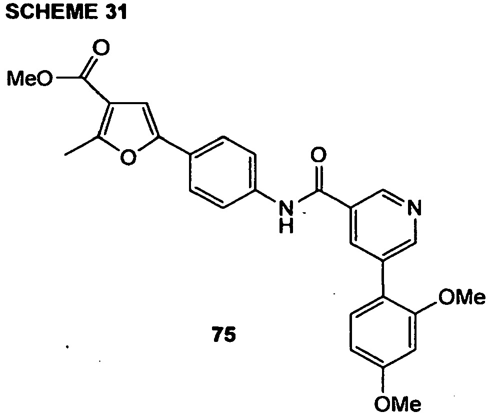

5-(4-{[5-(2,4-dimethoxy-phenyl)-pyridine-3-carbonyl]-amino}-phenyl)-2-methyl-furan-3-carboxylic acid methyl ester (75) is synthesized according to the procedure described in step 1 and 2 of EXAMPLE 16 starting from the aniline (47) and 5-bromo-nicotinoyl chloride and is isolated as a grey solid (103mg, 32% over 2 steps). ¹H NMR (400 MHz, CD₃OD): 2.68 (s, 3 H); 3.89 (s, 3 H); 3.90 (s, 3 H); 3.91 (s, 3 H); 6.71-6.76 (m, 2 H); 6.99 (s, 1 H); 7.42 (d, 1 H, *J*= 8.1 Hz); 7.74 (d, 2 H, *J*= 8.6 Hz); 7.84 (d, 2 H, *J*= 8.6 Hz); 8.47 (t, 1 H, *J*= 2.0 Hz); 8.87 (d, 1 H, *J*= 2.0 Hz); 9.00 (d, 1 H, *J*= 2.0 Hz).

### EXAMPLE 18

### 5-(4-{[5-(2,4-Dibydrozy-phenyl)-pyridine-3-carbonyl]-amino}-phenyl)-2-methylfuran-3-carboxylic acid (76)

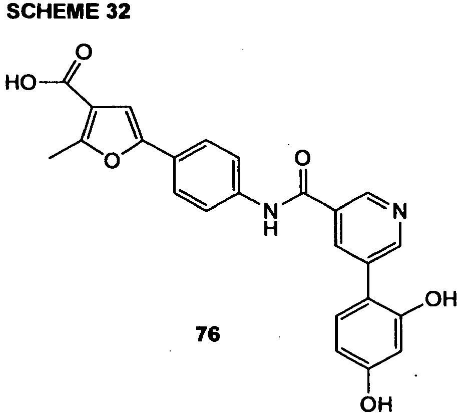

According to the procedure described in step 3 of EXAMPLE 16 the reaction of the amide (75) with a 1M BBr₃ solution affords 5-(4-{[5-(2,4-dihydroxy-phenyl)-pyridine-3-carbonyl]-amino}-phenyl)-2-methyl-furan-3-carboxylic acid (76) as a brown solid (22mg, 24%). ¹H NMR (400 MHz, CD₃OD): 2.69 (s, 3 H); 3.39 (s, 1 H); 6.51 (s, 1 H); 6.52 (dd, 1 H, *J*₁ = 8.1 Hz, *J*₂ = 2.3 Hz); 6.98 (s, 1 H); 7.36 (d, 1 H, *J*= 8.1 Hz); 7.74 (br.d, 2 H, *J=* 8.6 Hz); 7.85 (br.d, 2 H, *J=* 8.6 Hz); 8.73 (s, 1 H); 9.08 (br.s, 2 H).

### EXAMPLE 19

### 3-[(2',4'-Dimethoxy-biphenyl-3-carbonyl)-amino]-benzoic acid methyl ester (77)

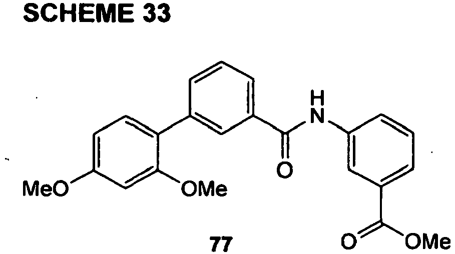

3-[(2',4'-Dimethoxy-biphenyl-3-carbonyl)-amino]-benzoic acid methyl ester (77) is synthesized according to the procedure described in step 1 of EXAMPLE 8 starting from 3-amino-benzoic acid methyl ester and 2',4'-dimethoxy biphenyl-3-carbonyl chloride (54) and is isolated as a colorless oil (143mg, 73%). ¹H NMR (400 MHz, CDCl₃ :3.80 (s, 3 H); 3.85 (s, 3 H); 3.91 (s, 3 H); 6.57 (s, 1 H); 6.58 (dd, 1 H, *J*₁, = 8.1 Hz, *J*₂ = 2.3 Hz); 7.26 (d, 1 H, *J=* 8.1 Hz); 7.45 (t, 1 H, *J=* 7.8 Hz); 7.50 (t, 1 H, *J= 7.8* Hz); 7.68 (dt, 1 H, *J*₁ = 7.8 Hz, *J*₂ = 1.3 Hz); 7.78 (ddd, 1 H, *J*₁ = 7.8 Hz, *J*₂ = 1.8 Hz, *J*₃ = 1.3 H); 7.81 (dt, 1 H, *J*₁ = 7.8 Hz, *J*₂ = 1.3 Hz); 7.88 (br.s, 1 H); 7.97 (t, 1 H, *J* = 1.8 Hz); 8.04 (ddd, 1 H, *J*₁ = 8.1 Hz, *J*₂ = 2.3 Hz, *J*₃ = 1.0 H); 8.13 (t, 1 H, *J*= 1.8 Hz).

### EXAMPLE 20

### 3-[(2',4'-Dimethoxy-biphenyl-3-carbonyl)-amino]-benzoic acid (78)

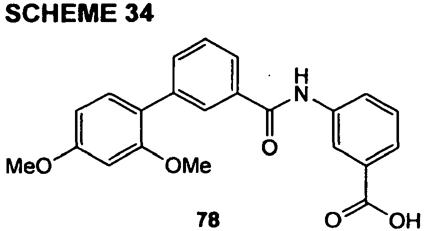

Saponification of the carboxylic acid methyl ester (77) in MeCN according to the procedure described in step 2 of EXAMPLE 8 affords 3-[(2',4'-dimethoxy-biphenyl-3-carbonyl)-amino]-benzoic acid (78) as a white solid (109mg, 80%). ¹H NMR (400 MHz, (CD₃)₂SO): 3.77 (s, 3 H); 3.81 (s, 3 H); 6.65 (dd, 1 H, *J*₁ = 8.3 Hz, *J*₂ = 2.3 Hz); 6.69 (d, 1 H, *J=* 2.3 Hz); 7.32 (d, 1 H, *J=* 8.3 Hz); 7.45 (t, 1 H, *J=* 7.8 Hz); 7.52 (t, 1 H, *J=* 7.8 Hz); 7.66 (br.d, 2 H, *J=* 7.6 Hz); 7.87 (dt, 1 H, *J*₁ = 7.8 Hz, *J*₂ = 1.5 Hz); 8.00-8.05 (m, 1 H); 8.01 (t, 1 H, *J* = 1.5 Hz); 8.38 (br.s, 1 H); 10.39 (s, 1 H).

### EXAMPLE 21

### 3-[(2',4'-Dihydrozy-biphenyl-3-carbonyl)-amino]-benzoic acid (79)

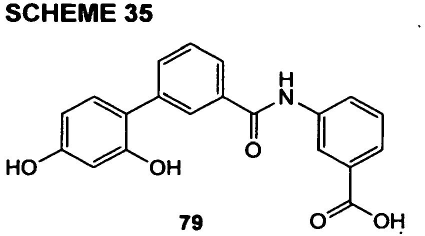

According to the procedure described in EXAMPLE 12 the reaction of the carboxylic acid (78) with a 1M BBr₃ solution affords 3-[(2',4'-dihydroxy-biphenyl-3-carbonyl)-amino]-benzoic acid (79) as a off white solid (19mg, 26%). ¹H NMR (400 MHz, (CD₃OD): 6.45 (dd, 1 H, *J*₁ = 8.1 Hz, *J*₂ = 2.3 Hz); 6.46 (s, 1 H); 7.22 (d, 1 H, *J*₁ = 7.6 Hz, *J*₂ = 1.5 Hz); 7.44 (t, 1 H, *J*= 7.8 Hz); 7.53 (t, 1 H, *J*= 7.8 Hz); 7.77-7.82 (m, 2 H); 7.85 (d, 1 H, *J* = 7.8 Hz); 8.04 (d, 1 H, *J* = 8.1 Hz); 8.10-8.15 (m, 1 H); 8.12 (t, 1 H, *J* = 1.8 Hz).

### EXAMPLE 22

### (5-{2-[(3',5'-Dimethoxy-biphenyl-3-carbonyl)-amino]-phenyl}-thiophen-2-yl)-acetic acid methyl ester (80)

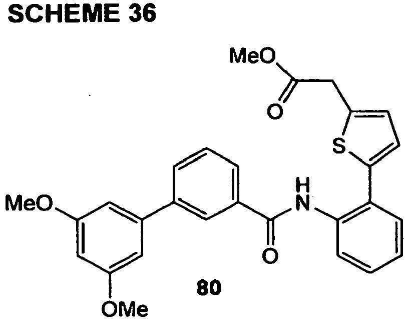

(5-{2-[(3',5'-Dimethoxy-biphenyl-3-carbonyl)-amino]-phenyl}-thiophen-2-yl)-acetic acid methyl ester (80) is synthesized according to the procedure described in step 1 of EXAMPLE 8 starting from the aniline (43) and 3',5'-dimethoxy biphenyl-3-carbonyl chloride (60). In difference to the purification procedure described there the crude product (80) is suspended in EtOAc, filtrated and the filter cake washed with EtOAc (2 times). Compound (80) is obtained as a yellow solid (187mg, 68%). ¹H NMR (400 MHz, CDCl₃): 3.71 (s, 3 H); 3.84 (s, 6 H); 3.87 (s, 2 H); 6.48 (t, 1 H, *J*= 2.3 Hz); 6.69 (d, 2 H, *J= 2.3* Hz); 6.99-7.02 (m, 1 H); 7.03 (d, 1 H, *J*= 3.5Hz); 7.17 (dd, 1 H, *J*₁ = 7.6 Hz, *J*₂ = 1.3 Hz); 7.39-7.44 (m, 2 H); 7.50 (t, 1 H, *J*= 7.8 Hz); 7.68-7.73 (m, 2 H); 7.90-7.94 (m, 1 H); 8.43 (s, 1 H); 8.53 (d, 1 H, *J* = 7.6 Hz).

### EXAMPLE 23

### (5-{2-[(3',5'-Dimethoxy-biphenyl-3-carbonyl)-amino]-phenyl}-thiophen-2-yl)-acetic acid (81)

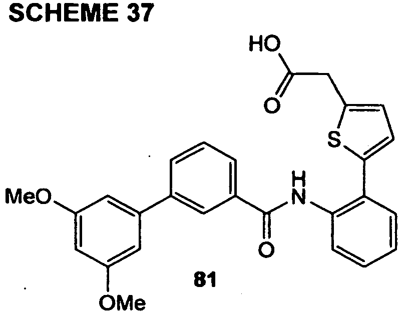

Saponification of the carboxylic acid methyl ester (80) in MeCN according to the procedure described above in step 2 of EXAMPLE 8 affords (5-{2-[(3',5'-dimethoxy-biphenyl-3-carbonyl)-amino]-phenyl}-thiophen-2-yl)-acetic acid (81) as a yellow solid (188mg, quant.). ¹H NMR (400 MHz, CD₃OD): 3.83 (s, 2 H); 3.89 (br.s, 6 H); 6.56(t, 1 H, *J=* 2.3 Hz); 6.86 (d, 2 H, *J=* 1.8 Hz); 6.97-7.00 (m, 1 H); 7.19 (d, 1 H, *J*= 3.5 Hz); 7.38 (td, 1 H, *J*₁ = 7.6 Hz, *J*₂ = 1.3 Hz); 7.44 (td, 1 H, *J*₁ = 7.6 Hz, *J*₂ = 1.5 Hz); 7.61 (t, 1 H, *J=* 7.8 Hz); 7.64 (dd, 1 H, *J*₁ = 7.8 Hz, *J*₂ = 1.3 Hz); 7.72-7.76 (m, 1 H); 7.84-7.87 (m, 1 H); 7.90-7.94 (m, 1 H); 8.16 (br.s, 1 H).

### EXAMPLE 24

### (5-{2-[(3',5'-Dihydrozy-biphenyl-3-carbonyl)-amino]-phenyl}-thiophen-2-yl)-acetic acid (82)

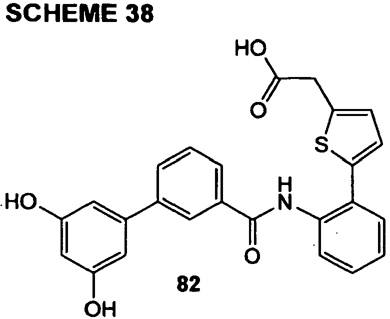

According to the procedure described above in EXAMPLE 12 reaction of the carboxylic acid (81) with a 1M BBr₃ solution affords (5-{2-[(3',5'-dihydroxy-biphenyl-3-carbonyl)-amino]-phenyl}-thiophen-2-yl)-acetic acid (82) as a light brown solid (33mg, 35%). ¹H NMR (400 MHz, CD₃0D): 3.87 (s, 2 H); 6.34 (t, 1 H, *J*= 2.0 Hz); 6.63 (d, 2 H, *J=* 2.0 Hz); 7.01 (d, 1 H, *J* = 3.5 Hz); 7.20 (d, 1 H, *J* = 3.5 Hz); 7.38 (td, 1 H, *J*₁ = 7.6 Hz, *J*₂ = 1.5 Hz); 7.44 (td, 1 H, *J*₁ = 7.6 Hz, *J*₂ = 1.5 Hz); 7.58 (t, 1 H, *J*= 7.8 Hz); 7.64 (dd, 1 H, *J*₁ *=* 7.6 Hz, *J*₂ = 1.5 Hz); 7.74 (d, 1 H, *J*= 7.8Hz)*;* 7.79 (dd, 1 H, *J*₁ = 7.8 Hz, *J*₂ = 1.0 Hz); 7.89 (d, 1 H, *J* = 7.6 Hz); 8.10 (s, 1 H).

### EXAMPLE 25

### 5-{2-[(3',5'-Dimethoxy-biphenyl-3-carbonyl)-amino]-phenyl}-thiophene-2-carboxylic acid. methyl ester (83)

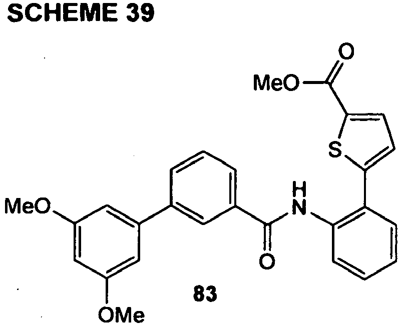

5-{2-[(3',5'-Dimethoxy-biphenyl-3-carbonyl)-amino]-phenyl}-thiophene-2-carboxylic acid methyl ester (83) is synthesized according to the procedure described in step 1 of EXAMPLE 8 starting from aniline (45) and carboxylic acid chloride (60). In difference to the purification procedure described there the crude product (80) is suspended in EtOAc, filtrated and the filter cake washed with EtOAc (2 times). Compound (83) is obtained as a yellow solid (168mg, 63%). ¹H NMR (400 MHz, CDCl₃): 3.84 (s, 6 H); 3.87 (s, 3 H); 6.48 (t, 1 H, *J*= 2.0 Hz); 6.89 (d, 2 H, *J*= 2.0 Hz); 7.20 (d, 1 H, *J*= 3.8 Hz); 7.21-7.25 (m, 1 H); 7.43 (td, 1 H, *J*₁ = 7.8 Hz, *J*₂ = 1.5 Hz); 7.47 (t, 1 H, *J=* 7.8 Hz); 7.49 (t, 1 H, *J*= 7.8 Hz); 7.65 (d, 1 H, *J*= 7.8 Hz); 7.72 (dd, 1 H, *J*₁ = 7.8 Hz, *J*₂ = 1.0 Hz); 7.84 (d, 1 H, *J* = 3.8 Hz); 7.97 (d, 1 H, *J* = 1.5 Hz); 8.22 (s, 1 H); 8.46 (d, 1 H, *J* = 7.8 Hz).

### EXAMPLE 26

### 5-{2-[(3',5'-Dimethoxy-biphenyl-3-carbonyl)-amino]-phenyl}-tbiophene-2-carboxylic acid (84)

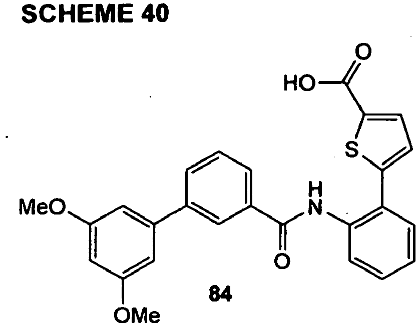

Saponification of the carboxylic acid methyl ester (83) in MeCN according to the procedure described above in step 2 of EXAMPLE 8 affords 5-{2-[(3',5'-dimethoxy-biphenyl-3-carbonyl)-amino]-phenyl}-thiophene-2-carboxylic acid (84) as a white solid (159mg, 97%). ¹H NMR (400 MHz, CD₃OD): 3.88 (s, 6 H); 6.55 (t, 1 H, *J*= 2.0 Hz); 6.86 (br.s, 2 H); 7.38 (d, 1 H, *J*= 3.8 Hz); 7.46 (td, 1 H, *J*₁ = 7.6 Hz, *J*₂ = 1.5 Hz); 7.52 (td, 1 H, *J*₁ = 7.6 Hz, *J*₂ = 1.5 Hz); 7.58-7.65 (m, 2 H); 7.71-7.74 (m, 1 H); 7.75 (d, 1 H, *J*= 3.8 Hz); 7.87 (dd, 1 H, *J*₁ = 7.8 Hz; *J*₂ = 1.0 Hz); 7.91-7.96 (m, 1 H); 8.19 (s, 1 H).

### EXAMPLE 27

### 5-{2-[(3',5'-Dihydroxy-biphenyl-3-carbonyl)-amino]-phenyl}-thiophene-2-carboxylic acid (85)

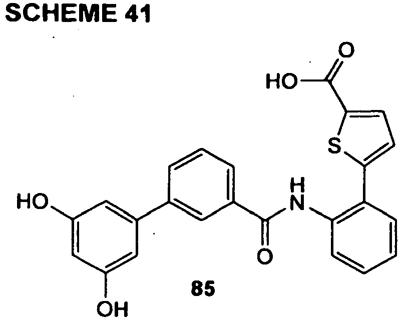

According to the procedure described above in EXAMPLE 12 reaction of the carboxylic acid (84) with a 1M BBr₃ solution affords 5-{2-[(3',5'-dihydroxy-biphenyl-3-carbonyl)-amino]-phenyl}-thiophene-2-carboxylic acid (85) as a white solid (24mg, 50%). ¹H NMR (400 MHz, CD₃OD): 6.33 (t, 1 H, *J*= 2.0 Hz); 6.66 (d, 2 H, *J*= 2.0 Hz); 7.38 (d, 1 H, *J=* 4.0 Hz); 7.46 (td, 1 *H, J*₁ = 7.6 Hz, *J*₂ = 1.5 Hz); 7.52 (td, 1 H, *J*₁ = 7.6 Hz, *J*₂ = 1.5 Hz); 7.58 (t, 1 H, *J* = 7.6 Hz); 7.63 (d, 1 H, *J* = 7.6 Hz); 7.73 (dd, 1 H , *J*₁ = 7.6 Hz, *J*₂ = 1.3 Hz); 7.75 (d, 1 H, *J* = 4.0); 7.80 (d, 1 H, *J* = 7.8 Hz); 7.87-7.91 (m, 1 H); 8.16 (s, 1 H).

### EXAMPLE 28

### 2',4'-Dimethoxy-biphenyl-3-carboxylic acid (2-thiophen-2-yl-phenyl)-amide (86)

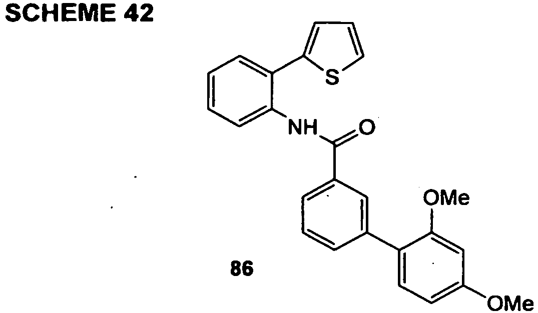

2',4'-Dimethoxy-biphenyl-3-carboxylic acid (2-thiophen-2-yl-phenyl)-amide (86) is synthesized according to the procedure described above in step 1 of EXAMPLE 8 starting from the aniline (48) and 2',4'-dimethoxy biphenyl-3-carbonyl chloride (54) and is isolated as a light yellow solid (159mg, 58%). ¹H NMR (400 MHz, C₆D₆): 3.27 (s, 3 H); 3.50 (s, 3 H); 6.52 (dd, 1 H, *J*₁ = 8.3 Hz, *J*₂ = 2.5 Hz); 6.58 (d, 1 H, *J* = 2.5 Hz); 6.76 (dd, 1 H, *J*₁ = 5.0 Hz, *J*₂ = 3.5 Hz) 6.87 (dd, 1 H, *J*₁ *=* 3.5 Hz, *J*₂ = 1.3 Hz); 6.92 (dd, 1 H, *J*₁ = 5.0 Hz, *J*₂ = 1.3 Hz); 6.98 (td, 1 H, *J*₁ = 7.6 Hz, *J*₂ = 1.3 Hz); 7.23 (t, 1 H, *J* = 7.6 Hz); 7.28-7.33 (m, 1 H); 7.39 (dd, 1 H, *J*₁ = 7.6 Hz*, J*₂ *=* 1.5 Hz); 7.71 (dt, 1 H, *J*₁ = 7.8 Hz, *J*₂ = 1.3 Hz); 7.77 (ddd, 1 H, *J*₁ = 7.8 Hz, *J*₂ = 1.5 Hz, *J*₃ = 1.3 Hz); 8.28 (t, 1 H, *J* = 1.5 Hz); 8.46 (br. s, 1 H); 9,18 (d, 1 H, *J* = 8.3 Hz).

### EXAMPLE 29

### 2',4'-Dihydroxy-bipbenyl-3-carboxylic acid (2-thiophen-2-yl-phenyl)-amide (87)

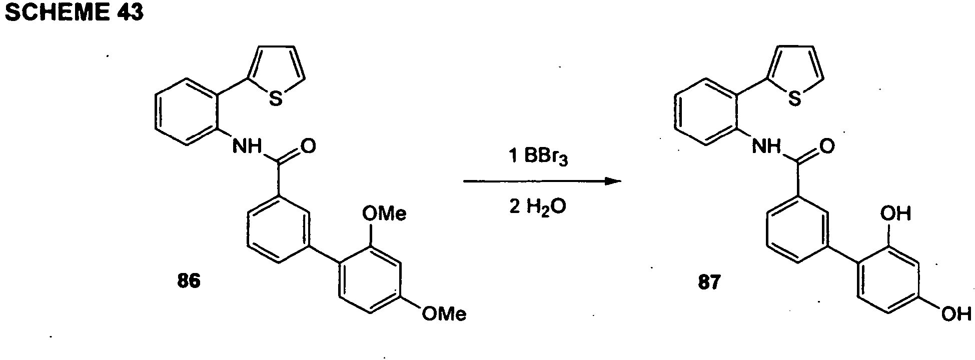

(The following reaction is carried out in an N₂ atmosphere.) Suspend 2',4'-dimethoxy-biphenyl-3-carboxylic acid (2-thiophen-2-yl-phenyl)-amide (86) (122mg, 0.29mmol) in anhydrous dichloromethane (9.7mL), cool the mixture to -78°C and add drop by drop 1M BBr₃ solution in dichloromethane (1.17mL, 1.17mmol). Stir the reaction mixture for 30min at -78°C, slowly warm up to 0°C (over a period of 2h) and stir additional 30min at 0°C. Slowly add ice water, stir 1 h at rt to complete the reaction, separate layers and extract aqu. layer with EtOAc (3 times). Wash combined organic layer with brine and dry with Na₂SO₄. Purify the crude product by preparative RP HPLC (gradient, water/CH₃CN 95:5 to 5:95) to obtain 2',4'-dihydroxy-biphenyl-3-carboxylic acid (2-thiophen-2-yl-phenyl)-amide (87) as an off white solid (35mg, 31%). ¹H NMR (400 MHz, CD₃OD) 6.41-6.46 (m, 2 H) 7.14 (dd, 1 H, *J*₁ = 5.0 Hz, *J*₂ = 3.5 Hz); 7.17 (d, 1 H, *J*= 8.1 Hz); 7.36 (dd, 1 H, *J*₁ = 3.5 Hz, *J*₂ = 1.0 Hz); 7.39 (dd, 1 H, *J*₁ = 7.6 Hz, *J*₂ = 1.0 Hz); 7.45 (td, 1 H, *J*₁ = 7.6 Hz, *J*₂ = 1.5 Hz); 7.47-7.49 (m, 1 H); 7.50 (t, 1 H , *J* = 7.8 Hz) ; 7.65 (dd, 1 H, *J*₁ = 7.6 Hz, *J*₂ = 1.5 Hz); 7.72 (d, 1 H , *J* = 7.8 Hz); 7.75-7.81 (m, 2 H); 8.06 (s, 1 H).

### EXAMPLE 30

### (5-{3-[(2',4'-Dimethoxy-biphenyl-3-carbonyl)-amino]-phenyl}-thiophen-2-yl)-acetic acid methyl ester (88)

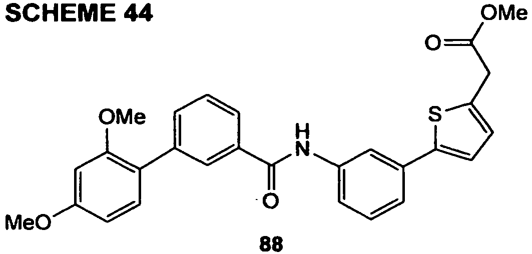

(5-{3-[(2',4'-Dimethoxy-biphenyl-3-carbonyl)-amino]-phenyl}-thiophen-2-yl)-acetic acid methyl ester **(88)** is synthesized according to the procedure described above in step 1 of EXAMPLE 8 in a reaction time of 3h starting from the aniline (50) and the carboxylic acid chloride (54) and is isolated as a yellow solid (213mg, 87%). ¹H NMR (400 MHz, CDCl₃): 3.73 (s, 3 H); 3.80 (s, 3 H); 3.83 (s, 2 H); 3.85 (s, 3 H); 6.57 (s, 1 H); 6.58 (dd, 1 H, *J*₁ = 8.3 Hz, *J*₂ = 2.3 Hz); 6.89 (d, 1 H, *J*= 3.5 Hz); 7.18 (d, 1 H, *J*= 3.5 Hz); 7.27 (d, 1 H, *J* = 8.3 Hz); 7.32-7.37 (m, 2 H); 7.50 (t, 1 H, *J* = 7.6 Hz); 7.54-7.58 (m, 1 H); 7.68 (dt, 1 H , *J*₁ = 7.8 Hz, *J*₂ = 1.3 Hz); 7.79 (dt, 1 H , *J*₁ = 7.8 Hz, *J*₂ = 1.3 Hz); 7.79-7.82 (m, 1 H); 7.86 (t, 1 H, *J* = 1.3 Hz); 7.97 (t, 1 H, *J* = 1.5 Hz).

### EXAMPLE 31

### (5-{3-[(2',4'-Dimethoxy-biphenyl-3-carbonyl)-amino]-phenyl}-thiophen-2-yl)-acetic acid (89)

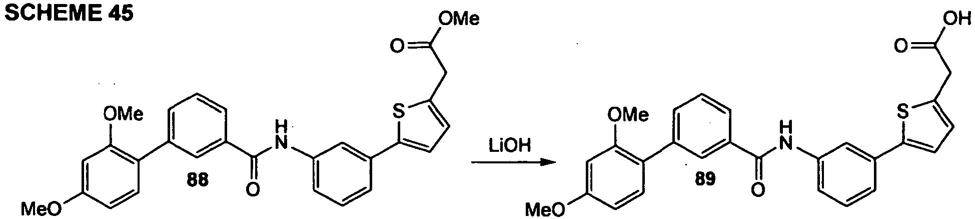

Dissolve (5-{3-[(2',4'-dimethoxy-biphenyl-3-carbonyl)-amino]-phenyl}-thiophen-2-yl)-acetic acid methyl ester **(88)** (190mg, 0.39mmol) in MeCN (3.9mL), add 1M aqu LiOH (1.9mL, 1.9mmol) and stir 16h at rt. Concentrate reaction mixture under reduced pressure and add 1M aqu. HCl (ca. 2mL, to get pH ca.3). Add EtOAc and water and extract the separated aqueous layer several times with EtOAc. Wash combined organic layer with brine and dry it with Na₂SO₄ to obtain (5-{3-[(2',4'-dimethoxy-biphenyl-3-carbonyl)-amino]-phenyl}-thiophen-2-yl)-acetic acid **(89)** as orange solid (185mg, quant.). No further purification. ¹H NMR (400 MHz, CD₃CN): 3.81 (s, 3 H); 3.84 (s, 3 H); 3.85 (s, 2 H); 6.64 (dd, 1 H, *J*₁ = 8.3 Hz, *J*₂ = 2.3 Hz); 6.66 (d, 1 H, *J* = 2.3 Hz); 6.94 (d, 1 H, *J* = 3.5 Hz); 7.25 (d, 1 H, *J* = 3.5 Hz); 7.33 (d, 1 H, *J*= 8.3 Hz); 7.36-7.41 (m, 2 H); 7.52 (t, 1 H, *J* = 7.8 Hz); 7.67-7.71 (m, 2 H); 7.85 (d, 1 H, *J* = 7.8 Hz); 8.02 (br.s; 2 H); 8.80 (s, 1 H).

### EXAMPLE 32

### (5-{3-[(2',4'-Dihydroxy-biphenyl-3-carbonyl)-amino]-pbenyl}-thiophen-2-yl)-acetic acid (90)

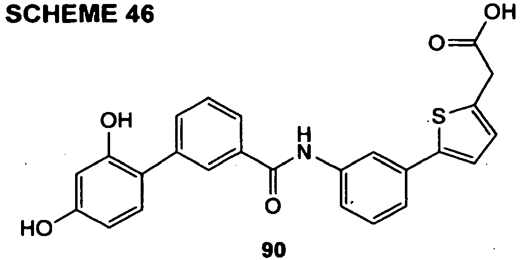

According to the procedure described above in EXAMPLE 12 reaction of the carboxylic acid (89) with a 1M BBr₃ solution affords (5-{3-[(2',4'-dihydroxy-biphenyl-3-carbonyl)-amino]-phenyl}-thiophen-2-yl)-acetic acid (90) as a yellow/brown solid (1.2mg, 1.3%). ¹H NMR (400 MHz, CD₃OD): 3.89 (s, 2 H); 6.45 (dd, 1 H, *J*₁ = 8.1 Hz, *J*₂ = 2.3 Hz); 6.46 (s, 1 H); 6.97 (d, 1 H, *J* = 3.5 Hz); 7.22 (d, 1 H, *J* = 8.1 Hz); 7.30 (d, 1 H, *J* = 3.5 Hz); 7.40 (t, 1 H, *J* = 7.6 Hz); 7.41-7.45 (m, 1 H); 7.54 (t, 1 H, *J* = 7.6 Hz); 7.67 (d, 1 H, *J* = 7.3 Hz); 7.80 (d, 1 H, *J* = 7.8 Hz); 7.85 (d, 1 H, *J* = 7.8 Hz); 8.07 (s, 1 H); 8.12 (s, 1 H).

### EXAMPLE 33

### 5-{6-[(2',4'-Dimethoxy-biphenyl-3-carbonyl)-amino]-pyridin-3-yl}-2-methyl-furan-3-carboxylic acid methyl ester (91) and 5-{6-[(2',4'-Dihydroxy-biphenyl-3-carbonyl)-amino]-pyridin-3-yl}-2-methyl-furan-3-carboxylic acid (93)

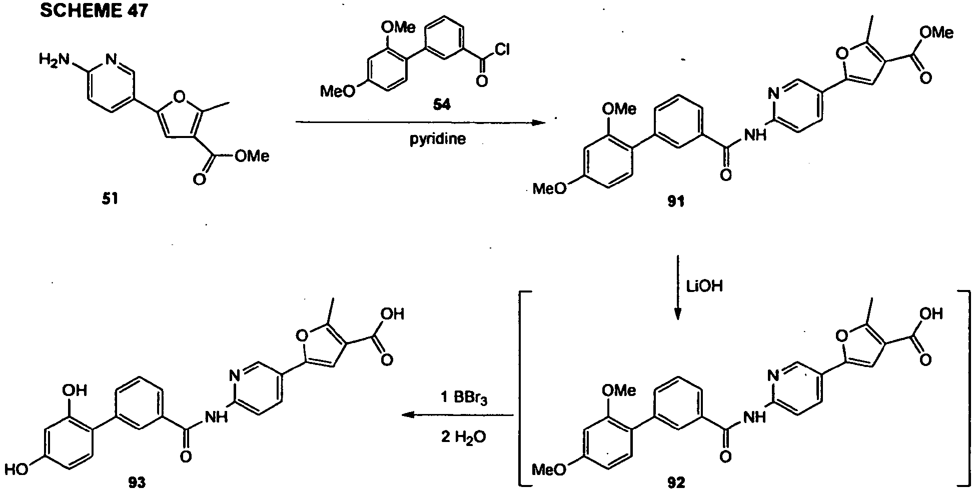

**Step 1:** (The following reaction is carried out in an N₂ atmosphere.) Dissolve 5-(6-amino-pyridin-3-yl)-2-methyl-furan-3-carboxylic acid methyl ester (51) (80.0mg, 0.34mmol) in anhydrous dichloromethane (5.4mL) and anhydrous pyridine (1.2mL) and cool the reaction mixture to 0°C. Add slowly a suspension of 2',4'-dimethoxy-biphenyl-3-carbonylchloride (54) (105mg, 0.38mmol) in dichloromethane (3.0mL) and stir the reaction mixture for 1h at 0°C and 19h at rt. Separate reaction mixture between water and EtOAc and extract the aqueous layer with EtOAc (3x). Wash the combined organic layer with water (6 times) to remove pyridine and with brine and dry with Na₂SO₄. Purify the crude product by preparative radial chromatography (silica gel 60PF, CyH/EtOAc 2+1) to obtain 5-{6-[(2',4'-dimethoxy-biphenyl-3-carbonyl)-amino]-pyridin-3-yl}-2-methyl-furan-3-carboxylic acid methyl ester (91) as an off-white solid (74mg, 45%). ¹H NMR (400 MHz, CDCl₃): 2.65 (s, 3 H); 3.80 (s, 3 H); 3.84 (s, 3 H); 3.85 (s, 3 H); 6.57 (s, 1 H); 6.58 (dd, 1 H, *J*₁ = 8.1 Hz, *J*₂ = 2.5 Hz); 6.89 (s, 1 H); 7.26 (d, 1 H, *J* = 8.1 Hz); 7.51 (t, 1 H, *J* = 7.6 Hz); 7.70 (dt, 1 H, *J*₁ = 7.8 Hz, *J*₂ = 1.3 Hz); 7.84 (dt, 1 H, *J*₁ = 7.8 Hz, *J*₂ = 1.3 Hz); 7.97 (dd, 1 H, *J*₁ = 8.8 Hz, *J*₂ = 2.3 Hz); 8.03 (t, 1 H, *J* = 1.5 Hz); 8.45 (d, 1 H, *J* = 8.8 Hz); 8.57 (d, 1 H, *J* = 2.3 Hz); 8.70 (br.s, 1 H).

**Step 2:** Dissolve 5-{6-[(2',4'-dimethoxy-biphenyl-3-carbonyl)-amino]-pyridin-3-yl}-2-methyl-furan-3-carboxylic acid methyl ester (91) (67.1mg, 0.14mmol) in THF (3mL) and MeOH (0.5mL) and add 1M aqu LiOH (0.71mL, 0.7 1 mmol). Stir reaction mixture 16h at rt. Remove solvents under reduced pressure and dissolve the residue in a mixture of THF/acetonitrile (2+1). Add Amberlite IRC86 and stir additional 2h at rt. Remove supernatant and stir remaining resin several times (10min each) with new portions of THF and acetonitrile. Concentrate collected supernatants under reduced pressure to obtain crude 5-{6-[(2',4'-dimethoxy-biphenyl-3-carbonyl)-amino]-pyridin-3-yl}-2-methyl-furan-3-carboxylic acid (92) as a light yellow solid (87mg, quant.) without further purification. ¹H NMR (400 MHz, (CD₃)₂SO): 2.61 (s, 3 H); 3.77 (s, 3 H); 3.81 (s, 3 H); 6.65 (dd, 1 H, *J*₁ = 8.6 Hz, *J*₂ = 2.5 Hz); 6.68 (d, 1 H, *J* = 2.5 Hz); 7.19 (s, 1 H); 7.38 (d, 1 H, *J* = 8.3 Hz); 7.50 (t, 1 H, *J* = 7.8 Hz); 7.68 (dt, 1 H, *J*₁ = 7.8 Hz, *J*₂ = 1.3 Hz); 7.91 (dt, 1 H, *J*₁ = 7.8 Hz, *J*₂ = 1.3 Hz); 8.05 (t, 1 H, *J* = 1.5 Hz); 8.13 (dd, 1 H, *J*₁ = 8.8 Hz, *J*₂ = 2.3 Hz); 8.27 (d, 1 H, *J* = 8.8 Hz); 8.75 (d, 1 H, *J*= 1.3 Hz); 11.00 (s, 1 H); 12.69 (br.s, 1 H).

**Step 3:** (The following reaction is carried out in an N₂ atmosphere.) Dissolve acid (92) (63.9mg, 0.14mmol) in a mixture of anhydrous 1,2-dichloroethane (4.0mL) and anhydrous dichloromethane (1.0mL), cool the solution to -50°C and add dropwise 1M BBr₃ solution in dichloromethane (0.70mL, 0.70mmol). Stir the reaction mixture for 30min at -78°C and after removal of the cooling bath for additional 1.5h at rt. Cool to 0°C, add dropwise water and stir the resulting mixture 30min at rt. Concentrate under reduced pressure and purify the crude product by preparative RP HPLC (gradient, water/CH₃CN 95:5 to 5:95) to obtain 5-{6-[(2',4'-dihydroxy-biphenyl-3-carbonyl)-amino]-pyridin-3-yl}-2-methyl-furan-3-carboxylic acid (93) (18mg, 30%) as an off-white solid. ¹H NMR (400 MHz, CD₃OD): 2.70 (s, 3 H); 6.45 (dd, 1 H, *J*₁ = 7.8 Hz, *J*₂ = 2.3 Hz); 6.47 (s, 1 H); 7.09 (br.s, 1 H); 7.22 (dd, 1 H, *J*₁ = 7.8 Hz, *J*₂ = 1.0 Hz); 7.56 (t, 1 H, *J*= 7.8 Hz); 7.82 (d, 1 H, *J*= 7.8 Hz); 7.88 (d, 1 H, *J* = 7.6 Hz); 8.15 (br.s, 2 H); 8.36 (br.d, 1 H, *J*= 7.8 Hz); 8.72 (br.s, 1 H).

### EXAMPLE 34

### 5-{4-[(2',4'-Dimethoxy-biphenyl-3-carbonyl)-amino]-phenyl}-2-methyl-furan-3-carboxylic acid ethyl ester (94)

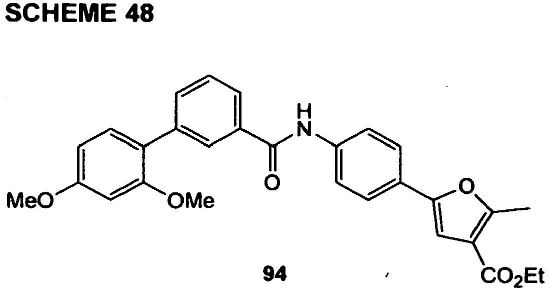

5-{4-[(2',4'-Dimethoxy-biphenyl-3-carbonyl)-amino]-phenyl}-2-methyl-furan-3-carboxylic acid ethyl ester (94) is synthesized according to the procedure described above in step 1 of EXAMPLE 8 starting from the aniline (25) and the carboxylic acid chloride (54) and is isolated as a yellow solid (190mg, 64%). ¹H NMR (400 MHz, C₆D₆): 1.17 (t, 3 H, *J*= 7.1 Hz); 2.59 (s, 3 H); 3.32 (s, 3 H); 3.50 (s, 3 H); 4.24 (q, 2 H, *J*= 7.1 Hz); 6.55 (dd, 1 H, *J*₁ = 8.3 Hz, *J*₂ = 2.3 Hz); 6.62 (d, 1 H, *J*= 2.3 Hz); 7.05 (s, 1 H); 7.32 (d, 1 H, *J =* 8.3 Hz); 7.25-7.34 (m, 2 H); 7.61 (d, 2 H, *J* = 9.0 Hz); 7.65 (d, 2 H, *J*= 9.0 Hz); 7.71 (d, 1 H, *J* = 7.8 Hz); 7.77 (d, 1 H, *J* = 7.8 Hz); 8.82 (s, 1 H).

### EXAMPLE 35

### 5-{4-[(2',4'-Dimethoxy-biphenyl-3-carbonyl)-amino]-phenyl}-2-methyl-furan-3-carboxylic acid (95)

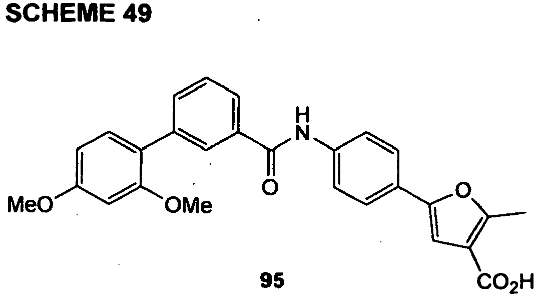

Saponification of the carboxylic acid ethyl ester (94) in a mixture of THF and MeOH (2+1) according to the procedure described above in EXAMPLE 31 affords 5-{4-[(2',4'-dimethoxy-biphenyl-3-carbonyl)-amino]-phenyl}-2-methyl-furan-3-carboxylic acid (95) as a off-white solid (137mg, quant.). ¹H NMR (400 MHz, (CD₃)₂SO): 2.60 (s, 3 H); 3.78 (s, 3 H); 3.82 (s, 3 H); 6.66 (dd, 1 H, *J*₁ = 8.3 Hz, *J*₂ = 2.2 Hz); 6.70 (d, 1 H, *J* = 2.2 Hz); 7.00 (s, 1 H); 7.32 (d, 1 H, *J =* 8.3 Hz); 7.53 (t, 1 H, *J =* 7.8 Hz); 7.66 (d, 1 H, *J* = 8.6 Hz); 7.69 (d, 2 H, *J* = 8.6 Hz); 7.86 (d, 3 H, *J* = 8.6 Hz); 7.99 (s, 1 H); 7.99 (s, 1 H); 10.34 (s, 1 H).

### EXAMPLE 36

### 5-{4-[(2',4'-Dihydroxy-biphenyl-3-carbonyl)-amino]-phenyl}-2-methyl-furan-3-carboxylic acid (96)

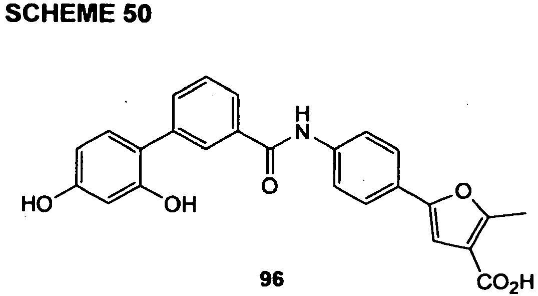

According to the procedure described above in EXAMPLE 12 reaction of the carboxylic acid (95) with a 1M BBr₃ solution affords 5-{4-[(2',4'-dihydroxy-biphenyl-3-carbonyl)-amino]-phenyl}-2-methyl-furan-3-carboxylic acid (96) as a light yellow solid (24mg, 32%). ¹H NMR (400 MHz, CD₃OD): 2.67 (s, 3 H); 6.43-6.47 (m, 1 H) 6.46 (s, 1 H); 6.95 (s, 1 H); 7.21 (d, 1 H, *J* = 8.6 Hz); 7.53 (t, 1 H, *J* = 7.7 Hz); 7.71 (d, 2 H , *J* = 8.8 Hz); 7.78-7.85 (m, 2 H); 7.81 (d, 2 H, *J* = 8.8 Hz); 8.11 (s, 1 H).

### EXAMPLE 37

### 2',4'-Dimethoxy-biphenyl-3-carboxylic acid (3-trifluoromethyl-phenyl)-amide (97)

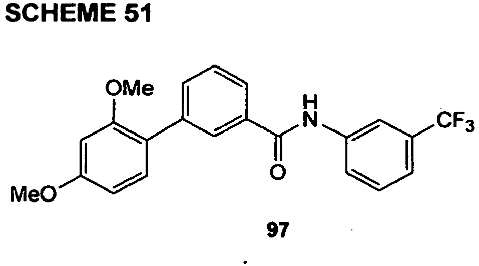

2',4'-Dimethoxy-biphenyl-3-carboxylic acid (3-trifluoromethyl-phenyl)-amide (97) is synthesized according to the procedure described above in step 1 of EXAMPLE 8 starting from 3-trifluoromethyl-phenylamine and carboxylic acid chloride (54) and is isolated as a yellow oil (32mg, 31%). ¹H NMR (400 MHz, CDCl₃): 3.80 (s, 3 H); 3.85 (s, 3 H); 6.56-6.60 (m, 1 H); 6.57 (s, 1 H); 7.26 (d, 1 H, *J*= 8.6 Hz); 7.39 (d, 1 H, *J*= 7.6 Hz); 7.47 (t, 1 H, *J* = 7.8 Hz); 7.50 (t, 1 H, *J* = 7.8 Hz); 7.69 (dt, 1 H, *J*₁ = 7.8 Hz, *J*₂ = 1.3 Hz); 7.77 (dt, 1 H, *J*₁ = 7.8 Hz, *J*₂ = 1.3 Hz); 7.84 (d, 1 H, *J* = 7.8 Hz); 7.88 (br. s, 1 H); 7.94 (s, 1 H); 7.96 (t 1 H, *J* = 1.5 Hz).

### EXAMPLE 38

### 2',4'-Dihydroxy-biphenyl-3-carboxylic acid (3-trifluoromethyl-phenyl)-amide (98)

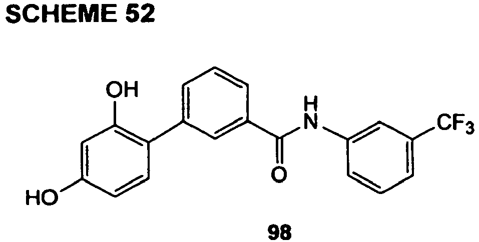

According to the procedure described above in EXAMPLE 12 reaction of 2',4'-dimethoxy-biphenyl-3-carboxylic acid (3-trifluoromethyl-phenyl)-amide (97) with a 1M BBr₃ solution affords 2',4'-dihydroxy-biphenyl-3-carboxylic acid (3-trifluoromethylphenyl)-amide (98) as a brown solid (27mg, 72%). ¹H NMR (400 MHz, CD₃OD): 6.45 (dd, 1 H, *J*₁ = 8.3 Hz, *J*₂ = 2.5 Hz); 6.46 (s, 1 H); 7.21 (dd, 1 H, *J*₁ = 8.6 Hz, *J*₂ = 1.3 Hz); 7.47 (d, 1 H, *J*= 7.8 Hz); 7.54 (t, 1 H, *J*= 7.8 Hz); 7.59 (t, 1 H, *J*= 8.0 Hz); 7.81 (dt, 1 H, *J*₁ = 7.8 Hz, *J*₂ = 1.3 Hz); 7.85 (ddd, 1 H, *J*₁ = 7.8 Hz, *J*₂ = 1.7 Hz, *J*₃ = 1.0 Hz); 7.99 (d, 1 H, J= 7.8 Hz); 8.12 (t, 1 H, *J* = 1.7 Hz); 8.21 (s, 1 H).

The compounds referred to in the following SCHEME 12 are those compounds referred to as the particularly preferred compounds herein.

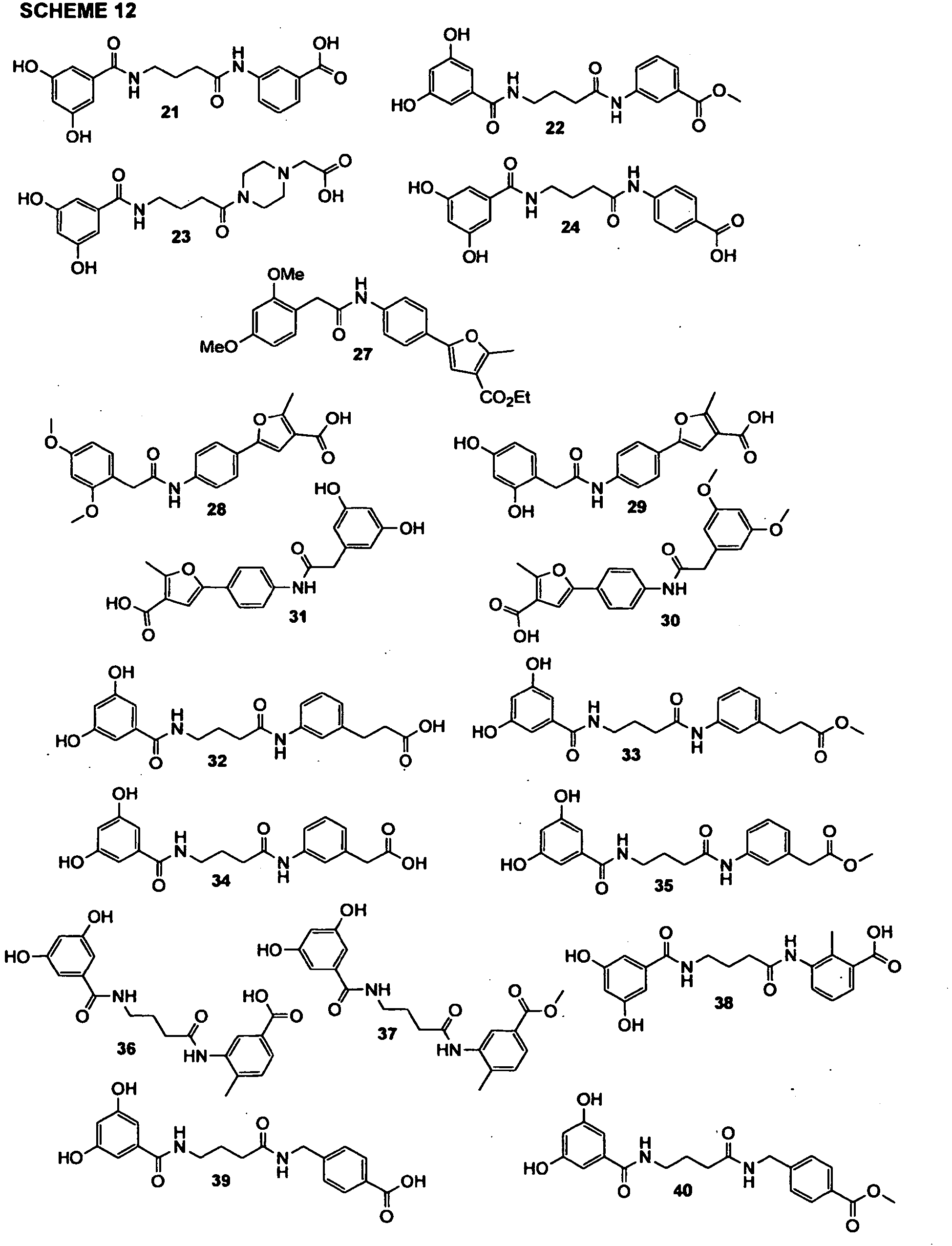

### Sialyl Lewis^{X} Tyrosine Sulfate Assay (sLe^{x} TSA):

Compounds of the present invention are assayed on a molecular level for their ability to inhibit the binding of P-, L-, or E-selectin chimeric molecules to sLe^{x} and tyrosinesulfate residues linked to a polymeric matrix as a PSGL-1 substitute. Selected IC₅₀-values are determined.

Microtiter plates are coated overnight in carbonate buffer pH9,6 with goat anti human Fc mAB (10 µg/ml). After washing in assay buffer (25mM 4-(2-hydroxyethyl)-1-piperazineethanesulfonic acid (HEPES), 150mM NaCl, 1mM CaCl₂ pH7,4) and blocking (3% bovine serum albumin (BSA) in assay buffer) plates are incubated for 2h at 37°C with human P-Selectin-IgG-chimera (0,61nM respectively 150ng/mL) or human L-Selectin-IgG-chimera (0,61nM respectively 89ng/mL) or human E-Selectin-IgG-chimera (0,61nM respectively 131ng/mL). 5µl of sLe^{x} -tyrosine sulfate polyacrylamide (1mg/ml) carrying 15% sLe^{x}, 10% Tyrosine-sulfate and 5% biotin is complexed with 20µl Streptavidin-Peroxidase solution (1mg/ml) and 25µl assay buffer without CaCl₂. For use in the assay, the ligand complex is diluted 1:10000 in assay buffer and further diluted 1:1 with varying amounts of compounds in assay buffer incl. 2%DMSO. This mixture is added to the wells precoated with E- or P-selectin. After incubation for 2h at 37°C, wells are washed for six times with in assay buffer incl. 0,005% Polyoxyethylenesorbitan monolaurate (TWEEN 20), developed for 10-15min with 20µl 3,3',5,5'-tetramethylbenzidine (TMB)/H₂0₂ substrate solution and stopped with 20µl 1M H₂SO₄. Bound sLe^{x} -Tyrosine sulfate ligand complex is determined by measuring optical density at 450nm vs. 620nm in a Fusion alpha-FP reader (sold from Packard Bioscience, Dreieich, Germany).

### Results from sLe^{x}TSA: in vitro inhibition Data for E-/ P-/ L-Selectin at 100µM

| **Compound** | **E-Selectin [% inhib.]** | **P-Selectin [% inhib.]** | **L-Selectin [% inhib.]** |
|---|---|---|---|
| **Bimosiamose** | 3.9 | 22.6 | 6.2 |
| **21** | 40.7 | 3.0 | 18.7 |
| **22** | 42.7 | 22.7 | 21.3 |
| **23** | 10.5 | 18.9 | 11.6 |
| **24** | 35.9 | 0.5 | 5.9 |
| **32** | 35.0 | 1.4 | 13.1 |
| **33** | 38.6 | 10.6 | 27.3 |
| **34** | 41.0 | 12.9 | 18.1 |
| **35** | 40.8 | 15.6 | 19.4 |
| **36** | 35.8 | 1.2 | 6.8 |
| **37** | 38.9 | 5.2 | 9.5 |
| **38** | 40.9 | 9.4 | 19.3 |
| **39** | 24.8 | 2.9 | 10.9 |
| **40** | 34.4 | 28.2 | 31.1 |

### Results from sLe^{x}TSA: IC₅₀ Data for E-/ P-/ L-Selectin

| **Compound** | **IC₅₀ E-Selectin [µM] IC₅₀** | **P-Selectin [µM] IC₅₀** | **L-Selectin [µM]** |
|---|---|---|---|
| | | | |
| **62** | — | 2.9 | 150.8 |
| **63** | — | 81.2 | 157.2 |
| **64** | — | 50.0 | 62.3 |
| **66** | — | 67.8 | 152.2 |
| **67** | — | 201.2 | 433.9 |
| **70** | — | 354.1 | 0.0 |
| **73** | — | 260.5 | 502.2 |
| **76** | **480.0** | **233.8** | **278.4** |
| **81** | — | 35,3 | 131,1 |
| **34** | — | 46,6 | 143,8 |
| **90** | — | 19.1 | 38.7 |

| **Compound** | **IC₅₀ E-Selectin [µM] IC₅₀** | **P-Selectin [µM]** | **IC₅₀ L-Selectin [µM]** |
|---|---|---|---|
| **Bimosiamose** | >500 | 95.0 | >500 |
| **28** | 200.7 | 237.7 | 318.6 |
| **30** | >500 | 133.5 | 376.1 |

### Flow Chamber Assay / Cell Adhesion and Rolling under Flow Conditions

To assess the capability of compounds to inhibit cell binding under dynamic conditions resembling the flow in a blood vessel, flow chamber assays addressing/ testing binding of HL-60 cells / various cell lines to P-selectin, L-selectin and E-selectin chimeric molecules are performed.

Cell attachment under flow conditions is determined using a parallel flow chamber system. A 35mm polystyrene culture dish is coated for 1 hour at room temperature with coating buffer (50mM tris-(hydroxymethyl) aminomethane buffer (Tris), 150 mM NaCl, 2 mM CaCl₂; pH 7,4) containing human E- or P-selectin-IgG chimera at concentrations of 2,5µg/ml or 10µg/ml, respectively. After removal of the coating solution non specific binding sites are blocked for an additional hour with 1% BSA in coating buffer at room temperature. After washing with assay buffer ("Roswell Park Memorial Institute 1640" (RPMI 1640) + 10mM HEPES) the dish is fitted into a parallel plate laminar flow chamber (sold from Glycotech, Rockville, MD) and mounted on an inverted phase-contrast microscope (sold from Olympus, Hamburg, Germany) equipped with a CCD camera (JVC) that is connected to a PC. Employing a peristaltic pump (sold from Ismatec, Wertheim-Mondfeld, Germany) the re-circulating system is equilibrated with assay buffer containing 125µM compound or vehicle control (DMSO). Cells (1 million / ml) are added to the chamber and allowed to distribute for 2 minutes at a high flow rate. The flow rate is then decreased resulting in a calculated flow shear of 1 dyne/cm². Video sequences of 10 low power fields are digitally recorded after 5 minutes continuous flow. The percentage of inhibition is calculated from the mean number of cells per field that attached to the coated dish surface in the presence versus absence of compound of at independent experiments.

### Data from Flow Chamber Assay for E- and P-Selectin

Values are given as normalized ratios of %-inhibition of compound x divided by %-inhibition of bimosiamose.

| **Compound** | **E-Selectin [Ratio]** | **P-Selectin [Ratio]** |
|---|---|---|
| **28** | 0.99 | n.s. |
| **29** | 1.31 | 0.74 |
| **30** | 1.03 | n.s. |
| **31** | 1.16 | 0.60 |

| **Compound** | **E-Selectin [Ratio]** | **P-Selectin [Ratio]** |
|---|---|---|
| **Bimosiamose** | | |
| **62** | 1.15 | 1.22 |
| **63** | 1.31 | 1.95 |
| **64** | 1.34 | 1.70 |
| **66** | 1.21 | 0.76 |
| **67** | 1.60 | 0.92 |
| **70** | 0.83 | 0.82 |
| **73** | 0.99 | 1.20 |
| **74** | 0.68 | 1.02 |
| **76** | 0.61 | 1.26 |
| **79** | 0.96 | 1.19 |
| **81** | 1.27 | 1.55 |
| **82** | 1.35 | 2.11 |
| **84** | 1.36 | 1.02 |
| **85** | 1.20 | 1.90 |
| **87** | 1.24 | 1.05 |
| **89** | 1.27 | 1.41 |
| **93** | 0.86 | 1.13 |
| **96** | 1.13 | 1.02 |
| **98** | 1.26 | 1.00 |

## Claims

1. Pharmaceutical compositions comprising at least one compound of the formulas (G1) or (G2) or (H1) or (H2) and a pharmaceutically acceptable carrier which is useful in a medicine, 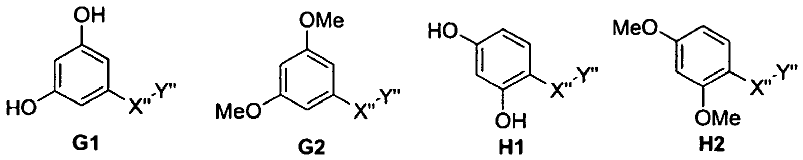 wherein the symbols and substituents have the following meaning
X"- is 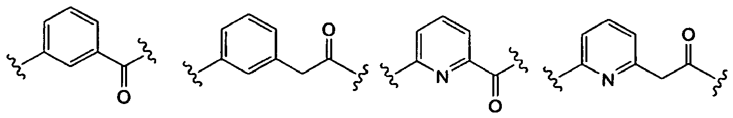 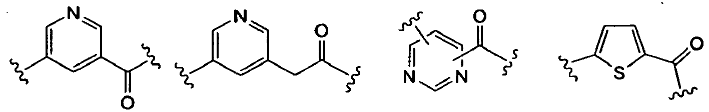 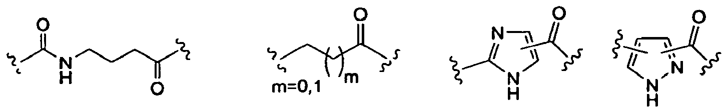 with m = 0,1;
Y" is 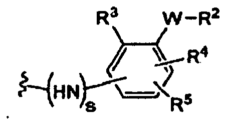 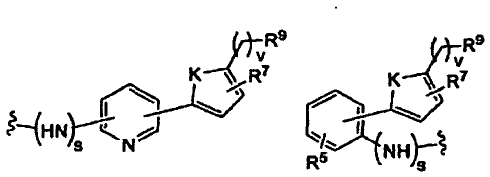 with s being 0 or 1,
R² being CO₂H, CO₂alkyl, CO₂aryl, CO₂NH₂, CO₂aralkyl, SO₃H, SO₂NH₂, PO(OH)₂, 1-H-tetrazolyl-, CHO, COCH₃, CH₂OH, NH₂, NHalkyl, N(alkyl)alkyl', OCH₃, CH₂OCH₃, SH, F, Cl, Br, I, CH₃, CH₂CH₃, CN, CF₃
R³ independently from R² being H, CH₃, CH₂CH₃, CF₃, F, Cl, Br, I, CN, NO₂ and
R⁴ independently from R² and R³ being H, CH₃, CH₂CH₃, CF₃, F, Cl, Br, I, CN, NO₂, R²
R⁵ being H, NO₂, CF₃, F, Cl. Br, I, CN, CH₃, OCH₃, SH, NH₂ and -W- = -(CH₂-)ᵥ, *cis*-CH=CH- or *trans*-CH=CH-, and v being 0,1,2;
in case that -W- is *cis*-CH=CH- or *trans*-CH=CH-, R² must not be NH₂ or SH;
R⁷ independently from R² being H, NO₂, CF₃, F, Cl, Br, I, CN, CH₃, OCH₃, SH, NH₂,
with K = NH, NMe, O, S
with R⁹ being CO₂H, CO₂alkyl, CO₂aryl, CO₂NH₂, CO₂aralkyl, CH₂SO₃H, CH₂SO₂NH₂, CH₂PO(OH)₂, 1-H-tetrazolyl, CHO, COCH₃, CH₂OH, CH₂NH₂, CH₂NHalkyl, CH₂N(alkyl)alkyl', CH₂OCH₃, CH₂SH,
or the pharmaceutically acceptable salts, esters or amides of the above identified compounds of formulas (G1) or (G2) or (H1) or (H2).

2. Chemical compounds having the general structure of formula (E1), (E2), (F 1), (F2), (G1), (G2), (H1) or (H2) 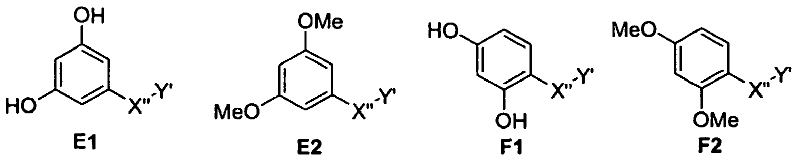 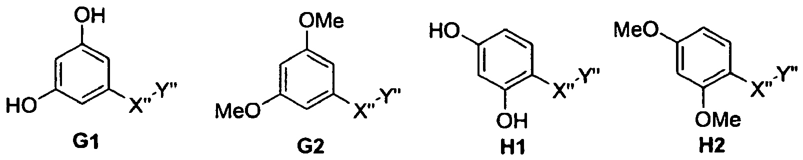 wherein -Y' is 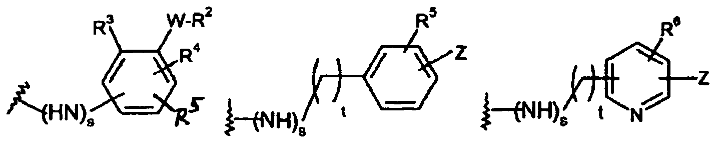 with R⁶ independently from R² being H, F, Cl, Me, tert-Bu, CN, NH₂;
-Z = 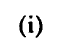 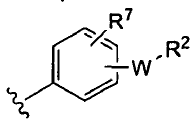 R⁷ independently from R² being H, NO₂, CF₃, F, Cl, Br, I, CN, CH₃, OCH₃, SH, NH₂, 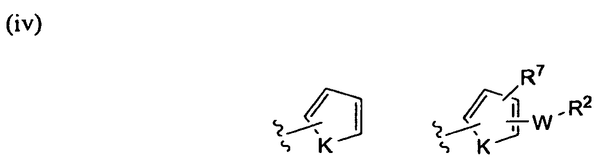 with K = NH, NMe, O, S 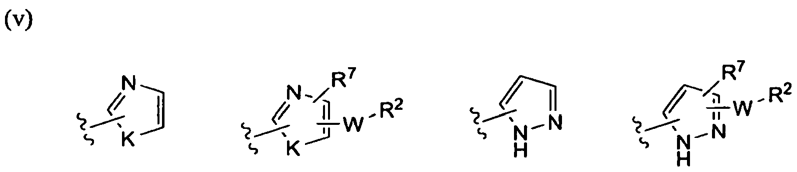 wherein all other indices, symbols and substituents are as defined in claim 1.

3. Compounds having the structure of formulas (G1) or (G2) or (H1) or (H2) as defined in claim 1 for use in the treatment of Chronic Obstructive Pulmonary Disease (COPD), acute lung injury (ALI), cardiopulmonary bypass, acute respiratory distress syndrome (ARDS), Crohn's disease, septic shock, sepsis, chronic inflammatory diseases such as psoriasis, atopic dermatitis, and rheumatoid arthritis, and reperfusion injury that occurs following heart attacks, strokes, atherosclerosis, and organ transplants, traumatic shock, multi-organ failure, autoimmune diseases like multiple sclerosis, percutaneous transluminal angioplasty, asthma and inflammatory bowel disease.

4. Dermatological composition comprising at least one compound of formulas (G1) or (G2) or (H1) or (H2) as defined in claim 1 for use in the dermatological treatment or dermatological prophylaxis of micro-inflammatory conditions, itching or skin ageing.

5. Use of compounds having the structure of formulas (G1) or (G2) or (H1) or (H2) as defined in claim 1 for the preparation of cosmetic compositions.

6. Cosmetic compositions comprising at least one compound of the formulas (G1) or (G2) or (H1) or (H2) as in claim 1 and at least one cosmetically tolerable component.

7. Dermatological compositions comprising at least one compound of formulas (G1) or (G2) or (H1) or (H2) as in claim 1 and at least one dermatologically tolerable component.

## Patentansprüche

1. Pharmazeutische Zusammensetzungen, umfassend mindestens eine Verbindung der Formeln (G1) oder (G2) oder (H1) oder (H2) sowie einen pharmazeutisch verträglichen Träger, welcher in einem Medikament verwendbar ist, 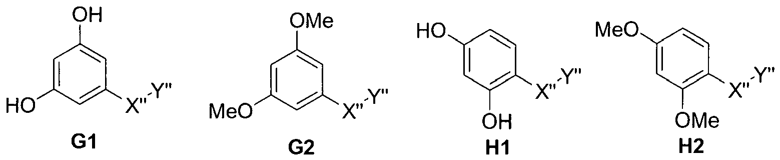 wobei die Symbole und Substituenten folgende Bedeutungen haben:
X"- ist 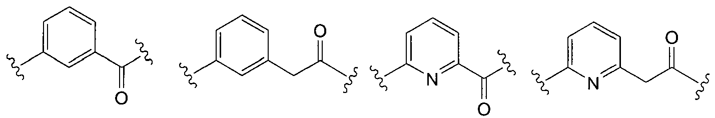 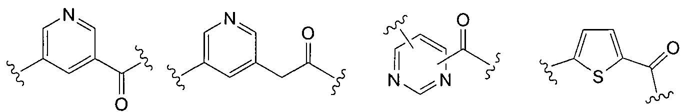 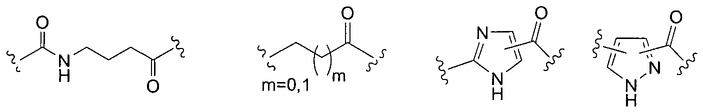 wobei m = 0,1;
Y" ist 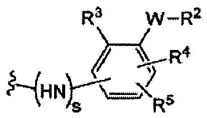 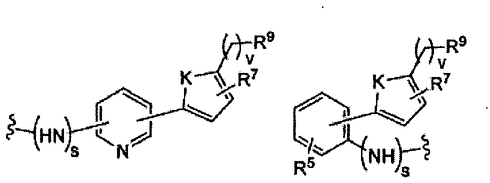 wobei s für 0 oder 1 steht,
R² für CO₂H, CO₂Alkyl, CO₂Aryl, CO₂NH₂ CO₂aralkyl SO₃H, SO₂NH₂, PO(OH)₂, 1-H-Tetrazolyl-, CHO, COCH₃, CH₂OH, NH₂, NHAlkyl, N(Alkyl)Alkyl', OCH₃, CH₂OCH₃, SH, F, Cl, Br, I, CH₃, CH₂CH₃, CN, CF₃ steht,
R³ unabhängig von R² für H, CH₃, CH₂CH₃, CF₃, F, Cl, Br, I, CN, NO₂ steht, und
R⁴ unabhängig von R² und R³ für H, CH₃, CH₂CH₃, CF₃, F, Cl, Br, I, CN, NO₂, R² steht,
R⁵ für H, NO₂, CF₃, F, Cl. Br, I, CN, CH₃, OCH₃, SH, NH₂ steht,
und -W- = -(CH₂-)ᵥ, *cis*-CH=CH- oder *trans*-CH=CH-, und v für 0,1,2 steht;
wenn -W- für cis-CH=CH- oder *trans*-CH=CH- steht, darf R² nicht NH₂ oder SH sein;
R⁷ unabhängig von R² für H, NO₂, CF₃, F, Cl, Br, I, CN, CH₃, OCH₃, SH, NH₂ steht,
wobei K = NH, NMe, O, S, wobei R⁹ für CO₂H, CO₂Alkyl, CO₂Aryl, CO₂NH₂, CO₂Aralkyl, CH₂SO₃H, CH₂SO₂NH₂, CH₂PO(OH)₂, 1-H-Tetrazolyl, CHO, COCH₃, CH₂OH, CH₂NH₂, CH₂NHAlkyl, CH₂N(Alkyl)Alkyl', CH₂OCH₃, CH₂SH steht,
oder die pharmazeutisch akzeptablen Salze, Ester oder Amide der oben genannten Verbindungen der Formeln (G1) oder (G2) oder (H1) oder (H2).

2. Chemische Verbindungen, die die allgemeine Struktur der Formeln (E1), (E2),
(F1), (F2), (G1), (G2), (H1) oder (H2) aufweisen 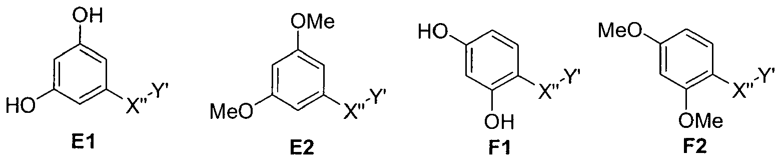 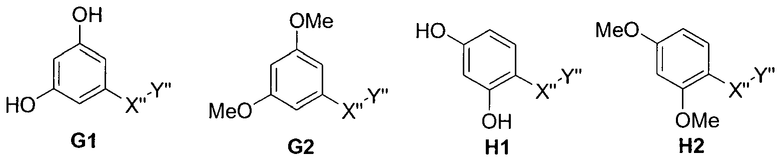 wobei -Y' 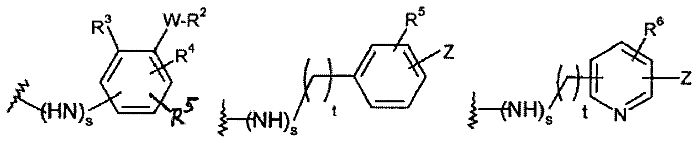 ist,
wobei R⁶ unabhängig von R² für H, F, Cl, Me, tert-Bu, CN, NH₂ steht;
-Z = 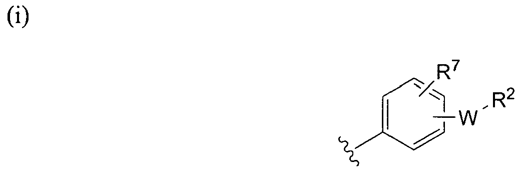 R⁷ unabhängig von R² für H, NO₂, CF₃, F, Cl, Br, I, CN, CH₃, OCH₃, SH, NH₂ steht, 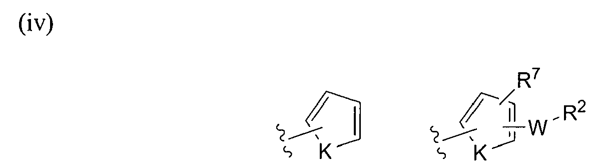 wobei K = NH, NMe, O, S 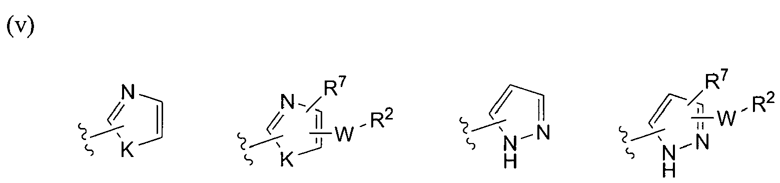 worin alle weiteren Indizes, Symbole und Substituenten wie in Anspruch 1 definiert sind.

3. Verbindungen, die die Struktur der Formeln (G1) oder (G2) oder (H1) oder (H2) wie in Anspruch 1 definiert aufweisen, zur Verwendung bei der Behandlung von einer chronisch obstruktiven Lungenerkrankung (COPD), akutem Lungenversagen (ALI), einem cardiopulmonalen Bypass, einem akuten respiratorischen Distresssyndrom (ARDS), Morbus Crohn, einem septischen Schock, Sepsis, chronischen Entzündungserkrankungen wie Psoriasis, atopischer Dermatitis und rheumatoider Arthritis sowie einem Reperfusionsschaden, der nach Herzinfarkten, Schlaganfällen, Arteriosklerose und Organtransplantationen auftritt, einem traumatischen Schock, Multiorganversagen, Autoimmunerkrankungen wie Multipler Sklerose, perkutaner transluminaler Angioplastie, Asthma und einer Darmentzündung.

4. Dermatologische Zusammensetzung umfassend mindestens eine Verbindung der Formeln (G1) oder (G2) oder (H1) oder (H2) wie in Anspruch 1 definiert zur Verwendung bei der dermatologischen Behandlung oder dermatologischen Prophylaxe von Mikroentzündungserkrankungen, Juckreiz oder Hautalterung.

5. Verwendung der Verbindungen, die die Struktur der Formeln (G1) oder (G2) oder (H1) oder (H2) wie in Anspruch 1 definiert aufweisen, zur Herstellung von kosmetischen Zusammensetzungen.

6. Kosmetische Zusammensetzungen umfassend mindestens eine Verbindung der Formeln (G1) oder (G2) oder (H1) oder (H2) wie in Anspruch 1 und mindestens eine kosmetisch zulässige Komponente.

7. Dermatologische Zusammensetzungen umfassend mindestens eine Verbindung der Formeln (G1) oder (G2) oder (H1) oder (H2) wie in Anspruch 1 und mindestens eine dermatologisch zulässige Komponente.

## Revendications

1. Compositions pharmaceutiques comprenant au moins un composé de formules (G1) ou (G2) ou (H1) ou (H2) et un support pharmaceutiquement acceptable qui est utile dans un médicament, 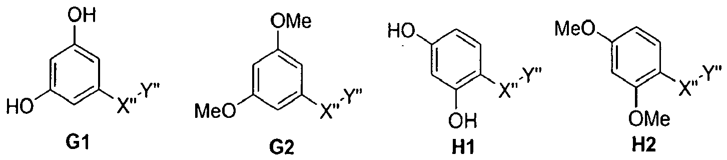 dans laquelle les symboles et substituants ont la signification suivante:
- X" est 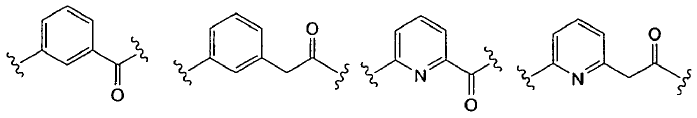 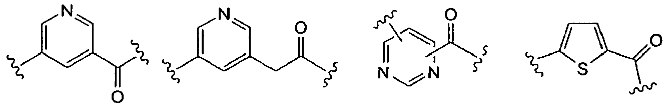 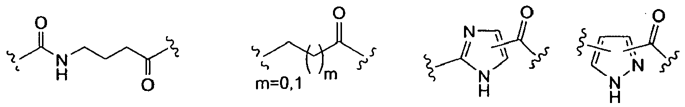 où m = 0,1 ;
- Y" est 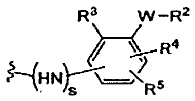 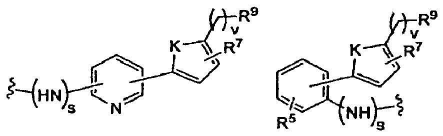 où s est 0 ou 1,
- R² est CO₂H, CO₂alkyl, CO₂aryl, CO₂NH₂, CO₂aralkyl, SO₃H, SO₂NH₂, PO(OH)₂, 1-H-tétrazolyle-, CHO, COCH₃, CH₂OH, NH₂, NH-alkyle, N(alkyle)-alkyle', OCH₃ , CH₂OCH₃, SH, F, Cl, Br, I, CH₃, CH₂CH₃, CN, CF₃
- R³, indépendamment de R² est H, CH₃, CH₂CH₃, CF₃, F, Cl, Br, I, CN, NO₂ et
- R⁴ indépendamment de R² et R³ est H, CH₃, CH₂CH₃, CF₃, F, Cl, Br, I, CN, NO₂, R²
- R⁵ est H, NO₂, CF₃, F, Cl, Br, I, CN, CH₃, OCH₃, SH, NH₂ et
- W- = -(CH₂-)ᵥ, *cis*-CH = CH- ou trans-CH = CH-, où v est 0,1,2; dans le cas où-W- est un groupe *cis*-CH = CH- ou trans-CH = CH-, R² ne doit pas être NH₂ ou SH;
- R⁷, indépendamment de R² est H, NO₂, CF₃, F, Cl, Br, I, CN, CH₃, OCH₃, SH, NH₂
- K = NH, NMe, O, S
- R⁹ est CO₂H, CO₂alkyl, CO₂aryl, CO₂NH₂, CO₂aralkyl, CH₂SO₃H, CH₂SO₂NH₂, CH₂PO(OH)₂, 1-H-tétrazolyle-, CHO, COCH₃, CH₂OH, CH₂NH₂, CH₂NHalkyle, CH₂N(alkyle)alkyle', CH₂OCH₃, CH₂SH,
ou les sels pharmaceutiquement acceptables, esters ou amides de composés de formules ci-dessus identifiés (G1) ou (G2) ou (H1) ou (H2).

2. Composés chimiques présentant la structure générale des formules (E1), (E2), (F1), (F2), (G1), (G2), (H1) ou (H2) 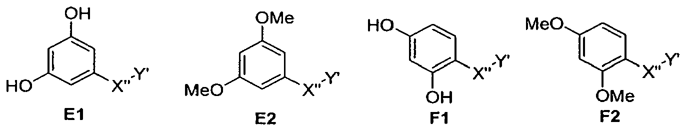 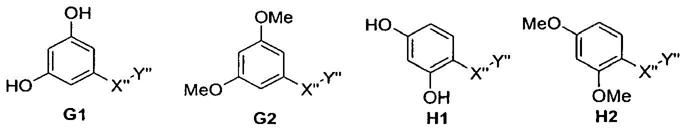 où:
- Y' est 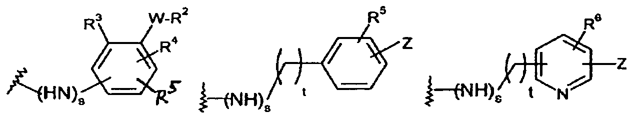
- R⁶, indépendamment de R² est H, F, Cl, Me, tert-Bu, CN, NH₂;
- - Z= 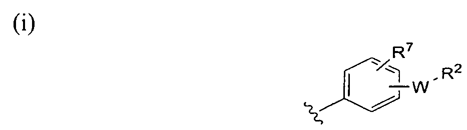
- R⁷, indépendamment de R² est H, NO₂, CF₃, F, Cl, Br, I, CN, CH₃, OCH₃, SH, NH₂ 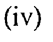 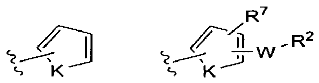 où K = NH, NMe, O, S 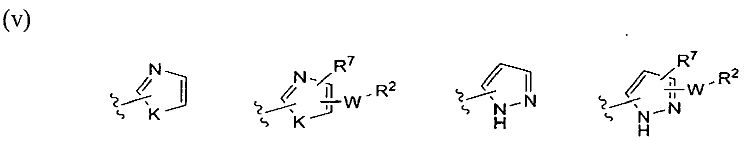
où l'ensemble des indices, symboles et substituants sont tels que définis dans la revendication 1.

3. Utilisation de composés ayant la structure de formule (G1) ou (G2) ou (H1) ou (H2) tel que définis dans la revendication 1 pour la préparation d'un médicament pour le traitement des maladies pulmonaires obstructives chroniques (MPOC), des lésions pulmonaires aiguës (ALI), des by-pass cardio-pulmonaires, du syndrome de détresse respiratoire aiguë (SDRA), de la maladie de Crohn, du choc septique, de la septicémie, des maladies inflammatoires chroniques telles que le psoriasis, la dermatite atopique, la polyarthrite rhumatoïde et des lésions de reperfusion se produisant après des crises cardiaques, des accidents vasculaires cérébraux, de l'athérosclérose et des transplantations d'organes, des chocs traumatiques, des affections multiviscérales, des maladies auto-immunes comme la sclérose en plaques, l'angioplastie transluminale percutanée, l'asthme et les maladies inflammatoires de l'intestin.

4. Composition dermatologique comprenant au moins un composé de formule (G1) ou (G2) ou (H1) ou (H2) tel que définis dans la revendication 1, pour la préparation d'un médicament pour le traitement dermatologique ou la prophylaxie de troubles inflammatoires, démangeaisons ou vieillissement de la peau.

5. Utilisation de composés ayant la structure de formule (G1) ou (G2) ou (H1) ou (H2) tel que définis dans la revendication 1 pour la préparation de composés cosmétiques.

6. Compositions cosmétiques comprenant au moins un composé de formules (G1) ou (G2) ou (H1) ou (H2) tel que définis dans la revendication 1 ayant au moins un composant cosmétiquement tolérable.

7. Compositions dermatologiques comprenant au moins un composé de formules (G1) ou (G2) ou (H1) ou (H2) tel que définis dans la revendication 1 et au moins un composant dermatologiquement tolérable.
